(19) Europäisches Patentamt
European Patent Office
Office européen des brevets

(11) EP 2 469 278 A1

(12) EUROPEAN PATENT APPLICATION

(43) Date of publication:
27.06.2012 Bulletin 2012/26

(51) Int Cl.:
*G01N 33/533* (2006.01) *G01N 33/542* (2006.01)
*C07K 14/195* (2006.01) *C12N 15/10* (2006.01)

(21) Application number: 10290670.8

(22) Date of filing: 21.12.2010

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME

(71) Applicants:
- Institut Pasteur
75015 Paris (FR)
- Centre National de la Recherche Scientifique CNRS
75794 Paris Cedex 16 (FR)

(72) Inventors:
- Bedouelle, Hugues
75015 Paris (FR)
- Miranda, Frédéric
1000 Bruxelles (BE)
- Pecorari, Frédéric
44880 Sautron (FR)
- Zidane, Nora
75015 Paris (FR)

(74) Representative: Leblois-Préhaud, Hélène Marthe Georgette et al
Cabinet Orès
36, rue de St Petersbourg
75008 Paris (FR)

# (54) Reagentless fluorescent biosensors from nanofitins, rational design methods to create reagentless fluorescent biosensors and methods of their use

(57) The present invention relates to reagentless fluorescent biosensors from Nanofitins which comprise at least an OB-fold protein and a fluorophore and are specific for a target ; the method for preparing such reagentless fluorescent biosensors comprises: (a) substituting each of the variable residues of said OB-fold protein, individually with a cysteine residue, thereby obtaining a set of cysteine mutants; (b) coupling said cysteine residue of each of the cysteine mutants of step (a) to a fluorophore, thereby obtaining biosensors; (c) purifying the biosensors of step (b); (d) measuring the equilibrium constant ($K_D$) between each of said purified biosensors of step (c) and said target, or the dissociation ($K_{off}$) and association ($k_{on}$) rate constants for each of said biosensors of step (c) and said target; and measuring the fluorescence variation of each of said biosensors of step (c) between a free and target bound state; and (f) determining the sensitivity ($s$) and/or relative sensitivity ($s_r$) of each of said biosensors of step (c) from the measurements of step (d).



Printed by Jouve, 75001 PARIS (FR)

SEQUENCE LISTING

<110> INSTITUT PASTEUR
CENTRE NATIONAL DE LA RECHERCHE SCIENTIFIQUE
BEDOUELLE, Hugues
MIRANDA, Frédéric
PECORARI, Frédéric
ZIDANE, Nora

<120> Reagentless fluorescent biosensors from Nanofitins, rational design methods to create reagentless fluorescent biosensors and methods of their use

<130> F226EP182

<160> 64

<170> PatentIn version 3.5

<210> 1
<211> 66
<212> PRT
<213> Sulfolobus acidocaldarius

<400> 1

```
Met Val Lys Val Lys Phe Lys Tyr Lys Gly Glu Glu Lys Glu Val Asp
1               5                   10                  15

Thr Ser Lys Ile Lys Lys Val Trp Arg Val Gly Lys Met Val Ser Phe
            20                  25                  30

Thr Tyr Asp Asp Asn Gly Lys Thr Gly Arg Gly Ala Val Ser Glu Lys
        35                  40                  45

Asp Ala Pro Lys Glu Leu Leu Asp Met Leu Ala Arg Ala Glu Arg Glu
    50                  55                  60

Lys Lys
65
```

<210> 2
<211> 65
<212> PRT
<213> Sulfolobus acidocaldarius

<400> 2

```
Met Ala Lys Val Arg Phe Lys Tyr Lys Gly Glu Glu Lys Glu Val Asp
1               5                   10                  15

Thr Ser Lys Ile Lys Lys Val Trp Arg Val Gly Lys Met Val Ser Phe
            20                  25                  30

Thr Tyr Asp Asp Asn Gly Lys Thr Gly Arg Gly Ala Val Ser Glu Lys
        35                  40                  45

Asp Ala Pro Lys Glu Leu Met Asp Met Leu Ala Arg Ala Glu Lys Lys
    50                  55                  60
```

Lys
65

<210> 3
<211> 64
<212> PRT
<213> Sulfolobus solfataricus

<400> 3

Met Ala Thr Val Lys Phe Lys Tyr Lys Gly Glu Glu Lys Glu Val Asp
1 5 10 15

Ile Ser Lys Ile Lys Lys Val Trp Arg Val Gly Lys Met Ile Ser Phe
20 25 30

Thr Tyr Asp Glu Gly Gly Gly Lys Thr Gly Arg Gly Ala Val Ser Glu
35 40 45

Lys Asp Ala Pro Lys Glu Leu Leu Gln Met Leu Glu Lys Gln Lys Lys
50 55 60

<210> 4
<211> 64
<212> PRT
<213> Sulfolobus tokodaii

<400> 4

Met Val Thr Val Lys Phe Lys Tyr Lys Gly Glu Glu Lys Glu Val Asp
1 5 10 15

Ile Ser Lys Ile Lys Lys Val Trp Arg Val Gly Lys Met Ile Ser Phe
20 25 30

Thr Tyr Asp Asp Asn Gly Lys Thr Gly Arg Gly Ala Val Ser Glu Lys
35 40 45

Asp Ala Pro Lys Glu Leu Leu Gln Met Leu Glu Lys Ser Gly Lys Lys
50 55 60

<210> 5
<211> 64
<212> PRT
<213> Sulfolobus shibatae

<400> 5

Met Val Thr Val Lys Phe Lys Tyr Lys Gly Glu Glu Lys Glu Val Asp
1 5 10 15

Thr Ser Lys Ile Lys Lys Val Trp Arg Val Gly Lys Met Ile Ser Phe
20 25 30

Thr Tyr Asp Glu Gly Gly Gly Lys Thr Gly Arg Gly Ala Val Ser Glu
35 40 45

Lys Asp Ala Pro Lys Glu Leu Leu Gln Met Leu Glu Lys Gln Lys Lys
50 55 60

<210> 6
<211> 64
<212> PRT
<213> Sulfolobus shibatae

<400> 6

Met Ala Thr Val Lys Phe Lys Tyr Lys Gly Glu Glu Lys Gln Val Asp
1 5 10 15

Ile Ser Lys Ile Lys Lys Val Trp Arg Val Gly Lys Met Ile Ser Phe
20 25 30

Thr Tyr Asp Glu Gly Gly Gly Lys Thr Gly Arg Gly Ala Val Ser Glu
35 40 45

Lys Asp Ala Pro Lys Glu Leu Leu Gln Met Leu Glu Lys Gln Lys Lys
50 55 60

<210> 7
<211> 64
<212> PRT
<213> Sulfolobus solfataricus

<400> 7

Met Ala Thr Val Lys Phe Lys Tyr Lys Gly Glu Glu Lys Gln Val Asp
1 5 10 15

Ile Ser Lys Ile Lys Lys Val Trp Arg Val Gly Lys Met Ile Ser Phe
20 25 30

Thr Tyr Asp Glu Gly Gly Gly Lys Thr Gly Arg Gly Ala Val Ser Glu
35 40 45

Lys Asp Ala Pro Lys Glu Leu Leu Gln Met Leu Glu Lys Gln Lys Lys
50 55 60

<210> 8
<211> 87
<212> PRT
<213> Escherichia coli

<220>
<221> mat_peptide
<222> (20)..(87)

<400> 8

Met Lys Lys Met Phe Met Ala Val Leu Phe Ala Leu Val Ser Val Asn
-15 -10 -5

Ala Met Ala Ala Asp Cys Ala Lys Gly Lys Ile Glu Phe Ser Lys Tyr
-1 1 5 10

Asn Glu Asp Asp Thr Phe Thr Val Lys Val Ser Gly Arg Glu Tyr Trp
15 20 25

Thr Asn Arg Trp Asn Leu Gln Pro Leu Leu Gln Ser Ala Gln Leu Thr
30 35 40 45

Gly Met Thr Val Thr Ile Ile Ser Asn Thr Cys Ser Ser Gly Ser Gly
50 55 60

Phe Ala Glu Val Gln Phe Asn
65

<210> 9
<211> 89
<212> PRT
<213> Escherichia coli

<220>
<221> mat_peptide
<222> (20)..(89)

<400> 9

Met Lys Lys Met Phe Met Ala Val Leu Phe Ala Leu Val Ser Val Asn
-15 -10 -5

Ala Met Ala Ala Asp Cys Ala Lys Gly Lys Ile Glu Phe Ser Lys Tyr
-1 1 5 10

Asn Glu Asp Asp Thr Phe Thr Val Lys Val Asp Gly Lys Glu Tyr Trp
15 20 25

Thr Ser Arg Trp Asn Leu Gln Pro Leu Leu Gln Ser Ala Gln Leu Thr
30 35 40 45

Gly Met Thr Val Thr Ile Lys Ser Ser Thr Cys Glu Ser Gly Ser Gly
50 55 60

Phe Ala Glu Val Gln Phe Asn Asn Asp
65 70

<210> 10
<211> 128
<212> PRT
<213> Homo sapiens

<400> 10

Met Ser Leu Arg Leu Asp Thr Thr Pro Ser Cys Asn Ser Ala Arg Pro
1 5 10 15

Leu His Ala Leu Gln Val Leu Leu Leu Leu Ser Leu Leu Leu Thr Ala

```
            20                  25                  30

Leu Ala Ser Ser Thr Lys Gly Gln Thr Lys Arg Asn Leu Ala Lys Gly
        35                  40                  45

Lys Glu Glu Ser Leu Asp Ser Asp Leu Tyr Ala Glu Leu Arg Cys Met
    50                  55                  60

Cys Ile Lys Thr Thr Ser Gly Ile His Pro Lys Asn Ile Gln Ser Leu
65                  70                  75                  80

Glu Val Ile Gly Lys Gly Thr His Cys Asn Gln Val Glu Val Ile Ala
                85                  90                  95

Thr Leu Lys Asp Gly Arg Lys Ile Cys Leu Asp Pro Asp Ala Pro Arg
            100                 105                 110

Ile Lys Lys Ile Val Gln Lys Lys Leu Ala Gly Asp Glu Ser Ala Asp
        115                 120                 125

<210>  11
<211>  142
<212>  PRT
<213>  Azotobacter vinelandii

<400>  11

Met Lys Ile Ser Ala Arg Asn Val Phe Lys Gly Thr Val Ser Ala Leu
1               5                   10                  15

Lys Glu Gly Ala Val Asn Ala Glu Val Asp Ile Leu Leu Gly Gly Gly
            20                  25                  30

Asp Lys Leu Ala Ala Val Val Thr Leu Glu Ser Ala Arg Ser Leu Gln
        35                  40                  45

Leu Ala Ala Gly Lys Glu Val Val Ala Val Val Lys Ala Pro Trp Val
    50                  55                  60

Leu Leu Met Thr Asp Ser Ser Gly Tyr Arg Leu Ser Ala Arg Asn Ile
65                  70                  75                  80

Leu Thr Gly Thr Val Lys Thr Ile Glu Thr Gly Ala Val Asn Ala Glu
                85                  90                  95

Val Thr Leu Ala Leu Gln Gly Gly Thr Glu Ile Thr Ser Met Val Thr
            100                 105                 110

Lys Glu Ala Val Ala Glu Leu Gly Leu Lys Pro Gly Ala Ser Ala Ser
        115                 120                 125

Ala Val Ile Lys Ala Ser Asn Val Ile Leu Gly Val Pro Ala
    130                 135                 140
```

<210> 12
<211> 505
<212> PRT
<213> Escherichia coli

<220>
<221> CONFLICT
<222> (30)..(30)

<220>
<221> domain OB-fold
<222> (31)..(149)

<400> 12

Met Ser Glu Gln His Ala Gln Gly Ala Asp Ala Val Val Asp Leu Asn
1 5 10 15

Asn Glu Leu Lys Thr Arg Arg Glu Lys Leu Ala Asn Leu Arg Glu Gln
20 25 30

Gly Ile Ala Phe Pro Asn Asp Phe Arg Arg Asp His Thr Ser Asp Gln
35 40 45

Leu His Ala Glu Phe Asp Gly Lys Glu Asn Glu Glu Leu Glu Ala Leu
50 55 60

Asn Ile Glu Val Ala Val Ala Gly Arg Met Met Thr Arg Arg Ile Met
65 70 75 80

Gly Lys Ala Ser Phe Val Thr Leu Gln Asp Val Gly Gly Arg Ile Gln
85 90 95

Leu Tyr Val Ala Arg Asp Asp Leu Pro Glu Gly Val Tyr Asn Glu Gln
100 105 110

Phe Lys Lys Trp Asp Leu Gly Asp Ile Leu Gly Ala Lys Gly Lys Leu
115 120 125

Phe Lys Thr Lys Thr Gly Glu Leu Ser Ile His Cys Thr Glu Leu Arg
130 135 140

Leu Leu Thr Lys Ala Leu Arg Pro Leu Pro Asp Lys Phe His Gly Leu
145 150 155 160

Gln Asp Gln Glu Ala Arg Tyr Arg Gln Arg Tyr Leu Asp Leu Ile Ser
165 170 175

Asn Asp Glu Ser Arg Asn Thr Phe Lys Val Arg Ser Gln Ile Leu Ser
180 185 190

Gly Ile Arg Gln Phe Met Val Asn Arg Gly Phe Met Glu Val Glu Thr
195 200 205

Pro Met Met Gln Val Ile Pro Gly Gly Ala Ala Ala Arg Pro Phe Ile
210 215 220

Thr His His Asn Ala Leu Asp Leu Asp Met Tyr Leu Arg Ile Ala Pro
225 230 235 240

Glu Leu Tyr Leu Lys Arg Leu Val Val Gly Gly Phe Glu Arg Val Phe
245 250 255

Glu Ile Asn Arg Asn Phe Arg Asn Glu Gly Ile Ser Val Arg His Asn
260 265 270

Pro Glu Phe Thr Met Met Glu Leu Tyr Met Ala Tyr Ala Asp Tyr Lys
275 280 285

Asp Leu Ile Glu Leu Thr Glu Ser Leu Phe Arg Thr Leu Ala Gln Asp
290 295 300

Ile Leu Gly Lys Thr Glu Val Thr Tyr Gly Asp Val Thr Leu Asp Phe
305 310 315 320

Gly Lys Pro Phe Glu Lys Leu Thr Met Arg Glu Ala Ile Lys Lys Tyr
325 330 335

Arg Ser Glu Thr Asp Met Ala Asp Leu Asp Asn Phe Asp Ser Ala Lys
340 345 350

Ala Ile Ala Glu Ser Ile Gly Ile His Val Glu Lys Ser Trp Gly Leu
355 360 365

Gly Arg Ile Val Thr Glu Ile Phe Glu Glu Val Ala Glu Ala His Leu
370 375 380

Ile Gln Pro Thr Phe Ile Thr Glu Tyr Pro Ala Glu Val Ser Pro Leu
385 390 395 400

Ala Arg Arg Asn Asp Val Asn Pro Glu Ile Thr Asp Arg Phe Glu Phe
405 410 415

Phe Ile Gly Gly Arg Glu Ile Gly Asn Gly Phe Ser Glu Leu Asn Asp
420 425 430

Ala Glu Asp Gln Ala Gln Arg Phe Leu Asp Gln Val Ala Ala Lys Asp
435 440 445

Ala Gly Asp Asp Glu Ala Met Phe Tyr Asp Glu Asp Tyr Val Thr Ala
450 455 460

Leu Glu His Gly Leu Pro Pro Thr Ala Gly Leu Gly Ile Gly Ile Asp
465 470 475 480

Arg Met Val Met Leu Phe Thr Asn Ser His Thr Ile Arg Asp Val Ile
485 490 495

Leu Phe Pro Ala Met Arg Pro Val Lys
500 505

<210> 13
<211> 585
<212> PRT
<213> Escherichia coli

<220>
<221> OB-fold domain
<222> (14)..(106)

<400> 13

Met Arg Thr Glu Tyr Cys Gly Gln Leu Arg Leu Ser His Val Gly Gln
1 5 10 15

Gln Val Thr Leu Cys Gly Trp Val Asn Arg Arg Arg Asp Leu Gly Ser
20 25 30

Leu Ile Phe Ile Asp Met Arg Asp Arg Glu Gly Ile Val Gln Val Phe
35 40 45

Phe Asp Pro Asp Arg Ala Asp Ala Leu Lys Leu Ala Ser Glu Leu Arg
50 55 60

Asn Glu Phe Cys Ile Gln Val Thr Gly Thr Val Arg Ala Arg Asp Glu
65 70 75 80

Lys Asn Ile Asn Arg Asp Met Ala Thr Gly Glu Ile Glu Val Leu Ala
85 90 95

Ser Ser Leu Thr Ile Ile Asn Arg Ala Asp Val Leu Pro Leu Asp Ser
100 105 110

Asn His Val Asn Thr Glu Glu Ala Arg Leu Lys Tyr Arg Tyr Leu Asp
115 120 125

Leu Arg Arg Pro Glu Met Ala Gln Arg Leu Lys Thr Arg Ala Lys Ile
130 135 140

Thr Ser Leu Val Arg Arg Phe Met Asp Asp His Gly Phe Leu Asp Ile
145 150 155 160

Glu Thr Pro Met Leu Thr Lys Ala Thr Pro Glu Gly Ala Arg Asp Tyr
165 170 175

Leu Val Pro Ser Arg Val His Lys Gly Lys Phe Tyr Ala Leu Pro Gln
180 185 190

```
Ser Pro Gln Leu Phe Lys Gln Leu Leu Met Met Ser Gly Phe Asp Arg
        195                 200                 205

Tyr Tyr Gln Ile Val Lys Cys Phe Arg Asp Glu Asp Leu Arg Ala Asp
    210                 215                 220

Arg Gln Pro Glu Phe Thr Gln Ile Asp Val Glu Thr Ser Phe Met Thr
225                 230                 235                 240

Ala Pro Gln Val Arg Glu Val Met Glu Ala Leu Val Arg His Leu Trp
                245                 250                 255

Leu Glu Val Lys Gly Val Asp Leu Gly Asp Phe Pro Val Met Thr Phe
            260                 265                 270

Ala Glu Ala Glu Arg Arg Tyr Gly Ser Asp Lys Pro Asp Leu Arg Asn
        275                 280                 285

Pro Met Glu Leu Thr Asp Val Ala Asp Leu Leu Lys Ser Val Glu Phe
    290                 295                 300

Ala Val Phe Ala Gly Pro Ala Asn Asp Pro Lys Gly Arg Val Ala Ala
305                 310                 315                 320

Leu Arg Val Pro Gly Gly Ala Ser Leu Thr Arg Lys Gln Ile Asp Glu
                325                 330                 335

Tyr Gly Asn Phe Val Lys Ile Tyr Gly Ala Lys Gly Leu Ala Tyr Ile
            340                 345                 350

Lys Val Asn Glu Arg Ala Lys Gly Leu Glu Gly Ile Asn Ser Pro Val
        355                 360                 365

Ala Lys Phe Leu Asn Ala Glu Ile Ile Glu Asp Ile Leu Asp Arg Thr
    370                 375                 380

Ala Ala Gln Asp Gly Asp Met Ile Phe Phe Gly Ala Asp Asn Lys Lys
385                 390                 395                 400

Ile Val Ala Asp Ala Met Gly Ala Leu Arg Leu Lys Val Gly Lys Asp
                405                 410                 415

Leu Gly Leu Thr Asp Glu Ser Lys Trp Ala Pro Leu Trp Val Ile Asp
            420                 425                 430

Phe Pro Met Phe Glu Asp Asp Gly Glu Gly Gly Leu Thr Ala Met His
        435                 440                 445

His Pro Phe Thr Ser Pro Lys Asp Met Thr Ala Ala Glu Leu Lys Ala
    450                 455                 460
```

Ala Pro Glu Asn Ala Val Ala Asn Ala Tyr Asp Met Val Ile Asn Gly
465                 470                 475                 480

Tyr Glu Val Gly Gly Gly Ser Val Arg Ile His Asn Gly Asp Met Gln
                485                 490                 495

Gln Thr Val Phe Gly Ile Leu Gly Ile Asn Glu Glu Glu Gln Arg Glu
            500                 505                 510

Lys Phe Gly Phe Leu Leu Asp Ala Leu Lys Tyr Gly Thr Pro Pro His
        515                 520                 525

Ala Gly Leu Ala Phe Gly Leu Asp Arg Leu Thr Met Leu Leu Thr Gly
    530                 535                 540

Thr Asp Asn Ile Arg Asp Val Ile Ala Phe Pro Lys Thr Thr Ala Ala
545                 550                 555                 560

Ala Cys Leu Met Thr Glu Ala Pro Ser Phe Ala Asn Pro Thr Ala Leu
                565                 570                 575

Ala Glu Leu Ser Ile Gln Val Val Lys
            580                 585

<210> 14
<211> 438
<212> PRT
<213> Thermococcus kodakaraensis

<220>
<221> domain OB-fold
<222> (15)..(100)

<400> 14

Met Tyr Arg Thr His Tyr Ser Ser Glu Ile Thr Glu Glu Leu Asn Gly
1               5                   10                  15

Gln Lys Val Lys Val Ala Gly Trp Val Trp Glu Val Lys Asp Leu Gly
            20                  25                  30

Gly Ile Lys Phe Leu Trp Ile Arg Asp Arg Asp Gly Ile Val Gln Ile
        35                  40                  45

Thr Ala Pro Lys Lys Lys Val Asp Pro Glu Leu Phe Lys Leu Ile Pro
    50                  55                  60

Lys Leu Arg Ser Glu Asp Val Val Ala Val Glu Gly Val Val Asn Phe
65                  70                  75                  80

Thr Pro Lys Ala Lys Leu Gly Phe Glu Ile Leu Pro Glu Lys Ile Val
                85                  90                  95

Val Leu Asn Arg Ala Glu Thr Pro Leu Pro Leu Asp Pro Thr Gly Lys
100 105 110

Val Lys Ala Glu Leu Asp Thr Arg Leu Asp Asn Arg Phe Met Asp Leu
115 120 125

Arg Arg Pro Glu Val Met Ala Ile Phe Lys Ile Arg Ser Ser Val Phe
130 135 140

Lys Ala Val Arg Asp Phe Phe His Glu Asn Gly Phe Ile Glu Ile His
145 150 155 160

Thr Pro Lys Ile Ile Ala Thr Ala Thr Glu Gly Gly Thr Glu Leu Phe
165 170 175

Pro Met Lys Tyr Phe Glu Glu Asp Ala Phe Leu Ala Gln Ser Pro Gln
180 185 190

Leu Tyr Lys Gln Ile Met Met Ala Ser Gly Leu Asp Arg Val Tyr Glu
195 200 205

Ile Ala Pro Ile Phe Arg Ala Glu Glu His Asn Thr Thr Arg His Leu
210 215 220

Asn Glu Ala Trp Ser Ile Asp Ser Glu Met Ala Phe Ile Glu Asp Glu
225 230 235 240

Glu Glu Val Met Ser Phe Leu Glu Arg Leu Val Ala His Ala Ile Asn
245 250 255

Tyr Val Arg Glu His Asn Ala Lys Glu Leu Asp Ile Leu Asn Phe Glu
260 265 270

Leu Glu Glu Pro Lys Leu Pro Phe Pro Arg Val Ser Tyr Asp Lys Ala
275 280 285

Leu Glu Ile Leu Gly Asp Leu Gly Lys Glu Ile Pro Trp Gly Glu Asp
290 295 300

Ile Asp Thr Glu Gly Glu Arg Leu Leu Gly Lys Tyr Met Met Glu Asn
305 310 315 320

Glu Asn Ala Pro Leu Tyr Phe Leu Tyr Gln Tyr Pro Ser Glu Ala Lys
325 330 335

Pro Phe Tyr Ile Met Lys Tyr Asp Asn Lys Pro Glu Ile Cys Arg Ala
340 345 350

Phe Asp Leu Glu Tyr Arg Gly Val Glu Ile Ser Ser Gly Gly Gln Arg
355 360 365

Glu His Arg His Asp Ile Leu Val Glu Gln Ile Lys Glu Lys Gly Leu
370 375 380

Asn Pro Glu Ser Phe Glu Phe Tyr Leu Lys Ala Phe Arg Tyr Gly Met
385 390 395 400

Pro Pro His Gly Gly Phe Gly Leu Gly Ala Glu Arg Leu Ile Lys Gln
405 410 415

Met Leu Asp Leu Pro Asn Ile Arg Glu Val Ile Leu Phe Pro Arg Asp
420 425 430

Arg Arg Arg Leu Thr Pro
435

<210> 15
<211> 504
<212> PRT
<213> Escherichia coli

<220>
<221> domain OB-fold
<222> (65)..(150)

<400> 15

Ser Glu Gln Glu Thr Arg Gly Ala Asn Glu Ala Ile Asp Phe Asn Asp
1 5 10 15

Glu Leu Arg Asn Arg Arg Glu Lys Leu Ala Ala Leu Arg Gln Gln Gly
20 25 30

Val Ala Phe Pro Asn Asp Phe Arg Arg Asp His Thr Ser Asp Gln Leu
35 40 45

His Glu Glu Phe Asp Ala Lys Asp Asn Gln Glu Leu Glu Ser Leu Asn
50 55 60

Ile Glu Val Ser Val Ala Gly Arg Met Met Thr Arg Arg Ile Met Gly
65 70 75 80

Lys Ala Ser Phe Val Thr Leu Gln Asp Val Gly Gly Arg Ile Gln Leu
85 90 95

Tyr Val Ala Arg Asp Ser Leu Pro Glu Gly Val Tyr Asn Asp Gln Phe
100 105 110

Lys Lys Trp Asp Leu Gly Asp Ile Ile Gly Ala Arg Gly Thr Leu Phe
115 120 125

Lys Thr Gln Thr Gly Glu Leu Ser Ile His Cys Thr Glu Leu Arg Leu
130 135 140

```
Leu Thr Lys Ala Leu Arg Pro Leu Pro Asp Lys Phe His Gly Leu Gln
145                 150                 155                 160

Asp Gln Glu Val Arg Tyr Arg Gln Arg Tyr Leu Asp Leu Ile Ala Asn
                165                 170                 175

Asp Lys Ser Arg Gln Thr Phe Val Val Arg Ser Lys Ile Leu Ala Ala
            180                 185                 190

Ile Arg Gln Phe Met Val Ala Arg Gly Phe Met Glu Val Glu Thr Pro
        195                 200                 205

Met Met Gln Val Ile Pro Gly Gly Ala Ser Ala Arg Pro Phe Ile Thr
    210                 215                 220

His His Asn Ala Leu Asp Leu Asp Met Tyr Leu Arg Ile Ala Pro Glu
225                 230                 235                 240

Leu Tyr Leu Lys Arg Leu Val Val Gly Gly Phe Glu Arg Val Phe Glu
                245                 250                 255

Ile Asn Arg Asn Phe Arg Asn Glu Gly Ile Ser Val Arg His Asn Pro
            260                 265                 270

Glu Phe Thr Met Met Glu Leu Tyr Met Ala Tyr Ala Asp Tyr His Asp
        275                 280                 285

Leu Ile Glu Leu Thr Glu Ser Leu Phe Arg Thr Leu Ala Gln Glu Val
    290                 295                 300

Leu Gly Thr Thr Lys Val Thr Tyr Gly Glu His Val Phe Asp Phe Gly
305                 310                 315                 320

Lys Pro Phe Glu Lys Leu Thr Met Arg Glu Ala Ile Lys Lys Tyr Arg
                325                 330                 335

Pro Glu Thr Asp Met Ala Asp Leu Asp Asn Phe Asp Ala Ala Lys Ala
            340                 345                 350

Leu Ala Glu Ser Ile Gly Ile Thr Val Glu Lys Ser Trp Gly Leu Gly
        355                 360                 365

Arg Ile Val Thr Glu Ile Phe Asp Glu Val Ala Glu Ala His Leu Ile
    370                 375                 380

Gln Pro Thr Phe Ile Thr Glu Tyr Pro Ala Glu Val Ser Pro Leu Ala
385                 390                 395                 400

Arg Arg Asn Asp Val Asn Pro Glu Ile Thr Asp Arg Phe Glu Phe Phe
                405                 410                 415
```

Ile Gly Gly Arg Glu Ile Gly Asn Gly Phe Ser Glu Leu Asn Asp Ala
420 425 430

Glu Asp Gln Ala Glu Arg Phe Gln Glu Gln Val Asn Ala Lys Ala Ala
435 440 445

Gly Asp Asp Glu Ala Met Phe Tyr Asp Glu Asp Tyr Val Thr Ala Leu
450 455 460

Glu Tyr Gly Leu Pro Pro Thr Ala Gly Leu Gly Ile Gly Ile Asp Arg
465 470 475 480

Met Ile Met Leu Phe Thr Asn Ser His Thr Ile Arg Asp Val Ile Leu
485 490 495

Phe Pro Ala Met Arg Pro Gln Lys
500

<210> 16
<211> 270
<212> PRT
<213> Homo sapiens

<220>
<221> domain OB-fold
<222> (72)..(149)

<400> 16

Met Trp Asn Ser Gly Phe Glu Ser Tyr Gly Ser Ser Ser Tyr Gly Gly
1 5 10 15

Ala Gly Gly Tyr Thr Gln Ser Pro Gly Gly Phe Gly Ser Pro Ala Pro
20 25 30

Ser Gln Ala Glu Lys Lys Ser Arg Ala Arg Ala Gln His Ile Val Pro
35 40 45

Cys Thr Ile Ser Gln Leu Leu Ser Ala Thr Leu Val Asp Glu Val Phe
50 55 60

Arg Ile Gly Asn Val Glu Ile Ser Gln Val Thr Ile Val Gly Ile Ile
65 70 75 80

Arg His Ala Glu Lys Ala Pro Thr Asn Ile Val Tyr Lys Ile Asp Asp
85 90 95

Met Thr Ala Ala Pro Met Asp Val Arg Gln Trp Val Asp Thr Asp Asp
100 105 110

Thr Ser Ser Glu Asn Thr Val Val Pro Pro Glu Thr Tyr Val Lys Val
115 120 125

Ala Gly His Leu Arg Ser Phe Gln Asn Lys Lys Ser Leu Val Ala Phe
    130                 135                 140

Lys Ile Met Pro Leu Glu Asp Met Asn Glu Phe Thr Thr His Ile Leu
145                 150                 155                 160

Glu Val Ile Asn Ala His Met Val Leu Ser Lys Ala Asn Ser Gln Pro
                165                 170                 175

Ser Ala Gly Arg Ala Pro Ile Ser Asn Pro Gly Met Ser Glu Ala Gly
            180                 185                 190

Asn Phe Gly Gly Asn Ser Phe Met Pro Ala Asn Gly Leu Thr Val Ala
        195                 200                 205

Gln Asn Gln Val Leu Asn Leu Ile Lys Ala Cys Pro Arg Pro Glu Gly
    210                 215                 220

Leu Asn Phe Gln Asp Leu Lys Asn Gln Leu Lys His Met Ser Val Ser
225                 230                 235                 240

Ser Ile Lys Gln Ala Val Asp Phe Leu Ser Asn Glu Gly His Ile Tyr
                245                 250                 255

Ser Thr Val Asp Asp Asp His Phe Lys Ser Thr Asp Ala Glu
            260                 265                 270

<210> 17
<211> 121
<212> PRT
<213> Homo sapiens

<220>
<221> domain OB-fold
<222> (21)..(87)

<400> 17

Met Val Asp Met Met Asp Leu Pro Arg Ser Arg Ile Asn Ala Gly Met
1               5                   10                  15

Leu Ala Gln Phe Ile Asp Lys Pro Val Cys Phe Val Gly Arg Leu Glu
            20                  25                  30

Lys Ile His Pro Thr Gly Lys Met Phe Ile Leu Ser Asp Gly Glu Gly
        35                  40                  45

Lys Asn Gly Thr Ile Glu Leu Met Glu Pro Leu Asp Glu Glu Ile Ser
    50                  55                  60

Gly Ile Val Glu Val Val Gly Arg Val Thr Ala Lys Ala Thr Ile Leu
65                  70                  75                  80

Cys Thr Ser Tyr Val Gln Phe Lys Glu Asp Ser His Pro Phe Asp Leu
85 90 95

Gly Leu Tyr Asn Glu Ala Val Lys Ile Ile His Asp Phe Pro Gln Phe
100 105 110

Tyr Pro Leu Gly Ile Val Gln His Asp
115 120

<210> 18
<211> 616
<212> PRT
<213> Homo sapiens

<220>
<221> CONFLICT
<222> (177)..(180)

<220>
<221> domain OB-fold
<222> (195)..(290)

<400> 18

Met Val Gly Gln Leu Ser Glu Gly Ala Ile Ala Ala Ile Met Gln Lys
1 5 10 15

Gly Asp Thr Asn Ile Lys Pro Ile Leu Gln Val Ile Asn Ile Arg Pro
20 25 30

Ile Thr Thr Gly Asn Ser Pro Pro Arg Tyr Arg Leu Leu Met Ser Asp
35 40 45

Gly Leu Asn Thr Leu Ser Ser Phe Met Leu Ala Thr Gln Leu Asn Pro
50 55 60

Leu Val Glu Glu Glu Gln Leu Ser Ser Asn Cys Val Cys Gln Ile His
65 70 75 80

Arg Phe Ile Val Asn Thr Leu Lys Asp Gly Arg Arg Val Val Ile Leu
85 90 95

Met Glu Leu Glu Val Leu Lys Ser Ala Glu Ala Val Gly Val Lys Ile
100 105 110

Gly Asn Pro Val Pro Tyr Asn Glu Gly Leu Gly Gln Pro Gln Val Ala
115 120 125

Pro Pro Ala Pro Ala Ala Ser Pro Ala Ala Ser Ser Arg Pro Gln Pro
130 135 140

Gln Asn Gly Ser Ser Gly Met Gly Ser Thr Val Ser Lys Ala Tyr Gly
145 150 155 160

```
Ala Ser Lys Thr Phe Gly Lys Ala Ala Gly Pro Ser Leu Ser His Thr
                165                 170                 175

Ser Gly Gly Thr Gln Ser Lys Val Val Pro Ile Ala Ser Leu Thr Pro
            180                 185                 190

Tyr Gln Ser Lys Trp Thr Ile Cys Ala Arg Val Thr Asn Lys Ser Gln
        195                 200                 205

Ile Arg Thr Trp Ser Asn Ser Arg Gly Glu Gly Lys Leu Phe Ser Leu
    210                 215                 220

Glu Leu Val Asp Glu Ser Gly Glu Ile Arg Ala Thr Ala Phe Asn Glu
225                 230                 235                 240

Gln Val Asp Lys Phe Phe Pro Leu Ile Glu Val Asn Lys Val Tyr Tyr
                245                 250                 255

Phe Ser Lys Gly Thr Leu Lys Ile Ala Asn Lys Gln Phe Thr Ala Val
            260                 265                 270

Lys Asn Asp Tyr Glu Met Thr Phe Asn Asn Glu Thr Ser Val Met Pro
        275                 280                 285

Cys Glu Asp Asp His His Leu Pro Thr Val Gln Phe Asp Phe Thr Gly
    290                 295                 300

Ile Asp Asp Leu Glu Asn Lys Ser Lys Asp Ser Leu Val Asp Ile Ile
305                 310                 315                 320

Gly Ile Cys Lys Ser Tyr Glu Asp Ala Thr Lys Ile Thr Val Arg Ser
                325                 330                 335

Asn Asn Arg Glu Val Ala Lys Arg Asn Ile Tyr Leu Met Asp Thr Ser
            340                 345                 350

Gly Lys Val Val Thr Ala Thr Leu Trp Gly Glu Asp Ala Asp Lys Phe
        355                 360                 365

Asp Gly Ser Arg Gln Pro Val Leu Ala Ile Lys Gly Ala Arg Val Ser
    370                 375                 380

Asp Phe Gly Gly Arg Ser Leu Ser Val Leu Ser Ser Ser Thr Ile Ile
385                 390                 395                 400

Ala Asn Pro Asp Ile Pro Glu Ala Tyr Lys Leu Arg Gly Trp Phe Asp
                405                 410                 415

Ala Glu Gly Gln Ala Leu Asp Gly Val Ser Ile Ser Asp Leu Lys Ser
            420                 425                 430
```

Gly Gly Val Gly Gly Ser Asn Thr Asn Trp Lys Thr Leu Tyr Glu Val
        435                 440                 445

Lys Ser Glu Asn Leu Gly Gln Gly Asp Lys Pro Asp Tyr Phe Ser Ser
    450                 455                 460

Val Ala Thr Val Val Tyr Leu Arg Lys Glu Asn Cys Met Tyr Gln Ala
465                 470                 475                 480

Cys Pro Thr Gln Asp Cys Asn Lys Lys Val Ile Asp Gln Gln Asn Gly
                485                 490                 495

Leu Tyr Arg Cys Glu Lys Cys Asp Thr Glu Phe Pro Asn Phe Lys Tyr
            500                 505                 510

Arg Met Ile Leu Ser Val Asn Ile Ala Asp Phe Gln Glu Asn Gln Trp
        515                 520                 525

Val Thr Cys Phe Gln Glu Ser Ala Glu Ala Ile Leu Gly Gln Asn Ala
    530                 535                 540

Ala Tyr Leu Gly Glu Leu Lys Asp Lys Asn Glu Gln Ala Phe Glu Glu
545                 550                 555                 560

Val Phe Gln Asn Ala Asn Phe Arg Ser Phe Ile Phe Arg Val Arg Val
                565                 570                 575

Lys Val Glu Thr Tyr Asn Asp Glu Ser Arg Ile Lys Ala Thr Val Met
            580                 585                 590

Asp Val Lys Pro Val Asp Tyr Arg Glu Tyr Gly Arg Arg Leu Val Met
        595                 600                 605

Ser Ile Arg Arg Ser Ala Leu Met
    610                 615

<210> 19
<211> 495
<212> PRT
<213> Oxytricha nova

<220>
<221> domain OB-fold
<222> (221)..(299)

<400> 19

Met Ser Thr Ala Ala Lys Gln Asn Arg Ser Thr Ser Arg Val Ser Lys
1               5                   10                  15

Lys Lys Thr Ala Ala Pro Lys Glu Gly Ala Ala Lys Lys Ser Asp Lys
            20                  25                  30

Gly His Lys Tyr Glu Tyr Val Glu Leu Ala Lys Ala Ser Leu Thr Ser
35 40 45

Ala Gln Pro Gln His Phe Tyr Ala Val Val Ile Asp Ala Thr Phe Pro
50 55 60

Tyr Lys Thr Asn Gln Glu Arg Tyr Ile Cys Ser Leu Lys Ile Val Asp
65 70 75 80

Pro Thr Leu Tyr Leu Lys Gln Gln Lys Gly Ala Gly Asp Ala Ser Asp
85 90 95

Tyr Ala Thr Leu Val Leu Tyr Ala Lys Arg Phe Glu Asp Leu Pro Ile
100 105 110

Ile His Arg Ala Gly Asp Ile Ile Arg Val His Arg Ala Thr Leu Arg
115 120 125

Leu Tyr Asn Gly Gln Arg Gln Phe Asn Ala Asn Val Phe Tyr Ser Ser
130 135 140

Ser Trp Ala Leu Phe Ser Thr Asp Lys Arg Ser Val Thr Gln Glu Ile
145 150 155 160

Asn Asn Gln Asp Ala Val Ser Asp Thr Thr Pro Phe Ser Phe Ser Ser
165 170 175

Lys His Ala Thr Ile Glu Lys Asn Glu Ile Ser Ile Leu Gln Asn Leu
180 185 190

Arg Lys Trp Ala Asn Gln Tyr Phe Ser Ser Tyr Ser Val Ile Ser Ser
195 200 205

Asp Met Tyr Thr Ala Leu Asn Lys Ala Gln Ala Gln Lys Gly Asp Phe
210 215 220

Asp Val Val Ala Lys Ile Leu Gln Val His Glu Leu Asp Glu Tyr Thr
225 230 235 240

Asn Glu Leu Lys Leu Lys Asp Ala Ser Gly Gln Val Phe Tyr Thr Leu
245 250 255

Ser Leu Lys Leu Lys Phe Pro His Val Arg Thr Gly Glu Val Val Arg
260 265 270

Ile Arg Ser Ala Thr Tyr Asp Glu Thr Ser Thr Gln Lys Lys Val Leu
275 280 285

Ile Leu Ser His Tyr Ser Asn Ile Ile Thr Phe Ile Gln Ser Ser Lys
290 295 300

Leu Ala Lys Glu Leu Arg Ala Lys Ile Gln Asp Asp His Ser Val Glu
305 310 315 320

Val Ala Ser Leu Lys Lys Asn Val Ser Leu Asn Ala Val Val Leu Thr
325 330 335

Glu Val Asp Lys Lys His Ala Ala Leu Pro Ser Thr Ser Leu Gln Asp
340 345 350

Leu Phe His His Ala Asp Ser Asp Lys Glu Leu Gln Ala Gln Asp Thr
355 360 365

Phe Arg Thr Gln Phe Tyr Val Thr Lys Ile Glu Pro Ser Asp Val Lys
370 375 380

Glu Trp Val Lys Gly Tyr Asp Arg Lys Thr Lys Lys Ser Ser Ser Leu
385 390 395 400

Lys Gly Ala Ser Gly Lys Gly Asp Asn Ile Phe Gln Val Gln Phe Leu
405 410 415

Val Lys Asp Ala Ser Thr Gln Leu Asn Asn Asn Thr Tyr Arg Val Leu
420 425 430

Leu Tyr Thr Gln Asp Gly Leu Gly Ala Asn Phe Phe Asn Val Lys Ala
435 440 445

Asp Asn Leu His Lys Asn Ala Asp Ala Arg Lys Lys Leu Glu Asp Ser
450 455 460

Ala Glu Leu Leu Thr Lys Phe Asn Ser Tyr Val Asp Ala Val Val Glu
465 470 475 480

Arg Arg Asn Gly Phe Tyr Leu Ile Lys Asp Thr Lys Leu Ile Tyr
485 490 495

<210> 20
<211> 260
<212> PRT
<213> Oxytricha nova

<220>
<221> domain OB-fold
<222> (36)..(129)

<400> 20

Met Ser Lys Gly Ala Ser Ala Pro Gln Gln Gln Ser Ala Phe Lys Gln
1 5 10 15

Leu Tyr Thr Glu Leu Phe Asn Asn Glu Gly Asp Phe Ser Lys Val Ser
20 25 30

Ser Asn Leu Lys Lys Pro Leu Lys Cys Tyr Val Lys Glu Ser Tyr Pro
35 40 45

His Phe Leu Val Thr Asp Gly Tyr Phe Phe Val Ala Pro Tyr Phe Thr
50 55 60

Lys Glu Ala Val Asn Glu Phe His Ala Lys Phe Pro Asn Val Asn Ile
65 70 75 80

Val Asp Leu Thr Asp Lys Val Ile Val Ile Asn Asn Trp Ser Leu Glu
85 90 95

Leu Arg Arg Val Asn Ser Ala Glu Val Phe Thr Ser Tyr Ala Asn Leu
100 105 110

Glu Ala Arg Leu Ile Val His Ser Phe Lys Pro Asn Leu Gln Glu Arg
115 120 125

Leu Asn Pro Thr Arg Tyr Pro Val Asn Leu Phe Arg Asp Asp Glu Phe
130 135 140

Lys Thr Thr Ile Gln His Phe Arg His Thr Ala Leu Gln Ala Ala Ile
145 150 155 160

Asn Lys Thr Val Lys Gly Asp Asn Leu Val Asp Ile Ser Lys Val Ala
165 170 175

Asp Ala Ala Gly Lys Lys Gly Lys Val Asp Ala Gly Ile Val Lys Ala
180 185 190

Ser Ala Ser Lys Gly Asp Glu Phe Ser Asp Phe Ser Phe Lys Glu Gly
195 200 205

Asn Thr Ala Thr Leu Lys Ile Ala Asp Ile Phe Val Gln Glu Lys Gly
210 215 220

Lys Asp Ala Leu Asn Lys Ala Ala Asp His Thr Asp Gly Ala Lys Val
225 230 235 240

Lys Gly Gly Ala Lys Gly Lys Gly Lys Ala Ala Ala Lys Ala Ala Lys
245 250 255

Gly Lys Lys Leu
260

<210> 21
<211> 148
<212> PRT
<213> Homo sapiens

<220>
<221> domain OB-fold
<222> (32)..(148)

<220>
<221> CONFLICT
<222> (105)..(105)

<400> 21

```
Met Phe Arg Arg Pro Val Leu Gln Val Leu Arg Gln Phe Val Arg His
1               5                   10                  15

Glu Ser Glu Thr Thr Thr Ser Leu Val Leu Glu Arg Ser Leu Asn Arg
            20                  25                  30

Val His Leu Leu Gly Arg Val Gly Gln Asp Pro Val Leu Arg Gln Val
        35                  40                  45

Glu Gly Lys Asn Pro Val Thr Ile Phe Ser Leu Ala Thr Asn Glu Met
    50                  55                  60

Trp Arg Ser Gly Asp Ser Glu Val Tyr Gln Leu Gly Asp Val Ser Gln
65                  70                  75                  80

Lys Thr Thr Trp His Arg Ile Ser Val Phe Arg Pro Gly Leu Arg Asp
                85                  90                  95

Val Ala Tyr Gln Tyr Val Lys Lys Arg Ser Arg Ile Tyr Leu Glu Gly
            100                 105                 110

Lys Ile Asp Tyr Gly Glu Tyr Met Asp Lys Asn Asn Val Arg Arg Gln
        115                 120                 125

Ala Thr Thr Ile Ile Ala Asp Asn Ile Ile Phe Leu Ser Asp Gln Thr
    130                 135                 140

Lys Glu Lys Glu
145
```

<210> 22
<211> 133
<212> PRT
<213> Bordetella pertussis

<220>
<221> domain OB-fold
<222> (38)..(126)

<400> 22

```
Met Gln Arg Gln Ala Gly Leu Pro Leu Lys Ala Asn Pro Met His Thr
1               5                   10                  15

Ile Ala Ser Ile Leu Leu Ser Val Leu Gly Ile Tyr Ser Pro Ala Asp
            20                  25                  30
```

Val Ala Gly Leu Pro Thr His Leu Tyr Lys Asn Phe Thr Val Gln Glu
35 40 45

Leu Ala Leu Lys Leu Lys Gly Lys Asn Gln Glu Phe Cys Leu Thr Ala
50 55 60

Phe Met Ser Gly Arg Ser Leu Val Arg Ala Cys Leu Ser Asp Ala Gly
65 70 75 80

His Glu His Asp Thr Trp Phe Asp Thr Met Leu Gly Phe Ala Ile Ser
85 90 95

Ala Tyr Ala Leu Lys Ser Arg Ile Ala Leu Thr Val Glu Asp Ser Pro
100 105 110

Tyr Pro Gly Thr Pro Gly Asp Leu Leu Glu Leu Gln Ile Cys Pro Leu
115 120 125

Asn Gly Tyr Cys Glu
130

<210> 23
<211> 152
<212> PRT
<213> Bordetella pertussis

<220>
<221> domain OB-fold
<222> (46)..(144)

<400> 23

Met Leu Arg Arg Phe Pro Thr Arg Thr Thr Ala Pro Gly Gln Gly Gly
1 5 10 15

Ala Arg Arg Ser Arg Val Arg Ala Leu Ala Trp Leu Leu Ala Ser Gly
20 25 30

Ala Met Thr His Leu Ser Pro Ala Leu Ala Asp Val Pro Tyr Val Leu
35 40 45

Val Lys Thr Asn Met Val Val Thr Ser Val Ala Met Lys Pro Tyr Glu
50 55 60

Val Thr Pro Thr Arg Met Leu Val Cys Gly Ile Ala Ala Lys Leu Gly
65 70 75 80

Ala Ala Ala Ser Ser Pro Asp Ala His Val Pro Phe Cys Phe Gly Lys
85 90 95

Asp Leu Lys Arg Pro Gly Ser Ser Pro Met Glu Val Met Leu Arg Ala
100 105 110

Val Phe Met Gln Gln Arg Pro Leu Arg Met Phe Leu Gly Pro Lys Gln
115 120 125

Leu Thr Phe Glu Gly Lys Pro Ala Leu Glu Leu Ile Arg Met Val Glu
130 135 140

Cys Ser Gly Lys Gln Asp Cys Pro
145 150

<210> 24
<211> 80
<212> PRT
<213> Vibrio cholerae

<220>
<221> domain OB-fold
<222> (15)..(80)

<400> 24

Met Thr Pro Gln Asn Ile Thr Asp Leu Cys Ala Glu Tyr His Asn Thr
1 5 10 15

Gln Ile His Thr Leu Asn Asp Lys Ile Phe Ser Tyr Thr Glu Ser Leu
20 25 30

Ala Gly Lys Arg Glu Met Ala Ile Ile Thr Phe Lys Asn Gly Ala Thr
35 40 45

Phe Gln Val Glu Val Pro Gly Ser Gln His Ile Asp Ser Gln Lys Lys
50 55 60

Ala Ile Glu Arg Met Lys Asp Thr Leu Arg Ile Ala Tyr Leu Thr Glu
65 70 75 80

<210> 25
<211> 122
<212> PRT
<213> Escherichia coli

<220>
<221> domain OB-fold
<222> (37)..(122)

<400> 25

Met Ser Phe Lys Lys Ile Ile Lys Ala Phe Val Ile Met Ala Ala Leu
1 5 10 15

Val Ser Val Gln Ala His Ala Gly Ala Ser Gln Phe Phe Lys Asp Asn
20 25 30

Cys Asn Arg Thr Thr Ala Ser Leu Val Glu Gly Val Glu Leu Thr Lys
35 40 45

Tyr Ile Ser Asp Ile Asn Asn Asn Thr Asp Gly Met Tyr Val Val Ser
50 55 60

Ser Thr Gly Gly Val Trp Arg Ile Ser Arg Ala Lys Asp Tyr Pro Asp
65 70 75 80

Asn Val Met Thr Ala Glu Met Arg Lys Ile Ala Met Ala Ala Val Leu
85 90 95

Ser Gly Met Arg Val Asn Met Cys Ala Ser Pro Ala Ser Ser Pro Asn
100 105 110

Val Ile Trp Ala Ile Glu Leu Glu Ala Glu
115 120

<210> 26
<211> 87
<212> PRT
<213> Escherichia coli

<220>
<221> domain OB-fold
<222> (20)..(87)

<220>
<221> CONFLICT
<222> (83)..(83)

<220>
<221> CONFLICT
<222> (85)..(85)

<400> 26

Met Lys Lys Met Phe Ile Ala Val Leu Phe Ala Leu Val Ser Val Asn
1 5 10 15

Ala Met Ala Ala Asp Cys Ala Lys Gly Lys Ile Glu Phe Ser Lys Tyr
20 25 30

Asn Glu Asp Asn Thr Phe Thr Val Lys Val Ser Gly Arg Glu Tyr Trp
35 40 45

Thr Asn Arg Trp Asn Leu Gln Pro Leu Leu Gln Ser Ala Gln Leu Thr
50 55 60

Gly Met Thr Val Thr Ile Ile Ser Asn Thr Cys Ser Ser Gly Ser Gly
65 70 75 80

Phe Ala Gln Val Lys Phe Asn
85

<210> 27
<211> 220

<212> PRT
<213> Homo sapiens

<220>
<221> domain OB-fold
<222> (41)..(133)

<400> 27

```
Met Gly Ala Ala Ala Arg Thr Leu Arg Leu Ala Leu Gly Leu Leu Leu
1               5                   10                  15

Leu Ala Thr Leu Leu Arg Pro Ala Asp Ala Cys Ser Cys Ser Pro Val
            20                  25                  30

His Pro Gln Gln Ala Phe Cys Asn Ala Asp Val Val Ile Arg Ala Lys
        35                  40                  45

Ala Val Ser Glu Lys Glu Val Asp Ser Gly Asn Asp Ile Tyr Gly Asn
    50                  55                  60

Pro Ile Lys Arg Ile Gln Tyr Glu Ile Lys Gln Ile Lys Met Phe Lys
65                  70                  75                  80

Gly Pro Glu Lys Asp Ile Glu Phe Ile Tyr Thr Ala Pro Ser Ser Ala
                85                  90                  95

Val Cys Gly Val Ser Leu Asp Val Gly Gly Lys Lys Glu Tyr Leu Ile
            100                 105                 110

Ala Gly Lys Ala Glu Gly Asp Gly Lys Met His Ile Thr Leu Cys Asp
        115                 120                 125

Phe Ile Val Pro Trp Asp Thr Leu Ser Thr Thr Gln Lys Lys Ser Leu
    130                 135                 140

Asn His Arg Tyr Gln Met Gly Cys Glu Cys Lys Ile Thr Arg Cys Pro
145                 150                 155                 160

Met Ile Pro Cys Tyr Ile Ser Ser Pro Asp Glu Cys Leu Trp Met Asp
                165                 170                 175

Trp Val Thr Glu Lys Asn Ile Asn Gly His Gln Ala Lys Phe Phe Ala
            180                 185                 190

Cys Ile Lys Arg Ser Asp Gly Ser Cys Ala Trp Tyr Arg Gly Ala Ala
        195                 200                 205

Pro Pro Lys Gln Glu Phe Leu Asp Ile Glu Asp Pro
    210                 215                 220
```

<210> 28
<211> 235

<212> PRT
<213> Streptococcus pyogenes

<220>
<221> domain OB-fold
<222> (48)..(120)

<400> 28

Met Lys Lys Ile Asn Ile Ile Lys Ile Val Phe Ile Ile Thr Val Ile
1 5 10 15

Leu Ile Ser Thr Ile Ser Pro Ile Ile Lys Ser Asp Ser Lys Lys Asp
20 25 30

Ile Ser Asn Val Lys Ser Asp Leu Leu Tyr Ala Tyr Thr Ile Thr Pro
35 40 45

Tyr Asp Tyr Lys Asp Cys Arg Val Asn Phe Ser Thr Thr His Thr Leu
50 55 60

Asn Ile Asp Thr Gln Lys Tyr Arg Gly Lys Asp Tyr Tyr Ile Ser Ser
65 70 75 80

Glu Met Ser Tyr Glu Ala Ser Gln Lys Phe Lys Arg Asp Asp His Val
85 90 95

Asp Val Phe Gly Leu Phe Tyr Ile Leu Asn Ser His Thr Gly Glu Tyr
100 105 110

Ile Tyr Gly Gly Ile Thr Pro Ala Gln Asn Asn Lys Val Asn His Lys
115 120 125

Leu Leu Gly Asn Leu Phe Ile Ser Gly Glu Ser Gln Gln Asn Leu Asn
130 135 140

Asn Lys Ile Ile Leu Glu Lys Asp Ile Val Thr Phe Gln Glu Ile Asp
145 150 155 160

Phe Lys Ile Arg Lys Tyr Leu Met Asp Asn Tyr Lys Ile Tyr Asp Ala
165 170 175

Thr Ser Pro Tyr Val Ser Gly Arg Ile Glu Ile Gly Thr Lys Asp Gly
180 185 190

Lys His Glu Gln Ile Asp Leu Phe Asp Ser Pro Asn Glu Gly Thr Arg
195 200 205

Ser Asp Ile Phe Ala Lys Tyr Lys Asp Asn Arg Ile Ile Asn Met Lys
210 215 220

Asn Phe Ser His Phe Asp Ile Tyr Leu Glu Lys
225 230 235

<210> 29
<211> 250
<212> PRT
<213> Staphylococcus aureus

<220>
<221> domain OB-fold
<222> (41)..(132)

<400> 29

Ile Leu Val Ile Ser Thr Pro Asn Val Leu Ala Glu Ser Gln Pro Asp
1 5 10 15

Pro Lys Pro Asp Glu Leu His Lys Ser Ser Lys Phe Thr Gly Leu Met
20 25 30

Glu Asn Met Lys Val Leu Tyr Asp Asp Asn His Val Ser Ala Ile Asn
35 40 45

Val Lys Ser Ile Asp Gln Phe Leu Tyr Phe Asp Leu Ile Tyr Ser Ile
50 55 60

Lys Asp Thr Lys Leu Gly Asn Tyr Asp Asn Val Arg Val Glu Phe Lys
65 70 75 80

Asn Lys Asp Leu Ala Asp Lys Tyr Lys Asp Lys Tyr Val Asp Val Phe
85 90 95

Gly Ala Asn Tyr Tyr Tyr Gln Cys Tyr Phe Ser Lys Lys Thr Asn Asp
100 105 110

Ile Asn Ser His Gln Thr Asp Lys Arg Lys Thr Cys Met Tyr Gly Gly
115 120 125

Val Thr Glu His Asn Gly Asn Gln Leu Asp Lys Tyr Arg Ser Ile Thr
130 135 140

Val Arg Val Phe Glu Asp Gly Lys Asn Leu Leu Ser Phe Asp Val Gln
145 150 155 160

Thr Asn Lys Lys Lys Val Thr Ala Gln Glu Leu Asp Tyr Leu Thr Arg
165 170 175

His Tyr Leu Val Lys Asn Lys Lys Leu Tyr Glu Phe Asn Asn Ser Pro
180 185 190

Tyr Glu Thr Gly Tyr Ile Lys Phe Ile Glu Asn Glu Asn Ser Phe Trp
195 200 205

Tyr Asp Met Met Pro Ala Pro Gly Asp Lys Phe Asp Gln Ser Lys Tyr
210 215 220

Leu Met Met Tyr Asn Asp Asn Lys Met Val Asp Ser Lys Asp Val Lys
225 230 235 240

Ile Glu Val Tyr Leu Thr Thr Lys Lys Lys
245 250

<210> 30
<211> 234
<212> PRT
<213> Staphylococcus aureus

<220>
<221> domain OB-fold
<222> (57)..(131)

<400> 30

Met Asn Lys Lys Leu Leu Met Ile Phe Phe Ile Val Ser Pro Leu Leu
1 5 10 15

Leu Ala Thr Ile Ala Thr Asp Phe Thr Pro Val Pro Leu Leu Ser Asn
20 25 30

Gln Ile Ile Lys Thr Ala Lys Ala Ser Thr Asn Asp Asn Ile Lys Asp
35 40 45

Leu Leu Asp Trp Tyr Ser Ser Gly Ser Asp Thr Phe Thr Asn Ser Glu
50 55 60

Val Leu Asp Asn Ser Leu Gly Ser Met Arg Ile Lys Asn Thr Asp Gly
65 70 75 80

Ser Ile Ser Leu Ile Ile Phe Pro Ser Pro Tyr Tyr Ser Pro Ala Phe
85 90 95

Thr Lys Gly Glu Lys Val Asp Leu Asn Thr Lys Arg Thr Lys Lys Ser
100 105 110

Gln His Thr Ser Glu Gly Thr Tyr Ile His Phe Gln Ile Ser Gly Val
115 120 125

Thr Asn Thr Glu Lys Leu Pro Thr Pro Ile Glu Leu Pro Leu Lys Val
130 135 140

Lys Val His Gly Lys Asp Ser Pro Leu Lys Tyr Trp Pro Lys Phe Asp
145 150 155 160

Lys Lys Gln Leu Ala Ile Ser Thr Leu Asp Phe Glu Ile Arg His Gln
165 170 175

Leu Thr Gln Ile His Gly Leu Tyr Arg Ser Ser Asp Lys Thr Gly Gly
180 185 190

Tyr Trp Lys Ile Thr Met Asn Asp Gly Ser Thr Tyr Gln Ser Asp Leu
195 200 205

Ser Lys Lys Phe Glu Tyr Asn Thr Glu Lys Pro Pro Ile Asn Ile Asp
210 215 220

Glu Ile Lys Thr Ile Glu Ala Glu Ile Asn
225 230

<210> 31
<211> 70
<212> PRT
<213> Escherichia coli

<220>
<221> domain OB-fold
<222> (2)..(70)

<400> 31

Met Ser Gly Lys Met Thr Gly Ile Val Lys Trp Phe Asn Ala Asp Lys
1 5 10 15

Gly Phe Gly Phe Ile Thr Pro Asp Asp Gly Ser Lys Asp Val Phe Val
20 25 30

His Phe Ser Ala Ile Gln Asn Asp Gly Tyr Lys Ser Leu Asp Glu Gly
35 40 45

Gln Lys Val Ser Phe Thr Ile Glu Ser Gly Ala Lys Gly Pro Ala Ala
50 55 60

Gly Asn Val Thr Ser Leu
65 70

<210> 32
<211> 138
<212> PRT
<213> Pyrobaculum aerophilum

<220>
<221> domain OB-fold
<222> (4)..(76)

<400> 32

Met Val Leu Lys Trp Val Met Ser Thr Lys Tyr Val Glu Ala Gly Glu
1 5 10 15

Leu Lys Glu Gly Ser Tyr Val Val Ile Asp Gly Glu Pro Cys Arg Val
20 25 30

Val Glu Ile Glu Lys Ser Lys Thr Gly Lys His Gly Ser Ala Lys Ala
35 40 45

Arg Ile Val Ala Val Gly Val Phe Asp Gly Gly Lys Arg Thr Leu Ser
    50                  55                  60

Leu Pro Val Asp Ala Gln Val Glu Val Pro Ile Ile Glu Lys Phe Thr
65                  70                  75                  80

Ala Gln Ile Leu Ser Val Ser Gly Asp Val Ile Gln Leu Met Asp Met
                85                  90                  95

Arg Asp Tyr Lys Thr Ile Glu Val Pro Met Lys Tyr Val Glu Glu Glu
            100                 105                 110

Ala Lys Gly Arg Leu Ala Pro Gly Ala Glu Val Glu Val Trp Gln Ile
        115                 120                 125

Leu Asp Arg Tyr Lys Ile Ile Arg Val Lys
    130                 135

<210> 33
<211> 711
<212> PRT
<213> Escherichia coli

<220>
<221> domain OB-fold
<222> (623)..(692)

<400> 33

Met Leu Asn Pro Ile Val Arg Lys Phe Gln Tyr Gly Gln His Thr Val
1               5                   10                  15

Thr Leu Glu Thr Gly Met Met Ala Arg Gln Ala Thr Ala Ala Val Met
            20                  25                  30

Val Ser Met Asp Asp Thr Ala Val Phe Val Thr Val Val Gly Gln Lys
        35                  40                  45

Lys Ala Lys Pro Gly Gln Asp Phe Phe Pro Leu Thr Val Asn Tyr Gln
    50                  55                  60

Glu Arg Thr Tyr Ala Ala Gly Arg Ile Pro Gly Ser Phe Phe Arg Arg
65                  70                  75                  80

Glu Gly Arg Pro Ser Glu Gly Glu Thr Leu Ile Ala Arg Leu Ile Asp
                85                  90                  95

Arg Pro Ile Arg Pro Leu Phe Pro Glu Gly Phe Val Asn Glu Val Gln
            100                 105                 110

Val Ile Ala Thr Val Val Ser Val Asn Pro Gln Val Asn Pro Asp Ile
        115                 120                 125

Val Ala Met Ile Gly Ala Ser Ala Ala Leu Ser Leu Ser Gly Ile Pro
130 135 140

Phe Asn Gly Pro Ile Gly Ala Ala Arg Val Gly Tyr Ile Asn Asp Gln
145 150 155 160

Tyr Val Leu Asn Pro Thr Gln Asp Glu Leu Lys Glu Ser Lys Leu Asp
165 170 175

Leu Val Val Ala Gly Thr Glu Ala Ala Val Leu Met Val Glu Ser Glu
180 185 190

Ala Gln Leu Leu Ser Glu Asp Gln Met Leu Gly Ala Val Val Phe Gly
195 200 205

His Glu Gln Gln Gln Val Val Ile Gln Asn Ile Asn Glu Leu Val Lys
210 215 220

Glu Ala Gly Lys Pro Arg Trp Asp Trp Gln Pro Glu Pro Val Asn Glu
225 230 235 240

Ala Leu Asn Ala Arg Val Ala Ala Leu Ala Glu Ala Arg Leu Ser Asp
245 250 255

Ala Tyr Arg Ile Thr Asp Lys Gln Glu Arg Tyr Ala Gln Val Asp Val
260 265 270

Ile Lys Ser Glu Thr Ile Ala Thr Leu Leu Ala Glu Asp Glu Thr Leu
275 280 285

Asp Glu Asn Glu Leu Gly Glu Ile Leu His Ala Ile Glu Lys Asn Val
290 295 300

Val Arg Ser Arg Val Leu Ala Gly Glu Pro Arg Ile Asp Gly Arg Glu
305 310 315 320

Lys Asp Met Ile Arg Gly Leu Asp Val Arg Thr Gly Val Leu Pro Arg
325 330 335

Thr His Gly Ser Ala Leu Phe Thr Arg Gly Glu Thr Gln Ala Leu Val
340 345 350

Thr Ala Thr Leu Gly Thr Ala Arg Asp Ala Gln Val Leu Asp Glu Leu
355 360 365

Met Gly Glu Arg Thr Asp Thr Phe Leu Phe His Tyr Asn Phe Pro Pro
370 375 380

Tyr Ser Val Gly Glu Thr Gly Met Val Gly Ser Pro Lys Arg Arg Glu
385 390 395 400

Ile Gly His Gly Arg Leu Ala Lys Arg Gly Val Leu Ala Val Met Pro
405 410 415

Asp Met Asp Lys Phe Pro Tyr Thr Val Arg Val Val Ser Glu Ile Thr
420 425 430

Glu Ser Asn Gly Ser Ser Ser Met Ala Ser Val Cys Gly Ala Ser Leu
435 440 445

Ala Leu Met Asp Ala Gly Val Pro Ile Lys Ala Ala Val Ala Gly Ile
450 455 460

Ala Met Gly Leu Val Lys Glu Gly Asp Asn Tyr Val Val Leu Ser Asp
465 470 475 480

Ile Leu Gly Asp Glu Asp His Leu Gly Asp Met Asp Phe Lys Val Ala
485 490 495

Gly Ser Arg Asp Gly Ile Ser Ala Leu Gln Met Asp Ile Lys Ile Glu
500 505 510

Gly Ile Thr Lys Glu Ile Met Gln Val Ala Leu Asn Gln Ala Lys Gly
515 520 525

Ala Arg Leu His Ile Leu Gly Val Met Glu Gln Ala Ile Asn Ala Pro
530 535 540

Arg Gly Asp Ile Ser Glu Phe Ala Pro Arg Ile His Thr Ile Lys Ile
545 550 555 560

Asn Pro Asp Lys Ile Lys Asp Val Ile Gly Lys Gly Gly Ser Val Ile
565 570 575

Arg Ala Leu Thr Glu Glu Thr Gly Thr Thr Ile Glu Ile Glu Asp Asp
580 585 590

Gly Thr Val Lys Ile Ala Ala Thr Asp Gly Glu Lys Ala Lys His Ala
595 600 605

Ile Arg Arg Ile Glu Glu Ile Thr Ala Glu Ile Glu Val Gly Arg Val
610 615 620

Tyr Thr Gly Lys Val Thr Arg Ile Val Asp Phe Gly Ala Phe Val Ala
625 630 635 640

Ile Gly Gly Gly Lys Glu Gly Leu Val His Ile Ser Gln Ile Ala Asp
645 650 655

Lys Arg Val Glu Lys Val Thr Asp Tyr Leu Gln Met Gly Gln Glu Val
660 665 670

Pro Val Lys Val Leu Glu Val Asp Arg Gln Gly Arg Ile Arg Leu Ser
675 680 685

Ile Lys Glu Ala Thr Glu Gln Ser Gln Pro Ala Ala Ala Pro Glu Ala
690 695 700

Pro Ala Ala Glu Gln Gly Glu
705 710

<210> 34
<211> 144
<212> PRT
<213> Homo sapiens

<220>
<221> domain OB-fold
<222> (31)..(97)

<400> 34

Met Pro Lys Asn Lys Gly Lys Gly Gly Lys Asn Arg Arg Arg Gly Lys
1 5 10 15

Asn Glu Asn Glu Ser Glu Lys Arg Glu Leu Val Phe Lys Glu Asp Gly
20 25 30

Gln Glu Tyr Ala Gln Val Ile Lys Met Leu Gly Asn Gly Arg Leu Glu
35 40 45

Ala Met Cys Phe Asp Gly Val Lys Arg Leu Cys His Ile Arg Gly Lys
50 55 60

Leu Arg Lys Lys Val Trp Ile Asn Thr Ser Asp Ile Ile Leu Val Gly
65 70 75 80

Leu Arg Asp Tyr Gln Asp Asn Lys Ala Asp Val Ile Leu Lys Tyr Asn
85 90 95

Ala Asp Glu Ala Arg Ser Leu Lys Ala Tyr Gly Glu Leu Pro Glu His
100 105 110

Ala Lys Ile Asn Glu Thr Asp Thr Phe Gly Pro Gly Asp Asp Asp Glu
115 120 125

Ile Gln Phe Asp Asp Ile Gly Asp Asp Asp Glu Asp Ile Asp Asp Ile
130 135 140

<210> 35
<211> 72
<212> PRT
<213> Escherichia coli

<220>
<221> domain OB-fold
<222> (5)..(72)

<400> 35

Met Ala Lys Glu Asp Asn Ile Glu Met Gln Gly Thr Val Leu Glu Thr
1               5                   10                  15

Leu Pro Asn Thr Met Phe Arg Val Glu Leu Glu Asn Gly His Val Val
            20                  25                  30

Thr Ala His Ile Ser Gly Lys Met Arg Lys Asn Tyr Ile Arg Ile Leu
        35                  40                  45

Thr Gly Asp Lys Val Thr Val Glu Leu Thr Pro Tyr Asp Leu Ser Lys
    50                  55                  60

Gly Arg Ile Val Phe Arg Ser Arg
65                  70

<210> 36
<211> 330
<212> PRT
<213> Chlorella virus

<220>
<221> domain OB-fold
<222> (238)..(317)

<400> 36

Met Val Pro Pro Thr Ile Asn Thr Gly Lys Asn Ile Thr Thr Glu Arg
1               5                   10                  15

Ala Val Leu Thr Leu Asn Gly Leu Gln Ile Lys Leu His Lys Val Val
            20                  25                  30

Gly Glu Ser Arg Asp Asp Ile Val Ala Lys Met Lys Asp Leu Ala Met
        35                  40                  45

Asp Asp His Lys Phe Pro Arg Leu Pro Gly Pro Asn Pro Val Ser Ile
    50                  55                  60

Glu Arg Lys Asp Phe Glu Lys Leu Lys Gln Asn Lys Tyr Val Val Ser
65                  70                  75                  80

Glu Lys Thr Asp Gly Ile Arg Phe Met Met Phe Phe Thr Arg Val Phe
                85                  90                  95

Gly Phe Lys Val Cys Thr Ile Ile Asp Arg Ala Met Thr Val Tyr Leu
            100                 105                 110

Leu Pro Phe Lys Asn Ile Pro Arg Val Leu Phe Gln Gly Ser Ile Phe
        115                 120                 125

Asp Gly Glu Leu Cys Val Asp Ile Val Glu Lys Lys Phe Ala Phe Val
130 135 140

Leu Phe Asp Ala Val Val Val Ser Gly Val Thr Val Ser Gln Met Asp
145 150 155 160

Leu Ala Ser Arg Phe Phe Ala Met Lys Arg Ser Leu Lys Glu Phe Lys
165 170 175

Asn Val Pro Glu Asp Pro Ala Ile Leu Arg Tyr Lys Glu Trp Ile Pro
180 185 190

Leu Glu His Pro Thr Ile Ile Lys Asp His Leu Lys Lys Ala Asn Ala
195 200 205

Ile Tyr His Thr Asp Gly Leu Ile Ile Met Ser Val Asp Glu Pro Val
210 215 220

Ile Tyr Gly Arg Asn Phe Asn Leu Phe Lys Leu Lys Pro Gly Thr His
225 230 235 240

His Thr Ile Asp Phe Ile Ile Met Ser Glu Asp Gly Thr Ile Gly Ile
245 250 255

Phe Asp Pro Asn Leu Arg Lys Asn Val Pro Val Gly Lys Leu Asp Gly
260 265 270

Tyr Tyr Asn Lys Gly Ser Ile Val Glu Cys Gly Phe Ala Asp Gly Thr
275 280 285

Trp Lys Tyr Ile Gln Gly Arg Ser Asp Lys Asn Gln Ala Asn Asp Arg
290 295 300

Leu Thr Tyr Glu Lys Thr Leu Leu Asn Ile Glu Glu Asn Ile Thr Ile
305 310 315 320

Asp Glu Leu Leu Asp Leu Phe Lys Trp Glu
325 330

<210> 37
<211> 359
<212> PRT
<213> Bacteriophage T7

<220>
<221> CONFLICT
<222> (2)..(2)

<220>
<221> domain OB-fold
<222> (242)..(349)

<400> 37

Met Met Asn Ile Lys Thr Asn Pro Phe Lys Ala Val Ser Phe Val Glu
1               5                   10                  15

Ser Ala Ile Lys Lys Ala Leu Asp Asn Ala Gly Tyr Leu Ile Ala Glu
            20                  25                  30

Ile Lys Tyr Asp Gly Val Arg Gly Asn Ile Cys Val Asp Asn Thr Ala
        35                  40                  45

Asn Ser Tyr Trp Leu Ser Arg Val Ser Lys Thr Ile Pro Ala Leu Glu
    50                  55                  60

His Leu Asn Gly Phe Asp Val Arg Trp Lys Arg Leu Leu Asn Asp Asp
65                  70                  75                  80

Arg Cys Phe Tyr Lys Asp Gly Phe Met Leu Asp Gly Glu Leu Met Val
                85                  90                  95

Lys Gly Val Asp Phe Asn Thr Gly Ser Gly Leu Leu Arg Thr Lys Trp
            100                 105                 110

Thr Asp Thr Lys Asn Gln Glu Phe His Glu Glu Leu Phe Val Glu Pro
        115                 120                 125

Ile Arg Lys Lys Asp Lys Val Pro Phe Lys Leu His Thr Gly His Leu
    130                 135                 140

His Ile Lys Leu Tyr Ala Ile Leu Pro Leu His Ile Val Glu Ser Gly
145                 150                 155                 160

Glu Asp Cys Asp Val Met Thr Leu Leu Met Gln Glu His Val Lys Asn
                165                 170                 175

Met Leu Pro Leu Leu Gln Glu Tyr Phe Pro Glu Ile Glu Trp Gln Ala
            180                 185                 190

Ala Glu Ser Tyr Glu Val Tyr Asp Met Val Glu Leu Gln Gln Leu Tyr
        195                 200                 205

Glu Gln Lys Arg Ala Glu Gly His Glu Gly Leu Ile Val Lys Asp Pro
    210                 215                 220

Met Cys Ile Tyr Lys Arg Gly Lys Lys Ser Gly Trp Trp Lys Met Lys
225                 230                 235                 240

Pro Glu Asn Glu Ala Asp Gly Ile Ile Gln Gly Leu Val Trp Gly Thr
                245                 250                 255

Lys Gly Leu Ala Asn Glu Gly Lys Val Ile Gly Phe Glu Val Leu Leu
            260                 265                 270

Glu Ser Gly Arg Leu Val Asn Ala Thr Asn Ile Ser Arg Ala Leu Met
275 280 285

Asp Glu Phe Thr Glu Thr Val Lys Glu Ala Thr Leu Ser Gln Trp Gly
290 295 300

Phe Phe Ser Pro Tyr Gly Ile Gly Asp Asn Asp Ala Cys Thr Ile Asn
305 310 315 320

Pro Tyr Asp Gly Trp Ala Cys Gln Ile Ser Tyr Met Glu Glu Thr Pro
325 330 335

Asp Gly Ser Leu Arg His Pro Ser Phe Val Met Phe Arg Gly Thr Glu
340 345 350

Asp Asn Pro Gln Glu Lys Met
355

<210> 38
<211> 242
<212> PRT
<213> Staphylococcus aureus

<220>
<221> domain OB-fold
<222> (100)..(202)

<220>
<221> CONFLICT
<222> (217)..(217)

<400> 38

Arg Leu Ile Leu Lys Leu Thr Lys Lys Lys Glu Val Leu Val Met Thr
1 5 10 15

Glu Tyr Leu Leu Ser Ala Gly Ile Cys Met Ala Ile Val Ser Ile Leu
20 25 30

Leu Ile Gly Met Ala Ile Ser Asn Val Ser Lys Gly Gln Tyr Ala Lys
35 40 45

Arg Phe Phe Phe Phe Ala Thr Ser Cys Leu Val Leu Thr Leu Val Val
50 55 60

Val Ser Ser Leu Ser Ser Ser Ala Asn Ala Ser Gln Thr Asp Asn Gly
65 70 75 80

Val Asn Arg Ser Gly Ser Glu Asp Pro Thr Val Tyr Ser Ala Thr Ser
85 90 95

Thr Lys Lys Leu His Lys Glu Pro Ala Thr Leu Ile Lys Ala Ile Asp
100 105 110

Gly Asp Thr Val Lys Leu Met Tyr Lys Gly Gln Pro Met Thr Phe Arg
115 120 125

Leu Leu Leu Val Asp Thr Pro Glu Thr Lys His Pro Lys Lys Gly Val
130 135 140

Glu Lys Tyr Gly Pro Glu Ala Ser Ala Phe Thr Lys Lys Met Val Glu
145 150 155 160

Asn Ala Lys Lys Ile Glu Val Glu Phe Asp Lys Gly Gln Arg Thr Asp
165 170 175

Lys Tyr Gly Arg Gly Leu Ala Tyr Ile Tyr Ala Asp Gly Lys Met Val
180 185 190

Asn Glu Ala Leu Val Arg Gln Gly Leu Ala Lys Val Ala Tyr Val Tyr
195 200 205

Lys Pro Asn Asn Thr His Glu Gln His Leu Arg Lys Ser Glu Ala Gln
210 215 220

Ala Lys Lys Glu Lys Leu Asn Ile Trp Ser Glu Asp Asn Ala Asp Ser
225 230 235 240

Gly Gln

<210> 39
<211> 203
<212> PRT
<213> Escherichia coli

<220>
<221> domain OB-fold
<222> (2)..(64)

<400> 39

Met Ile Gly Arg Leu Arg Gly Ile Ile Ile Glu Lys Gln Pro Pro Leu
1 5 10 15

Val Leu Ile Glu Val Gly Gly Val Gly Tyr Glu Val His Met Pro Met
20 25 30

Thr Cys Phe Tyr Glu Leu Pro Glu Ala Gly Gln Glu Ala Ile Val Phe
35 40 45

Thr His Phe Val Val Arg Glu Asp Ala Gln Leu Leu Tyr Gly Phe Asn
50 55 60

Asn Lys Gln Glu Arg Thr Leu Phe Lys Glu Leu Ile Lys Thr Asn Gly
65 70 75 80

Val Gly Pro Lys Leu Ala Leu Ala Ile Leu Ser Gly Met Ser Ala Gln
85 90 95

Gln Phe Val Asn Ala Val Glu Arg Glu Glu Val Gly Ala Leu Val Lys
100 105 110

Leu Pro Gly Ile Gly Lys Lys Thr Ala Glu Arg Leu Ile Val Glu Met
115 120 125

Lys Asp Arg Phe Lys Gly Leu His Gly Asp Leu Phe Thr Pro Ala Ala
130 135 140

Asp Leu Val Leu Thr Ser Pro Ala Ser Pro Ala Thr Asp Asp Ala Glu
145 150 155 160

Gln Glu Ala Val Ala Ala Leu Val Ala Leu Gly Tyr Lys Pro Gln Glu
165 170 175

Ala Ser Arg Met Val Ser Lys Ile Ala Arg Pro Asp Ala Ser Ser Glu
180 185 190

Thr Leu Ile Arg Glu Ala Leu Arg Ala Ala Leu
195 200

<210> 40
<211> 78
<212> PRT
<213> Bacteriophage Pf3

<220>
<221> domain OB-fold
<222> (2)..(78)

<220>
<221> CONFLICT
<222> (36)..(36)

<400> 40

Met Asn Ile Gln Ile Thr Phe Thr Asp Ser Val Arg Gln Gly Thr Ser
1 5 10 15

Ala Lys Gly Asn Pro Tyr Thr Phe Gln Glu Gly Phe Leu His Leu Glu
20 25 30

Asp Lys Pro Phe Pro Leu Gln Cys Gln Phe Phe Val Glu Ser Val Ile
35 40 45

Pro Ala Gly Ser Tyr Gln Val Pro Tyr Arg Ile Asn Val Asn Asn Gly
50 55 60

Arg Pro Glu Leu Ala Phe Asp Phe Lys Ala Met Lys Arg Ala
65 70 75

<210> 41
<211> 87
<212> PRT
<213> Bacteriophage M13

<220>
<221> domain OB-fold
<222> (2)..(87)

<220>
<221> CONFLICT
<222> (70)..(70)

<400> 41

Met Ile Lys Val Glu Ile Lys Pro Ser Gln Ala Gln Phe Thr Thr Arg
1               5                   10                  15

Ser Gly Val Ser Arg Gln Gly Lys Pro Tyr Ser Leu Asn Glu Gln Leu
            20                  25                  30

Cys Tyr Val Asp Leu Gly Asn Glu Tyr Pro Val Leu Val Lys Ile Thr
        35                  40                  45

Leu Asp Glu Gly Gln Pro Ala Tyr Ala Pro Gly Leu Tyr Thr Val His
    50                  55                  60

Leu Ser Ser Phe Lys Ile Gly Gln Phe Gly Ser Leu Met Ile Asp Arg
65                  70                  75                  80

Leu Arg Leu Val Pro Ala Lys
                85

<210> 42
<211> 292
<212> PRT
<213> Bacteriophage T4

<220>
<221> domain OB-fold
<222> (40)..(194)

<400> 42

Met Phe Lys Arg Lys Ser Thr Ala Glu Leu Ala Ala Gln Met Ala Lys
1               5                   10                  15

Leu Asn Gly Asn Lys Gly Phe Ser Ser Glu Asp Lys Gly Glu Trp Lys
            20                  25                  30

Leu Lys Leu Asp Asn Ala Gly Asn Gly Gln Ala Val Ile Arg Phe Leu
        35                  40                  45

Pro Ser Lys Asn Asp Glu Gln Ala Pro Phe Ala Ile Leu Val Asn His
    50                  55                  60

Gly Phe Lys Lys Asn Gly Lys Trp Tyr Ile Glu Thr Cys Ser Ser Thr
65 70 75 80

His Gly Asp Tyr Asp Ser Cys Pro Val Cys Gln Tyr Ile Ser Lys Asn
85 90 95

Asp Leu Tyr Asn Thr Asp Asn Lys Glu Tyr Ser Leu Val Lys Arg Lys
100 105 110

Thr Ser Tyr Trp Ala Asn Ile Leu Val Val Lys Asp Pro Ala Ala Pro
115 120 125

Glu Asn Glu Gly Lys Val Phe Lys Tyr Arg Phe Gly Lys Lys Ile Trp
130 135 140

Asp Lys Ile Asn Ala Met Ile Ala Val Asp Val Glu Met Gly Glu Thr
145 150 155 160

Pro Val Asp Val Thr Cys Pro Trp Glu Gly Ala Asn Phe Val Leu Lys
165 170 175

Val Lys Gln Val Ser Gly Phe Ser Asn Tyr Asp Glu Ser Lys Phe Leu
180 185 190

Asn Gln Ser Ala Ile Pro Asn Ile Asp Asp Glu Ser Phe Gln Lys Glu
195 200 205

Leu Phe Glu Gln Met Val Asp Leu Ser Glu Met Thr Ser Lys Asp Lys
210 215 220

Phe Lys Ser Phe Glu Glu Leu Asn Thr Lys Phe Gly Gln Val Met Gly
225 230 235 240

Thr Ala Val Met Gly Gly Ala Ala Ala Thr Ala Ala Lys Lys Ala Asp
245 250 255

Lys Val Ala Asp Asp Leu Asp Ala Phe Asn Val Asp Asp Phe Asn Thr
260 265 270

Lys Thr Glu Asp Asp Phe Met Ser Ser Ser Ser Gly Ser Ser Ser Ser
275 280 285

Ala Asp Asp Thr
290

<210> 43
<211> 175
<212> PRT
<213> Thermus thermophilus

<220>
<221> domain OB-fold
<222> (15)..(111)

<400> 43

Met Ala Asn Leu Lys Ser Leu Pro Val Gly Asp Lys Ala Pro Glu Val
1 5 10 15

Val His Met Val Ile Glu Val Pro Arg Gly Ser Gly Asn Lys Tyr Glu
20 25 30

Tyr Asp Pro Asp Leu Gly Ala Ile Lys Leu Asp Arg Val Leu Pro Gly
35 40 45

Ala Gln Phe Tyr Pro Gly Asp Tyr Gly Phe Ile Pro Ser Thr Leu Ala
50 55 60

Glu Asp Gly Asp Pro Leu Asp Gly Leu Val Leu Ser Thr Tyr Pro Leu
65 70 75 80

Leu Pro Gly Val Val Val Glu Val Arg Val Val Gly Leu Leu Leu Met
85 90 95

Glu Asp Glu Lys Gly Gly Asp Ala Lys Val Ile Gly Val Val Ala Glu
100 105 110

Asp Gln Arg Leu Asp His Ile Gln Asp Ile Gly Asp Val Pro Glu Gly
115 120 125

Val Lys Gln Glu Ile Gln His Phe Phe Glu Thr Tyr Lys Ala Leu Glu
130 135 140

Ala Lys Lys Gly Lys Trp Val Lys Val Thr Gly Trp Arg Asp Arg Lys
145 150 155 160

Ala Ala Leu Glu Glu Val Arg Ala Cys Ile Ala Arg Tyr Lys Gly
165 170 175

<210> 44
<211> 62
<212> PRT
<213> artificial sequence

<220>
<223> Sac7a protein/carboxy-terminal modified Sac7d species

<400> 44

Met Val Lys Val Lys Phe Lys Tyr Lys Gly Glu Glu Lys Glu Val Asp
1 5 10 15

Thr Ser Lys Ile Lys Lys Val Trp Arg Val Gly Lys Met Val Ser Phe
20 25 30

Thr Tyr Asp Asp Asn Gly Lys Thr Gly Arg Gly Ala Val Ser Glu Lys
35 40 45

Asp Ala Pro Lys Glu Leu Leu Asp Met Leu Ala Arg Ala Glu
50 55 60

<210> 45
<211> 59
<212> PRT
<213> artificial sequence

<220>
<223> Sac7b protein/carboxy-terminal modified Sac7d species

<400> 45

Met Val Lys Val Lys Phe Lys Tyr Lys Gly Glu Glu Lys Glu Val Asp
1 5 10 15

Thr Ser Lys Ile Lys Lys Val Trp Arg Val Gly Lys Met Val Ser Phe
20 25 30

Thr Tyr Asp Asp Asn Gly Lys Thr Gly Arg Gly Ala Val Ser Glu Lys
35 40 45

Asp Ala Pro Lys Glu Leu Leu Asp Met Leu Ala
50 55

<210> 46
<211> 66
<212> PRT
<213> artificial sequence

<220>
<223> hIgG binding protein

<220>
<221> VARIANT
<222> (7)..(7)
<223> Xaa is any amino acid

<220>
<221> VARIANT
<222> (8)..(8)
<223> Xaa is any amino acid

<220>
<221> VARIANT
<222> (9)..(9)
<223> Xaa is any amino acid

<220>
<221> VARIANT
<222> (44)..(44)
<223> Xaa is any amino acid

<220>
<221> VARIANT
<222> (46)..(46)
<223> Xaa is any amino acid

<220>
<221> VARIANT
<222> (46)..(46)
<223> Xaa is any amino acid

<400> 46

Ser Val Lys Val Lys Phe Xaa Xaa Xaa Gly Glu Glu Lys Glu Val Asp
1               5                   10                  15

Thr Ser Lys Ile Arg Asp Val Cys Arg Gln Gly Lys Asn Val Lys Phe
            20                  25                  30

Leu Tyr Asn Asp Asn Gly Lys Tyr Gly Ala Gly Xaa Val Xaa Glu Lys
        35                  40                  45

Asp Ala Pro Lys Glu Leu Leu Asp Met Leu Ala Arg Ala Glu Arg Glu
    50                  55                  60

Lys Lys
65

<210> 47
<211> 66
<212> PRT
<213> artificial sequence

<220>
<223> hIgG binding protein

<400> 47

Ser Val Lys Val Lys Phe Leu Leu Asn Gly Glu Glu Lys Glu Val Asp
1               5                   10                  15

Thr Ser Lys Ile Arg Asp Val Cys Arg Gln Gly Lys Asn Val Lys Phe
            20                  25                  30

Leu Tyr Asn Asp Asn Gly Lys Tyr Gly Ala Gly Asn Val Asp Glu Lys
        35                  40                  45

Asp Ala Pro Lys Glu Leu Leu Asp Met Leu Ala Arg Ala Glu Arg Glu
    50                  55                  60

Lys Lys
65

<210> 48
<211> 66
<212> PRT
<213> artificial sequence

<220>
<223> hIgG binding protein

<400> 48

Ser Val Lys Val Lys Phe Leu Leu Asn Gly Glu Glu Lys Glu Val Asp

1 5 10 15

Thr Ser Lys Ile Arg Asp Val Cys Arg Gln Gly Lys Asn Val Lys Phe
20 25 30

Leu Tyr Asn Asp Asn Gly Lys Tyr Gly Ala Gly Lys Val Asn Glu Lys
35 40 45

Asp Ala Pro Lys Glu Leu Leu Asp Met Leu Ala Arg Ala Glu Arg Glu
50 55 60

Lys Lys
65

<210> 49
<211> 66
<212> PRT
<213> artificial sequence

<220>
<223> hIgG binding protein

<400> 49

Ser Val Lys Val Lys Phe His Ser His Gly Glu Glu Lys Glu Val Asp
1 5 10 15

Thr Ser Lys Ile Arg Asp Val Cys Arg Gln Gly Lys Asn Val Lys Phe
20 25 30

Leu Tyr Asn Asp Asn Gly Lys Tyr Gly Ala Gly Asn Val Asp Glu Lys
35 40 45

Asp Ala Pro Lys Glu Leu Leu Asp Met Leu Ala Arg Ala Glu Arg Glu
50 55 60

Lys Lys
65

<210> 50
<211> 66
<212> PRT
<213> artificial sequence

<220>
<223> hIgG binding protein

<400> 50

Ser Val Lys Val Lys Phe Leu Cys His Gly Glu Glu Lys Glu Val Asp
1 5 10 15

Thr Ser Lys Ile Arg Asp Val Cys Arg Gln Gly Lys Asn Val Lys Phe
20 25 30

Leu Tyr Asn Asp Asn Gly Lys Tyr Gly Ala Gly Asn Val Asp Glu Lys

35 40 45

Asp Ala Pro Lys Glu Leu Leu Asp Met Leu Ala Arg Ala Glu Arg Glu
50 55 60

Lys Lys
65

<210> 51
<211> 66
<212> PRT
<213> artificial sequence

<220>
<223> hIgG binding protein

<400> 51

Ser Val Lys Val Lys Phe Arg Tyr Leu Gly Glu Glu Lys Glu Val Asp
1 5 10 15

Thr Ser Lys Ile Arg Asp Val Cys Arg Gln Gly Lys Asn Val Lys Phe
20 25 30

Leu Tyr Asn Asp Asn Gly Lys Tyr Gly Ala Gly Asn Val Asp Glu Lys
35 40 45

Asp Ala Pro Lys Glu Leu Leu Asp Met Leu Ala Arg Ala Glu Arg Glu
50 55 60

Lys Lys
65

<210> 52
<211> 66
<212> PRT
<213> artificial sequence

<220>
<223> hIgG binding protein

<400> 52

Ser Val Lys Val Lys Phe Ala Arg His Gly Glu Glu Lys Glu Val Asp
1 5 10 15

Thr Ser Lys Ile Arg Asp Val Cys Arg Gln Gly Lys Asn Val Lys Phe
20 25 30

Leu Tyr Asn Asp Asn Gly Lys Tyr Gly Ala Gly Asn Val Arg Glu Lys
35 40 45

Asp Ala Pro Lys Glu Leu Leu Asp Met Leu Ala Arg Ala Glu Arg Glu
50 55 60

Lys Lys

65

<210> 53
<211> 66
<212> PRT
<213> artificial sequence

<220>
<223> hIgG binding protein

<400> 53

Ser Val Lys Val Lys Phe Leu Leu Asn Gly Glu Glu Lys Glu Val Asp
1               5                   10                  15

Thr Ser Lys Ile Arg Asp Val Cys Arg Gln Gly Lys Asn Val Lys Phe
            20                  25                  30

Leu Tyr Asn Asp Asn Gly Lys Tyr Gly Ala Gly Asn Val Asp Glu Lys
        35                  40                  45

Asp Val Pro Lys Glu Leu Leu Asp Met Leu Ala Arg Ala Glu Arg Glu
    50                  55                  60

Lys Lys
65

<210> 54
<211> 66
<212> PRT
<213> artificial sequence

<220>
<223> hIgG binding protein

<400> 54

Ser Val Lys Val Lys Phe Leu Leu Asn Gly Glu Glu Gln Glu Val Asp
1               5                   10                  15

Thr Ser Lys Ile Arg Asp Val Cys Arg Gln Gly Lys Asn Val Lys Phe
            20                  25                  30

Leu Tyr Asn Asp Asn Gly Lys Tyr Gly Ala Gly Asn Val Asp Glu Lys
        35                  40                  45

Asp Ala Pro Lys Glu Leu Leu Asp Met Leu Ala Arg Ala Glu Arg Glu
    50                  55                  60

Lys Lys
65

<210> 55
<211> 66
<212> PRT
<213> artificial sequence

<220>
<223> hIgG binding protein

<400> 55

Ser Val Lys Val Lys Phe Leu Leu Asn Gly Glu Glu Lys Glu Val Asp
1               5                   10                  15

Thr Ser Lys Ile Arg Asp Val Cys Arg Gln Gly Lys Asn Val Lys Phe
            20                  25                  30

Leu Tyr Asn Asp Asn Gly Lys Tyr Gly Ala Gly Asn Val Asp Lys Lys
        35                  40                  45

Asp Ala Pro Lys Glu Leu Leu Asp Met Leu Ala Arg Ala Glu Arg Glu
    50                  55                  60

Lys Lys
65

<210> 56
<211> 66
<212> PRT
<213> artificial sequence

<220>
<223> hIgG binding protein

<400> 56

Ser Val Lys Val Lys Phe Leu Leu Asn Gly Glu Glu Lys Glu Val Asp
1               5                   10                  15

Thr Ser Lys Ile Arg Asp Val Cys Arg Gln Gly Lys Asn Val Lys Phe
            20                  25                  30

Leu Tyr Asn Asp Asn Asp Lys Tyr Gly Ala Gly Asn Val Asp Glu Lys
        35                  40                  45

Asp Ala Pro Lys Glu Leu Leu Asp Met Leu Ala Arg Ala Glu Arg Glu
    50                  55                  60

Lys Lys
65

<210> 57
<211> 66
<212> PRT
<213> artificial sequence

<220>
<223> hIgG binding protein

<400> 57

Ser Val Lys Val Lys Phe Leu Leu Asn Gly Glu Glu Lys Glu Val Asp
1               5                   10                  15

Thr Ser Lys Ile Arg Asp Val Cys Arg Gln Gly Lys Asn Val Lys Phe
20 25 30

Leu Tyr Asn Asn Asn Gly Lys Tyr Gly Ala Gly Asn Val Asp Glu Lys
35 40 45

Asp Ala Pro Lys Glu Leu Leu Glu Met Leu Ala Arg Ala Glu Arg Glu
50 55 60

Lys Lys
65

<210> 58
<211> 79
<212> PRT
<213> artificial sequence

<220>
<223> H4S

<400> 58

Met Arg Gly Ser His His His His His His Gly Ser Val Lys Val Lys
1 5 10 15

Phe Phe Trp Asn Gly Glu Glu Lys Glu Val Asp Thr Ser Lys Ile Val
20 25 30

Trp Val Lys Arg Ala Gly Lys Ser Val Leu Phe Ile Tyr Asp Asp Asn
35 40 45

Gly Lys Asn Gly Tyr Gly Asp Val Thr Glu Lys Asp Ala Pro Lys Glu
50 55 60

Leu Leu Asp Met Leu Ala Arg Ala Glu Arg Glu Lys Lys Leu Asn
65 70 75

<210> 59
<211> 79
<212> PRT
<213> artificial sequence

<220>
<223> H4S-W8C

<400> 59

Met Arg Gly Ser His His His His His His Gly Ser Val Lys Val Lys
1 5 10 15

Phe Phe Cys Asn Gly Glu Glu Lys Glu Val Asp Thr Ser Lys Ile Val
20 25 30

Trp Val Lys Arg Ala Gly Lys Ser Val Leu Phe Ile Tyr Asp Asp Asn
35 40 45

Gly Lys Asn Gly Tyr Gly Asp Val Thr Glu Lys Asp Ala Pro Lys Glu
50 55 60

Leu Leu Asp Met Leu Ala Arg Ala Glu Arg Glu Lys Lys Leu Asn
65 70 75

<210> 60
<211> 79
<212> PRT
<213> artificial sequence

<220>
<223> H4S-V21C

<400> 60

Met Arg Gly Ser His His His His His His Gly Ser Val Lys Val Lys
1 5 10 15

Phe Phe Trp Asn Gly Glu Glu Lys Glu Val Asp Thr Ser Lys Ile Cys
20 25 30

Trp Val Lys Arg Ala Gly Lys Ser Val Leu Phe Ile Tyr Asp Asp Asn
35 40 45

Gly Lys Asn Gly Tyr Gly Asp Val Thr Glu Lys Asp Ala Pro Lys Glu
50 55 60

Leu Leu Asp Met Leu Ala Arg Ala Glu Arg Glu Lys Lys Leu Asn
65 70 75

<210> 61
<211> 79
<212> PRT
<213> artificial sequence

<220>
<223> H4S-K24C

<400> 61

Met Arg Gly Ser His His His His His His Gly Ser Val Lys Val Lys
1 5 10 15

Phe Phe Trp Asn Gly Glu Glu Lys Glu Val Asp Thr Ser Lys Ile Val
20 25 30

Trp Val Cys Arg Ala Gly Lys Ser Val Leu Phe Ile Tyr Asp Asp Asn
35 40 45

Gly Lys Asn Gly Tyr Gly Asp Val Thr Glu Lys Asp Ala Pro Lys Glu
50 55 60

Leu Leu Asp Met Leu Ala Arg Ala Glu Arg Glu Lys Lys Leu Asn
65 70 75

<210> 62
<211> 79
<212> PRT
<213> artificial sequence

<220>
<223> H4S-A26C

<400> 62

Met Arg Gly Ser His His His His His His Gly Ser Val Lys Val Lys
1               5                   10                  15

Phe Phe Trp Asn Gly Glu Glu Lys Glu Val Asp Thr Ser Lys Ile Val
            20                  25                  30

Trp Val Lys Arg Cys Gly Lys Ser Val Leu Phe Ile Tyr Asp Asp Asn
        35                  40                  45

Gly Lys Asn Gly Tyr Gly Asp Val Thr Glu Lys Asp Ala Pro Lys Glu
    50                  55                  60

Leu Leu Asp Met Leu Ala Arg Ala Glu Arg Glu Lys Lys Leu Asn
65                  70                  75

<210> 63
<211> 79
<212> PRT
<213> artificial sequence

<220>
<223> H4S-K39C

<400> 63

Met Arg Gly Ser His His His His His His Gly Ser Val Lys Val Lys
1               5                   10                  15

Phe Phe Trp Asn Gly Glu Glu Lys Glu Val Asp Thr Ser Lys Ile Val
            20                  25                  30

Trp Val Lys Arg Ala Gly Lys Ser Val Leu Phe Ile Tyr Asp Asp Asn
        35                  40                  45

Gly Cys Asn Gly Tyr Gly Asp Val Thr Glu Lys Asp Ala Pro Lys Glu
    50                  55                  60

Leu Leu Asp Met Leu Ala Arg Ala Glu Arg Glu Lys Lys Leu Asn
65                  70                  75

<210> 64
<211> 3617
<212> DNA
<213> artificial sequence

<220>
<223> pH4S

<400> 64

ctcgagaaat cataaaaaat ttatttgctt tgtgagcgga taacaattat aatagattca 60
attgtgagcg gataacaatt tcacacagaa ttcattaaag aggagaaatt aactatgaga 120
ggatcgcatc accatcacca tcacggatcc gtcaaggtga aattcttctg gaacggcgaa 180
gaaaaagaag tggacactag taagatcgtg tgggttaagc gtgcgggcaa aagtgtgctc 240
tttatctacg acgacaacgg caagaacggt tacggcgacg tgaccgagaa agatgccccg 300
aaagagttat tagatatgtt agcgcgtgcg gaacgcgaga aaaagcttaa ttagctgagc 360
ttggactcct gttgatagat ccagtaatga cctcagaact ccatctggat ttgttcagaa 420
cgctcggttg ccgccgggcg ttttttattg gtgagaatcc aagctagctt ggcgagattt 480
tcaggagcta aggaagctaa aatggagaaa aaaatcactg gatataccac cgttgatata 540
tcccaatggc atcgtaaaga acattttgag gcatttcagt cagttgctca atgtacctat 600
aaccagaccg ttcagctgga tattacggcc tttttaaaga ccgtaaagaa aaataagcac 660
aagttttatc cggcctttat tcacattctt gcccgcctga tgaatgctca tccggaattt 720
cgtatggcaa tgaaagacgg tgagctggtg atatgggata gtgttcaccc ttgttacacc 780
gttttccatg agcaaactga aacgttttca tcgctctgga gtgaatacca cgacgatttc 840
cggcagtttc tacacatata ttcgcaagat gtggcgtgtt acggtgaaaa cctggcctat 900
ttccctaaag ggtttattga gaatatgttt ttcgtctcag ccaatccctg ggtgagtttc 960
accagttttg atttaaacgt ggccaatatg gacaacttct tcgcccccgt tttcaccatg 1020
ggcaaatatt atacgcaagg cgacaaggtg ctgatgccgc tggcgattca ggttcatcat 1080
gccgtttgtg atggcttcca tgtcggcaga atgcttaatg aattacaaca gtactgcgat 1140
gagtggcagg gcggggcgta atttttttaa ggcagttatt ggtgccctta aacgcctggg 1200
gtaatgactc tctagcttga ggcatcaaat aaaacgaaag gctcagtcga aagactgggc 1260
ctttcgtttt atctgttgtt tgtcggtgaa cgctctcctg agtaggacaa atccgccctc 1320
tagagctgcc tcgcgcgttt cggtgatgac ggtgaaaacc tctgacacat gcagctcccg 1380
gagacggtca cagcttgtct gtaagcggat gccgggagca gacaagcccg tcagggcgcg 1440
tcagcgggtg ttggcgggtg tcggggcgca gccatgaccc agtcacgtag cgatagcgga 1500
gtgtatactg gcttaactat gcggcatcag agcagattgt actgagagtg caccatatgc 1560
ggtgtgaaat accgcacaga tgcgtaagga gaaaataccg catcaggcgc tcttccgctt 1620
cctcgctcac tgactcgctg cgctcggtcg ttcggctgcg gcgagcggta tcagctcact 1680
caaaggcggt aatacggtta tccacagaat caggggataa cgcaggaaag aacatgtgag 1740
caaaaggcca gcaaaaggcc aggaaccgta aaaaggccgc gttgctggcg tttttccata 1800
ggctccgccc ccctgacgag catcacaaaa atcgacgctc aagtcagagg tggcgaaacc 1860
cgacaggact ataaagatac caggcgtttc cccctggaag ctccctcgtg cgctctcctg 1920
ttccgaccct gccgcttacc ggatacctgt ccgcctttct cccttcggga agcgtggcgc 1980

```
tttctcatag ctcacgctgt aggtatctca gttcggtgta ggtcgttcgc tccaagctgg     2040
gctgtgtgca cgaacccccc gttcagcccg accgctgcgc cttatccggt aactatcgtc     2100
ttgagtccaa cccggtaaga cacgacttat cgccactggc agcagccact ggtaacagga     2160
ttagcagagc gaggtatgta ggcggtgcta cagagttctt gaagtggtgg cctaactacg     2220
gctacactag aaggacagta tttggtatct gcgctctgct gaagccagtt accttcggaa     2280
aaagagttgg tagctcttga tccggcaaac aaaccaccgc tggtagcggt ggtttttttg     2340
tttgcaagca gcagattacg cgcagaaaaa aaggatctca agaagatcct ttgatctttt     2400
ctacggggtc tgacgctcag tggaacgaaa actcacgtta agggattttg gtcatgagat     2460
tatcaaaaag gatcttcacc tagatccttt taaattaaaa atgaagtttt aaatcaatct     2520
aaagtatata tgagtaaact tggtctgaca gttaccaatg cttaatcagt gaggcaccta     2580
tctcagcgat ctgtctattt cgttcatcca tagttgcctg actccccgtc gtgtagataa     2640
ctacgatacg ggagggctta ccatctggcc ccagtgctgc aatgataccg cgagacccac     2700
gctcaccggc tccagattta tcagcaataa accagccagc cggaagggcc gagcgcagaa     2760
gtggtcctgc aactttatcc gcctccatcc agtctattaa ttgttgccgg gaagctagag     2820
taagtagttc gccagttaat agtttgcgca acgttgttgc cattgctaca ggcatcgtgg     2880
tgtcacgctc gtcgtttggt atggcttcat tcagctccgg ttcccaacga tcaaggcgag     2940
ttacatgatc ccccatgttg tgcaaaaaag cggttagctc cttcggtcct ccgatcgttg     3000
tcagaagtaa gttggccgca gtgttatcac tcatggttat ggcagcactg cataattctc     3060
ttactgtcat gccatccgta agatgctttt ctgtgactgg tgagtactca accaagtcat     3120
tctgagaata gtgtatgcgg cgaccgagtt gctcttgccc ggcgtcaata cgggataata     3180
ccgcgccaca tagcagaact ttaaaagtgc tcatcattgg aaaacgttct tcggggcgaa     3240
aactctcaag gatcttaccg ctgttgagat ccagttcgat gtaacccact cgtgcaccca     3300
actgatcttc agcatctttt actttcacca gcgtttctgg gtgagcaaaa acaggaaggc     3360
aaaatgccgc aaaaaaggga ataagggcga cacggaaatg ttgaatactc atactcttcc     3420
tttttcaata ttattgaagc atttatcagg gttattgtct catgagcgga tacatatttg     3480
aatgtattta gaaaaataaa caaatagggg ttccgcgcac atttccccga aaagtgccac     3540
ctgacgtcta agaaaccatt attatcatga cattaaccta taaaaatagg cgtatcacga     3600
ggccctttcg tcttcac                                                    3617
```

## Description

The present invention relates to reagentless fluorescent biosensors from Nanofitins which comprise at least an OB-fold protein and a fluorophore as well as to methods to generate reagentless fluorescent (RF) biosensors, in particular wherein no structural data exists of the biosensor in combination with its molecular target.

A molecular biosensor transforms a specific molecular binding event into a detectable signal and comprises several modules: a recognition module, which can also be called a receptor, can be of biological origin or biomimetic and which recognises at least one specific target such as an antigen, ligand or analyte during the binding event; a transduction module, which transforms the recognition event into a measurable signal; and a means of evaluating the measurable signal data.

The recognition and transduction modules should be integrated into a compact device of molecular dimensions (Lowe, 1984) and a molecular biosensor can function without additional reagents and provide quantitative analytical information and follow the concentration of its target, continuously (Thevenot *et al.,* 2001).

The physical nature of the measurable signal can be very diverse (Morgan *et al.,* 1996). Fluorescence is an optical signal which allows one to detect molecular interactions with great sensitivity. The transduction is based on a variation of the fluorescence properties of the biosensor when it interacts with its analyte (Altschuh *et al.,* 2006). The fluorescence of a protein biosensor can be intrinsic, e. g. provided by its component residues of tyrosine and tryptophan, or extrinsic, e. g. provided by the chemical coupling of fluorescent groups. The coupling of several fluorophores to a unique molecule of biosensor can be beneficial but is usually difficult to implement (Smith *et al.,* 2005). Although intrinsic protein fluorescence can be used to study molecular interactions in purified experimental systems, extrinsic fluorescence is normally preferable to monitor specific interactions in complex media, without interference from other protein components (Foote and Winter, 1992).

The changes of fluorescence that occur upon recognition between a reagentless fluorescent biosensor and its target, result from different interactions between the fluorescent group and its environment in the free and bound forms of the biosensor.

The binding of the target can occur in the neighborhood of the fluorescent group and directly modify its environment.

Alternatively, the binding of the target can induce a conformational change in the biosensor and thus cause an interaction between the fluorescent group and the receptor indirectly.

Antibodies are perfectly suited to provide the recognition module of biosensors since they can be directed against almost any target with the potential exception of self-proteins. However, antibodies or their derivative fragments are often difficult and expensive to produce because of their molecular complexity and/or their insufficient stability.

For these reasons, two new artificial families of target binding proteins, DARPins and Nanofitins (formerly known as Affitins) have recently been developed by engineering two different protein families that have a stable polypeptide scaffold, are devoid of cysteine residues and disulfide bonds and consequently are well expressed in *Escherichia coli.* These new families of target binding proteins, DARPins and Nanofitins have been shown able to replace antibodies in many of their applications.

The family of the Designed Ankyrin Repeat Proteins (DARpins) is a well characterized artificial family of protein-target binding proteins (Mosavi *et al*., 2004; Li *et al.,* 2006).

The OB-fold protein family is a natural family of proteins which possess the oligonucleotide/oligosaccharide binding-fold (OB-fold), a five stranded β-barrel capped with an amphiphilic α-helix. Members of this family bind a wide range of structurally different ligands : ssDNA, dsDNA, RNA, oligosaccharides, proteins, metallic ions and catalytic substrates (Agrawal and Kishan, 2003; Arcus, 2002; Theobald *et al.,* 2003).

Combinatorial libraries of DARPins and OB-fold proteins have been generated by randomization of residues that have a strong probability of contributing to the target binding site, and for DARPins only, the subsequent assemblage of a random number of ankyrin modules between defined N- and C-terminal modules (Binz *et al.,* 2003). These libraries were used to select DARPins or Nanofitins that bound specific targets, using ribosome or phage display. DARPins and Nanofitins with high affinities and specificities have been obtained against a wide variety of protein targets Binz *et al.,* 2004,; Stumpp *et al.,* 2008; WO 2008/068637; Mouratou *et al.,* 2007; krehenbrink *et al.,* 2008; Cinier *et al*., 2009).

The inventors and others have engineered reagentless fluorescent (RF) biosensors from antibodies and DARPins, first when the three-dimensional structure of the complex with their target is known and then in the absence of such a knowledge *(Renard et al.,* 2002; Renard *et al.,* 2003; Jespers *et al.,* 2004; Renard and Bedouelle 2004; Brient-Litzler *et al.,* 2010). This work also formed the basis of WO 2001/065258 and WO 2009/115919 which described such antibody and DARPin based biosensor molecules.

The antibody is used in the form of a single-chain variable fragment or scFv. A residue of the single-chain variable fragment or the DARPin is identified which is in proximity to the target, when the target is in complex with the single-chain variable fragment or the DARPin. The selected residue is changed into a cysteine by site-directed mutagenesis. A fluorophore is chemically coupled to the mutant cysteine. The binding of the target shields the fluorophore from the solvent and can therefore be detected by a change of fluorescence.

The Inventors have now developed a new type of RF biosensors which incorporate the advantages of Nanofitins relative to DARPins or antibodies. In particular, they combine, in a single molecule, the favorable properties of antibodies and DARPins, e.g., a wide diversity of ligands and a high stability, solubility and recombinant production yield without their respective disadvantages, e.g. the insufficient stability and inefficient recombinant production of the antibodies and a binding limited to protein targets of the DARPins.

In addition, Nanofitins can bind virtually any target. Therefore, the corresponding RF biosensors extend the range of molecules that can be detected with the existing RF biosensors based upon antibodies and DARPins. Also the inventors have unexpectedly found that RF biosensors with a high sensitivity are obtained more often for the Nanofitins than for the DARPins.

The inventors have also developed methods to design and produce such new RF biosensors when the three-dimensional structure of the complex with the ligand is unknown.

Therefore, a first aspect of the present invention is a reagentless biosensor for a target, comprising at least:

an OB-fold protein;
a cysteine residue coupled to a fluorophore.

According to the present invention, "OB-fold protein" designates any polypeptide which comprises or consists of a domain having an OB-fold topology, as described by (Murzin, 1993) and (Arcus, 2002) and which binds said target specifically. The OB-fold is found in all kingdoms and was ranked as the 28$^{th}$ most represented fold in a survey of 20 genomes (Qian *et al.,* 2001). This topology corresponds to an architecture which comprises a five-stranded β-barrel capped at one end by an amphiphilic α-helix. Referring to the CATH protein structure classification (Pearl *et al.,* 2003), the OB-fold topology corresponds to the 2.40.50 fold family (CATH database version 3.0.0: Released May 2006). The OB-fold presents a β-sheet binding face that confers remarkable diversity in the types of compounds that can be recognized. In addition, the same positions of the binding face of OB-folds from different proteins are always involved in ligand recognition (Arcus, 2002; Mouratou *et al.,* 2007).

Such an OB-fold protein can be either a native protein (*i.e.*, an isolated, purified or recombinant protein having the same sequence as a natural protein), or an engineered protein like, for example, a fragment of a native protein, a variant derived from a native OB-fold protein by mutations, for example in its binding site, a fusion protein comprising an OB-fold domain from a first protein, and another moiety from another protein.

The finding by the inventors that Nanofitins can be used to generate reagentless fluorescent biosensors and in particular that these Nanofitin based biosensors can compare and in some cases outperform, in terms of sensitivity, ease of production and other characteristics, the previous antibody and DARPin based biosensors they generated was unexpected.

The Nanofitins have a constant and small number of hypervariable positions. For example, Nanofitins with high affinity for various protein targets of interest were obtained by randomizing 14 residues of the Sac7d protein from *Sulfolobus acidocaldarius* (WO 2008/068637; Mouratou et al., 2007). The DARPins include a random number of ankyrin modules, exceptionally two and generally three or more (WO 2009/115919; Brient-Litzler *et al.,* 2010). Each ankyrin module has 7 hypervariable positions. Therefore a DARPin has exceptionally 14 and generally 21 or more hypervariable positions. Antibodies have a much larger number of hypervariable positions with their six hypervariable loops. The low number of hypervariable positions in Nanofitins constituted an uncertainty but could be an advantage for the construction of RF biosensors. The inventors have unexpectedly found that despite the small number of hypervariable positions in Nanofitins, only part of them were important for the binding of a specific target and it was possible to couple a fluorophore to the other ones without increasing $K_d$ (*i.e.* decreasing affinity). Given this unexpected result, the small number of hypervariable positions in Nanofitins enables one to directly construct fluorophore conjugates in each of the hypervariable positions and determine their functionality and sensitivity ($s_r$) without having to characterize the properties of changes into Ala of Cys, as for the previous antibody and DARPin based RF biosensors.

In addition, the inventors have found that it was possible to construct an operational RF biosensor by coupling a fluorophore to additional positions that have not been randomized even though they contact the ligand in some natural OB-fold proteins. These positions, such as Lys39 in Sac7d based Nanofitins could represent universal positions for the construction of RF biosensors from Nanofitins.

Therefore several technical differences exist between antibodies, DARPins and Nanofitins. Importantly some of the technical differences between DARPins, antibodies and Nanofitins would probably have led the man skilled in the art to consider Nanofitins inferior to antibodies and DARPins for the preparation of peptide biosensors according to the present invention.

Surprisingly the inventors have now proven that these inherent characteristics of Nanofitins do not affect the performance of Nanofitin based reagentless biosensors and also that such biosensors share the attractive properties of Nanofitins, namely their solubility and stability, their ease of expression, purification and handling, and their wide diversity of ligands.

The inventors therefore provide a new class of RF biosensors which does not necessitate any manipulation, labelling or transformation of the sample. They have the advantages of a reagentless biosensor, namely a biosensor which can function without additional reagent. They can function in complex medium like serum. Their response is immediate, specific, selective, sensitive, linear and quantitative. Thus they can provide quantitative analytical information and follow the concentration of its analyte continuously in complex mixtures, together with the robust bio/physico-chemical properties of Nanofitins and without any apparent loss of sensitivity or binding affinity. The inventors have validated this new class of RF biosensors with the known Lys_H4 (also named H4), a Nanofitin which is directed against hen egg-white lysozyme (HEL; WO 2008/068637).

The inventors have shown that several RF biosensors based upon H4S (H4 mutant in which residue Cys29 has been changed into Ser29) work using the HEL protein as a model target.

Such reagentless fluorescent biosensors can be used in different formats: in solution, in microtiter-plates, in the form of protein chips, or at the tip of optical micro- or nano-fibers. They could be used for the continuous quantification of any target molecule of interest in complex mixtures, without any prior labelling of the target under analysis.

As mentioned above, the RF biosensors of the present invention which are based on Nanofitins have virtually no limitation concerning the target. For example, the target of interest can be a peptide, a protein, an oligosaccharide, a lipid, a lipopeptide, a carbohydrate (for example, a sugar), a single-stranded DNA, a double-stranded DNA, a RNA. Interestingly, the target of interest can be a natural or synthetic small molecule, limited to a few atoms, or even to only one atom. Examples of such small molecules include any kind of haptens (*i.e.*, small molecules which can elicit an immune response only when attached to a large carrier), vitamins, or metallic ions.

In healthcare, they could be used for the bed side monitoring of patients, the controlled continuous delivery of drugs, the control of artificial organs, some diagnostics, *in situ* measurements during surgical operations and the detection of doping drugs.

In industry, they could be used for the monitoring of reactions and processes, food control and pharmacokinetic studies.

In environmental protection/monitoring and civil or military defence, they could be used for the monitoring of pathogenic, toxic or polluting agents.

In fundamental research, they could be used in proteomics, for the profiling of cells, tissues or body fluids; in the biology of single cells, to continuously measure the concentration of an antigen within a single living cell; in neuro-chemistry and neuro-sciences, to measure the intra-cerebral concentration of neuro-peptides in response to external stimuli.

According to a particular embodiment of the invention, the biosensor comprises an OB-fold protein which is derived from a starting OB-fold protein by several substitutions at positions of the residues of the binding interface of said starting OB-fold protein with its native ligand, preferably by 5 to 32, more preferably by 8 to 20, even more preferably by 11 to 16 substitutions, possibly combined with the deletion of 1 to 4 residues and/or the insertion of 1 to 50 residues.

Such an OB-fold protein variant is named "Nanofitin" in the current Patent Application. The Nanofitins are derived from a starting OB-fold protein through the combinatorial mutation/selection process disclosed in WO 2008/068637 (page 2, line 16 to page 9, line 2; examples page 12 to page 34; figures 1 to 21 and sequence listing), the content of which is incorporated herein by reference. This combinatorial mutation/selection process consists in obtaining a combinatorial library corresponding to the randomization of a number of residues involved in the binding of a starting OB-fold protein with its native ligand, followed by a selection, in said library, of variants which bind specifically to a target of interest.

Non-limitative examples of OB-fold proteins which can be used in the construction of a reagentless biosensor according to the current invention are: Sac7d from *Sulfolobus acidocaldarius* (PDB 1azp; UNIPROT or GenBank P13123 or Q4JC17; SEQ ID NO: 1) and the truncated form of Sac7d such as those described by McAfee *et al.,* 1995 (Sac7a (SEQ ID NO: 44) and Sac7b (SEQ ID NO: 45)), Sac7e from *Sulfolobus acidocaldarius* (UNIPROT or GenBank P13125 or Q4JBQ1; SEQ ID NO: 2), Sso7d from *Sulfolobus solfataricus* (PDB lbf4; UNIPROT or GenBank P39476 or P81550; SEQ ID NO: 3), DBP 7 from *Sulfolobus tokodaii* (UNIPROT or GenBank Q96X56; SEQ ID NO: 4), Ssh7b from *Sulfolobus shibatae* (UNIPROT or GenBank 059632; SEQ ID NO: 5), Ssh7a from *Sulfolobus shibatae* (UNIPROT or GenBank P61990, 059631, P80170, Q9UW18; SEQ ID NO: 6), p7ss from *Sulfolobus solfataricus* (UNIPROT or GenBank P61991, 059631, P80170, Q9UW18; SEQ ID NO: 7), the N-terminal domain of SEB (Papageorgiou *et al.,* 1998), the chain A of the Shiga-like toxin IIe of *Escherichia coli* (PDB 2bosA or 1r4pB; SEQ ID NO: 8 and 9), the human Neutrophil Activating Peptide-2 (NAP-2, PDB 1tvxA; SEQ ID NO: 10), the Molybdenum Binding Protein (modg) of *Azotobacter vinelandii* (PDB 1h9j; SEQ ID NO: 11), the N-terminal domain of SPE-C (Roussel *et al.,* 1997), the $B_5$ subunit of *E. coli* Shiga-like toxin (Kitov *et al.,* 2000), Cdc13 (Mitton-Fry *et al.,* 2002), the cold-shock DNA-binding domain of the human Y-box protein YB-1 (Kloks *et al.,* 2002), the *E. coli* inorganic pyrophosphatase EPPase (Samygina *et al.,* 2001), or any of the proteins listed in Table 3 of the article by (Arcus, 2002) or in Table 3 of the present Application, such as the Lysyl-tRNA synthetase LysS of *E.coli* (PDB 1krs; UNIPROT or GenBank B7UHT9 ; SEQ ID NO : 12), the Asp-tRNA synthetase of *E.coli* (PDB 1c0aA; UNIPROT or GenBank P21889; SEQ ID NO: 13), the Asp-tRNA synthetase of *Thermococcus kodakaraensis* (PDB 1b8aA; UNIPROT or GenBank Q52428; SEQ ID NO: 14), the Lysyl-tRNA synthetase LysU of *E.coli* (PDB 1lylA; UNIPROT or GenBank P0A8N6; SEQ ID NO: 15), the human Replication protein A, 32kDa subunit (PDB 1quqA; UNIPROT or GenBank P15927; SEQ ID NO: 16), the human Replication protein A, 14kDa subunit (PDB 1quqB; UNIPROT or GenBank P35244; SEQ ID NO: 17), the human Replication protein A, 70kDa subunit (RPA70) fragment (PDB 1jmcA; UNIPROT or GenBank P27694; SEQ ID NO: 18), the Telomere-end-binding protein of *Oxytricha nova* (PDB 1otcA and 10tcB: UNIPROT or GenBank P29549 and P16458; SEQ ID NO: 19 and 20), the human mitochondrial ssDNA-binding protein (PDB 3ullA; UNIPROT or GenBank Q567R6; SEQ ID NO: 21), the Pertussis toxin S5 subunit of *Bordetella pertussis* (PDB 1prtF; UNIPROT or GenBank P04981; SEQ ID NO: 22), the Pertussis toxin S5 subunit (ATP bound) of *B. pertussis* (PDB 1bcpD; UNIPROT or GenBank P0A3R5; SEQ ID NO: 23), the Cholera Toxin of *Vibrio cholera* (PDB 3chbD; UNIPROT or GenBank D0UTQ9; SEQ ID NO: 24), the Heat-labile toxin of *E. coli* (PDB ltiiD; UNIPROT or GenBank P43529; SEQ ID NO: 25), the Verotoxin-1/Shiga toxin, B-pentamer of *E. coli* (PDB 2bosA; UNIPROT or GenBank Q9MBZ7; SEQ ID NO: 26), the human TIMP-2 (PDB 1br9; UNIPROT or GenBank P16035; SEQ ID NO: 27), the Superantigen SPE-C of *Streptococcus pyogenes* (PDB 1an8 UNIPROT or GenBank Q8NKX2; SEQ ID NO: 28), the Superantigen SPE of *Staphylococcus aureus* (PDB 3seb; UNIPROT or GenBank Q5MAA8; SEQ ID NO: 29), the Toxic shock syndrome toxin of *Staphylococcus aureus* (PDB 1aw7A; UNIPROT or GenBank A0FIN2; SEQ ID NO: 30), the Major cold-shock protein of *E.coli* (PDB 1jmc; UNIPROT or GenBank P0A9Y1; SEQ ID NO: 31), the Initiation translation factor 5a of *Pyrobaculum aerophylum* (PDB 1bkb; UNIPROT or GenBank P56635; SEQ ID NO: 32), the S1 RNA-binding domain of PNPase of *E.coli* (PDB 1sro; UNIPROT or GenBank P05055; SEQ ID NO: 33), the human Initiation translation factor 1, elF1a (PDB 1d7qA; UNIPROT or GenBank P47813; SEQ ID NO: 34), the Initiation translation factor 1, IF1 of *E.coli* (PDB 1ah9; UNIPROT or GenBank P69224; SEQ ID NO: 35), , the RNA guanylyltransferase of Chlorella virus, PBCV-1 (PDB 1ckmA; UNIPROT or GenBank Q84424; SEQ ID NO: 36), the ATP-dependent DNA ligase, of Bacteriophage T7 (PDB 1a0i; UNIPROT or GenBank P00969; SEQ ID NO: 37), the Staphylococcal nuclease, *Staphylococcus aureus* (PDB 1snc; UNIPROT or GenBank gi/224650; SEQ ID NO: 38), the DNA helicase RuvA subunit, N-terminal domain of *E.coli* (PDB 1hjp; UNIPROT or GenBank P0A811; SEQ ID NO: 39), the Gene V protein of *Pseudomonas* bacteriophage pf3 (PDB 1pfsA; UNIPROT or GenBank P03672; SEQ ID NO: 40), the Gene V protein of Filamentous bacteriophage f1, M13 (PDB 1gvp; UNIPROT or GenBank D0U157; SEQ ID NO: 41), the Gene 32 protein (gp32) core of Bacteriophage T4 (PDB 1gpc; UNIPROT or GenBank B3IYU0; SEQ ID NO: 42), and the Inorganic pyrophosphatase of *Thermus thermophilus* (PDB 2prd; UNIPROT or GenBank Q72H95; SEQ ID NO: 43). It can be noted that OB-folds domains originating from toxins can be used as starting OB-fold protein even for purposes in which toxicity is to be avoided, since mutations in their binding site, and hence change in their binding specificity, can completely abolish their toxicity.

By superimposing several sequences and 3D-structures of OB-fold domains using the web sites WU-Blast2 (http://www.ebi.ac.uk/blast2/index.html) (Lopez *et al.,* 2003), T-COFFEE (http://www.ch.embnet.ore/sofiware/TCoffee.html) (Notredame *et al.,* 2000), DALI lite (http://www.ebi.ac.uk/DaliLite/) (Holm and Park, 2000), and DALI (http://www.ebi.ac.uk/dali/Interactive.html) (Holm and Sander, 1998), the inventors have previously identified the positions of the residues involved in the binding of the OB-fold protein which could be substituted for obtaining Nanofitins (WO 2008/068637); these residues are often located in β-strands β3, β4 and β5 and in loops 1, 3 and 4 of the OB-fold (figures 1b and 2 of WO 2008/068637).

Taking as a reference the sequence of Sac7d of SEQ ID NO: 1, the residues which can be substituted are V2, K3, K5, K7, Y8, K9, G10, E14, T17, K21, K22, W24, V26, G27, K28, M29, S31, T33, D36, N37, G38, K39, T40, R42, A44, S46, E47, K48, D49, A50 and P51. Still with Sac7d as a reference, the residues which can be deleted are: A59, R60, A61 and E64. Advantageously, insertions of 1 to 15 amino acid residues can be performed in loop 3 (as identified in figures 1b and 2 of WO 2008/068637), for example in the region of residues 25 to 30 of Sac7d, preferably between residues 27 and 28, insertions of 1 to 15 amino acid residues can be performed in loop 4 (as identified in figures 1b and 2 of WO 2008/068637), for example in the region of residues 35 to 40 of Sac7d, preferably between residues 37 and 38, and insertions of 1 to 20 amino acid residues can be performed in loop 1 (as identified in figures 1b and 2 of WO 2008/068637), for example in the region of residues 7 to 12 of Sac7d, preferably between residues 9 and 10.

The residues of any other OB-fold protein which are located at positions which are equivalent to those listed above can be easily identified by comparing the 3D-structure of said other OB-fold protein with that of Sac7d. For example, the online tool "DaliLite pairwise comparison of protein structures" (http://www.ebi.ac.uk/Tools/dalilite/) was used to superimpose the structures of the protein under consideration on the structure of Sac7d chain A by using their PDB coordinates (PDB code 1AZP for Sac7d chain A). A visual analysis of the superimposed structures with the Pymol software (Pymol; http://www.pymol.org/) was used to identify the correspondences between residues. The residues in positions 7, 8, 9, 21, 22, 24, 26, 28, 29, 31, 33, 39, 40, 42, 44, 46 on one side of Sac7d (SEQ ID NO: 1) OB-fold correspond respectively to the following residues on the corresponding side of the OB-fold of other proteins:

- residues in positions 7, 8, 9, 21, 22, 24, 26, 28, 29, 31, 33, 40, 41, 43, 45, 47 of Sso7d from *Sulfolobus solfataricus* (SEQ ID NO: 3),

- residues in positions 60, 59, 58, 9, 10, 12, 14, 17, 18, 20, 22, X, X, 27, 29, 31 of Shiga-like toxin IIe from *E.coli* (PDB 1r4pB; SEQ ID NO: 9) ;
- residues in positions 25, 26, 27, 40, 41, 43, 45, 54, 55, 57, 59, 61, 63, 65, 67, 69 of the human Neutrophil Activating Peptide-2 (NAP-2, PDB 1tvxA; SEQ ID NO: 10) ;
- residues in positions 60, 61, 62, 86, 87, 89, 91, 93, 94, 96, 98, 105, 106, 108, 110, 112 of the Molybdenum Binding Protein (modg) from *Azotobacter vinelandii* (PDB 1h9j; SEQ ID NO: 11)).

The same approach can be used to engineer a different binding site using the residues from another side of the OB-fold.

In a preferred embodiment of the invention, the biosensor comprises an OB-fold protein which is isolated from a combinatorial library obtained by the randomization of 11, 12, 13, 14, 15, 16, 17 or 18 residues selected amongst those of said OB-fold protein sequence which correspond to K7, Y8, K9, K21, K22, W24, V26, K28, M29, S31, T33, K39, T40, R42, A44, S46, E47 and K48 of SEQ ID NO: 1, for example 11, 12, 13, 14, 15 or 16 residues selected amongst those of said OB-fold protein sequence which correspond to K7, Y8, K9, K21, K22, W24, V26, K28, M29, S31, T33, K39, T40, R42, A44 and S46 of SEQ ID NO: 1.

In a more preferred biosensor of the above embodiment, the randomized residues comprise at least the residues of said OB-fold protein sequence which correspond to K7, Y8, K9, W24, V26, M29, S31, T33, R42, A44 and S46 of SEQ ID NO: 1. A specific library, "library 11" is obtained by the randomization of the residues of said OB-fold protein sequence which correspond to K7, Y8, K9, W24, V26, M29, S31, T33, R42, A44 and S46 of SEQ ID NO: 1. In other combinatorial libraries, one, two or three additional residues selected amongst those of said OB-fold protein sequence which correspond to K21, K22 and T40 of SEQ ID NO: 1 are also randomized. Two other preferred libraries are "library 13", which is obtained by the randomization of the residues of said OB-fold protein sequence which correspond to are K7, Y8, K9, K21, K22, W24, V26, M29, S31, T33, R42, A44 and S46 of SEQ ID NO: 1, and "library 14", which is obtained by the randomization of the residues said OB-fold protein sequence which correspond to K7, Y8, K9, K21, K22, W24, V26, M29, S31, T33, T40, R42, A44 and S46 of SEQ ID NO: 1.

According to another particular embodiment of the invention, the Biosensor comprises an OB-fold protein which is selected from the group consisting of: Sac7d or Sac7e from *Sulfolobus acidocaldarius* (SEQ ID NO : 1 and 2), Sso7d from *Sulfolobus solfataricus* (SEQ ID NO : 3), DBP 7 from *Sulfolobus tokodaii* (SEQ ID NO : 4), Ssh7b from *Sulfolobus shibatae* (SEQ ID NO : 5), Ssh7a from *Sulfolobus shibatae* (SEQ ID NO : 6), p7ss from *Sulfolobus solfataricus* (SEQ ID NO : 7), Sac7a (SEQ ID NO: 44), Sac7b (SEQ ID NO: 45), and the Nanofitins derived from the preceding OB-fold proteins as defined in the current Patent Application..

A first more preferred Biosensor according to the invention comprises a Nanofitin which binds human immunoglobulins of class G (IgG) with high affinity (Kd ≤100 μM), said Nanofitin having a sequence selected from:

SVKVKF$X_1X_2X_3$GEEKEVDTSKIRDVCRQGKNVKFLYNDNGK YGAG$X_4$V$X_5$EKDAPKELLDMLARAEREKK (SEQ ID NO: 46), wherein $X_1$ to $X_5$ are, independently from each other, any amino acids, and the variants derived from SEQ ID NO: 11 by 1 to 15, more particularly one, two, three, four, five or six mutations or deletions in positions selected in the group consisting of positions 1-6, 11-20, 23, 25, 27-28, 30, 32, 36-39, 41, 43, 45 and 47-66. Particular examples of such variants are those in which said one, two, three or four mutations or deletions are in positions selected in the group consisting of positions 13, 24, 36, 38, 47, 50 and 56 (mutations in positions 13, 36, 38, 47, 50 and 56 have been obtained by ribosome display, and position 24 was voluntarily mutated to avoid S-S bridging. Preferably, $X_1X_2X_3$ is selected in the group consisting of LLN, KYK, HSH, LCH, RYL and ARH. Simultaneously or independently, preferred immunoglobulin-binding proteins are so that $X_4$ is N or K, and/or $X_5$ is D, N or R.

Particular examples of immunoglobulin-binding proteins are:

SVKVKFLLNGEEKEVDTSKIRDVCRQGKNVKFLYNDNGKYG AGNVDEKDAPKELLDMLARAEREKK (SEQ ID NO: 47),
SVKVKFLLNGEEKEVDTSKIRDVCRQGKNVKFLYNDNGKYG AGKVNEKDAPKELLDMLARAEREKK (SEQ ID NO: 48),
SVKVKFHSHGEEKEVDTSKIRDVCRQGKNVKFLYNDNGKY GAGNVDEKDAPKELLDMLARAEREKK (SEQ ID NO: 49),
SVKVKFLCHGEEKEVDTSKIRDVCRQGKNVKFLYNDNGKY GAGNVDEKDAPKELLDMLARAEREKK (SEQ ID NO: 50),
SVKVKFRYLGEEKEVDTSKIRDVCRQGKNVKFLYNDNGKY GAGNVDEKDAPKELLDMLARAEREKK (SEQ ID NO: 51),
SVKVKFARHGEEKEVDTSKIRDVCRQGKNVKFLYNDNGKY GAGNVREKDAPKELLDMLARAEREKK (SEQ ID NO: 52),
SVKVKFLLNGEEKEVDTSKIRDVCRQGKNVKFLYNDNGKYG AGNVDEKDVPKELLDMLARAEREKK (SEQ ID NO: 53),
SVKVKFLLNGEEQEVDTSKIRDVCRQGKNVKFLYNDNGKYG AGNVDEKDAPKELLDMLARAEREKK (SEQ ID NO: 54),
SVKVKFLLNGEEKEVDTSKIRDVCRQGKNVKFLYNDNGKYG AGNVDKKDAPKELLDMLARAEREKK (SEQ ID NO: 55),
SVKVKFLLNGEEKEVDTSKIRDVCRQGKNVKFLYNDNDKYG AGNVDEKDAPKELLDMLARAEREKK (SEQ ID NO: 56), and
SVKVKFLLNGEEKEVDTSKIRDVCRQGKNVKFLYNNNGKYG AGNVDEKDAPKELLEMLARAEREKK (SEQ ID NO: 57).

Another more preferred Biosensor according to the invention comprises a Nanofitin of: SEQ ID NO: 58 (H4S; Hen egg-white lysozyme binder).

According to another particular embodiment of the invention, the Biosensor comprises a Nanofitin which is derived from a starting OB-fold protein which is selected from the group consisting of the sequences SEQ ID NO: 8 to 42 and the fragments of said sequences which include at least the OB-fold domain (the positions of the OB-fold domain of these sequences is mentioned in Table 3 and in the attached sequence listing).

According to another particular embodiment of the invention, the biosensor according to the invention comprises an OB-fold protein which is coupled to another protein moiety. The coupling between the two moieties can be either a genetic fusion (the chimeric protein is then a fusion protein), or a chemical binding. In the later case, the binding can be either covalent or non-covalent. For example, the OB-fold protein is fused at its N- or C-terminal ends or both to another moiety selected among: a binding moiety with different specificities than the OB-fold protein such as a peptide tag (His*6, FLAG, HA, myc, *etc*.), a tag for *in vivo* biotinylation (provided BirA is co-expressed) to enable oriented immobilization on a support (such as a protein chip), a poly-lysine tag (for example, Lys*6) for enabling oriented immobilization on a support (such as an affinity chromatography resin); a targeting moiety such as a signal sequence (for example, to address the chimeric protein to a specific compartment like periplasm or nucleus). Advantageously, the fusion will encompass a peptide linker between the two moieties, which will be long enough to avoid inhibiting interactions between both moieties. The linker can optionally comprise a recognition site for a protease, if easy separation of both moieties is wanted. For example, the linker can comprise the sequence Glu-Asn-Leu-Tyr-Phe-Gln-Gly, which is the optimum recognition site for TEV protease (a highly site-specific cysteine protease that is found in the Tobacco Etch Virus). This enzyme has a high specificity and a high activity rate and triggers cleavage between the Gln and Gly residues of the above recognition site. It can hence be used, for example, for removing affinity tags from a purified chimeric protein according to the present invention.

In particular in the biosensor according to the present invention, the cysteine residue which is coupled to a fluorophore is either present in said OB-fold protein or is substituted for another suitable residue. In addition, nonessential cysteine residues which are present in said OB-fold protein are substituted with small residues like serine or alanine residues by site-directed mutagenesis.

The site for the coupling of the fluorophore can be chosen using, either the rational approaches previously developed by the inventors for antibody and DARPin based RF biosensors, or a new approach that the inventors have developed for Nanofitins according to a further aspect of the invention.

When structural data of the OB-fold protein in complex with its target are known the coupling site corresponds to a residue which is not in direct contact with the target but whose solvent accessible surface area (ASA) is modified when the OB-fold protein binds to the target. The methods previously described in WO2001/065258, WO2009/115919, Renard *et al.,* 2002, Renard and Bedouelle 2004 and Brient-Litzler *et al.,* 2010 for producing antibody or DARPin based biosensors using this design methodology can be used for producing OB-fold protein/Nanofitin based RF biosensors according to the current Patent Application.

However, structural data are rarely available and in the case of a newly generated Nanofitin with a selected specificity such structural data will not be available. The prior art methodology for choosing the coupling sites when no structural data are available relies on the identification of at least one residue which is adjacent (for example by reference to its sequence or to a canonical structure) to a residue which is functionally important for interaction with the target. Methods for producing antibody or DARPin based biosensors using this design methodology are described in WO2001/065258, WO2009/115919 and Renard *et al.,* 2003. These methods comprise: (i) the identification of the residues ($R_1$) of the biosensor which make significant contribution to target binding by mutagenesis of all, or of a subset, of the residues of the biosensor and determining variations in at least one measurable chemical or physical parameter of interaction with said target ($K_D$, $K_{off}$, $k_{on}$, $\Delta\Delta G$, $R_{eq}$), (ii) the modification to cysteine of at least one of a second set of residues ($R_2$) which are located in sequence proximity or an in spatial proximity to one or more residues of the first set ($R_1$), and (iii) the coupling of the modified biosensor to a fluorophore at this cysteine.

The adjacent residues ($R_2$) are preferably the residues -1 and +1 along the peptide backbone relative to one or more of the residues ($R_1$) or the residues in Van-Der-Waals contact which one or more of the residues ($R_1$).

Such methods can be used for producing OB-fold protein/Nanofitin based RF biosensors according to the current Patent Application.

According to another particular embodiment of the invention, the biosensor has a fluorophore coupled to one residue of said OB-fold protein selected from the residues corresponding to V2, K3, K5, K7, Y8, K9, G10, E14, T17, K21, K22, W24, V26, G27, K28, M29, S31, T33, D36, N37, G38, K39, T40, R42, A44, S46, E47, K48, D49, A50, and P51 of SEQ ID NO: 1, the residue being changed to a cysteine residue if it is not already a cysteine residue.

In a first preferred embodiment of the invention, the fluorophore is coupled to one residue of said OB-fold protein selected from the residues corresponding to K7, Y8, K9, K21, K22, W24, V26, M29, S31, T33, T40, R42, A44 and S46 of SEQ ID NO: 1. Specific examples of said embodiment include the biosensors consisting of SEQ ID NO: 59, 60, 61 and 62 in which one of the residues W8, V21, K24 and A26 of H4S has been substituted with a cysteine residue and coupled to a fluorophore.

In a second preferred embodiment of the invention, the fluorophore is coupled to one residue of said OB-fold protein corresponding to K39 of SEQ ID NO: 1. This position is a universal position which can be used advantageously to engineer biosensors from OB-fold protein variants isolated from libraries which are randomized at other positions (library 11, 13 or 14 as described in the current Patent Application). Non-limitative examples of OB-fold protein variants include variants of Sac7d and its homologs like Sso7d, Shiga-like toxin IIe, human Neutrophil Activating Peptide-2 and Molybdenum Binding Protein. A specific example of said embodiment is the biosensor consisting of SEQ ID NO: 63, in which the residue K39 of H4S has been substituted with a cysteine residue and coupled to a fluorophore.

A fluorophore is a component of a molecule which causes a molecule to be fluorescent. It is a functional group in a molecule which will absorb energy of a specific wavelength and re-emit energy at a different (but equally specific) wavelength. The amount and wavelength of the emitted energy depend on both the fluorophore and the chemical environment of the fluorophore.

In particular the fluorophore is selected from the group consisting of: 6-acryloyl-2-dimethylaminophtalene (acrylodan), 4-chloro-7-nitrobenz-2-oxa-1,3-diazole (CNBD), 5-iodoacetamidoflurescein (5-IAF), (N-((2-(iodoacetoxy) ethyl)-N-methyl)amino-7-nitrobenz-2-oxa-1,3-diazole (IANBD ester), Cy3, Cy5 or a fluorophore having an aliphatic chain of 1 to 6 carbon atoms.

Another aspect of the present invention is a protein-based chip, characterized in that it consists of a solid support on which at least one biosensor as described in the current Patent Application is immobilized.

Another aspect of the present invention is a solution comprising at least one biosensor as described in the current Patent Application.

Another aspect of the present invention is an optical fibre comprising at a first end thereof at least one biosensor as described in the current Patent Application and comprising at a second end thereof means to attach the optical fibre to a device configured to receive and interpret the output of the at least one biosensor.

Another aspect of the invention is the use of a biosensor as described in the current Patent Application for screening libraries of molecules, for sorting molecules, for sorting cells, for producing protein-based chips, for detecting, assaying or locating a target.

Another aspect of the invention is a reagent for detecting, assaying or locating targets, characterized in that it includes at least one biosensor as described in the current Patent Application.

Another aspect of the invention is a method for detecting, assaying or locating a ligand in a heterogeneous sample, characterized in that it comprises bringing said heterogeneous sample into contact with at least one reagent as described in the current Patent Application.

Another aspect of the invention is a kit for detecting, assaying or locating a ligand in a heterogeneous sample, characterized in that it includes at least one reagent as described in the current Patent Application.

Another aspect of the invention is a method for producing reagentless biosensors which comprise at least an OB-fold protein and are specific for a target, characterized in that it comprises the following steps:

(a) substituting each of the variable residues of said OB-fold protein, individually with a cysteine residue, thereby obtaining a set of cysteine mutants,

(b) coupling said cysteine residue of each of the cysteine mutants in a) to a fluorophore, thereby obtaining biosensors,

(c) purifying the biosensors of step (b),

(d) measuring the equilibrium constant ($K_D$) between each of said purified biosensors and said target, or the dissociation ($k_{off}$) and association ($k_{on}$) rate constants for each of said biosensors and said target; and measuring the fluorescence variation of each of said biosensors between a free and target bound state; and

(e) determining the sensitivity ($s$) and/or relative sensitivity ($s_r$) of each of said biosensors from the measurements of step (d).

This method makes it possible to rank the biosensors according to their sensitivity and to determine the biosensors having the highest sensitivity, a parameter which is used to characterize any measuring instrument.

The variable residues in step (a) correspond to the residues of the binding interface of the starting OB-fold protein with its native ligand which are randomized in the combinatorial libraries of OB-fold protein variants which are used to isolate Nanofitins which bind to the target as described in the current Patent Application.

In particular prior to step (b), the cysteine mutants obtained in step (a) are subjected to a controlled chemical reduction.

In particular the biosensor may be purified in a soluble form.

In particular after step (c) or step (d) or step (e), the method comprises an additional step of immobilizing said biosensor on a solid support.

In particular after step (e), the method comprises an additional step of ranking the biosensors according to their sensitivity.

The OB-fold proteins which are used to generate new target specific Nanofitins comprise a canonical polypeptide backbone (OB-fold) in which some of the residues are fixed to provide the characteristic five stranded β-barrel capped with an amphiphilic α-helix and some of the residues of the β-sheet binding face that have a strong probability of contributing to target binding are varied, known as variable residues, in a random or semi random fashion so as to alter the binding properties of the Nanofitin.

The inventors have found that by focussing efforts upon these variable residues the method for producing biosensors works more efficiently. In addition, the inventors have found unexpectedly that this methods works with Nanofitins which have a small number of variable residues. For this reason, said method is particularly advantageous for making Nanofitin-based biosensors.

This method can be used also for producing reagentless biosensors from other natural or artificial families of target binding proteins which are similarly constructed like for examples antibody Fv fragments and DARPins.

For a better understanding of the invention and to show how the same may be carried into effect, there will now be shown by way of example only, specific embodiments, methods and processes according to the present invention with reference to the accompanying drawings in which:

**Figure 1.** Shows the titration of H4S conjugates, monitored by fluorescence. The experiments were performed at 25 ˚C in buffer C. The total concentration of H4S conjugate, measured by $A_{280}$, was equal to 0.3 μM. The total concentration in homologous (HEL) or heterologous (BSA) antigen is given along the x axis. The continuous curves correspond to the fitting of Equation 5 to the experimental values of $\Delta F/F_0$ (see Materials and Methods for details). HEL as antigen: closed diamonds, fluorophore at position Trp8; closed triangles, Val21; closed circles, Lys24; open circles, Ala26; open triangles, Lys39. BSA as antigen: open diamonds, Lys24.

**Figure 2.** Shows the relative sensitivities $s_r$ of the H4S conjugates at 25 ˚C in buffer C as a function of their concentration. This figure is a plot of Equation 8, using the parameters listed in Table 1. The $s_r$ parameter relates the relative variation of fluorescence intensity $\Delta F/F_0$ and the relative concentration of antigen $[A]_0/[B]_0$ for the low values of $[A]_0$, where $[A]_0$ and $[B]_0$ are the total concentration of antigen and conjugate in the binding reaction, respectively (Equations 7 and 8). Closed diamonds, fluorophore at position Trp8; closed triangles, Val21; closed circles, Lys24; open circles, Ala26; open triangles, Lys39; open diamonds, Tyr42.

**Figure 3.** Shows the effect of the concentration in serum on the fluorescence signals for conjugates between IANBD and either H4S(K24C) or 2-mercaptoethanol. The experiments were performed in a mixture (v:1-v) of serum and buffer C. The total concentration of conjugate was equal to 0.3 μM. The total concentration of HEL was equal to 10 μM and thus saturating. Open squares, 2-mercaptoethanol-ANBD (the results were identical in the presence or absence of HEL); open circles, H4S(K24ANBD) without HEL; closed circles, H4S(K24ANBD) with HEL. AU, arbitrary units of fluorescence. The continuous curves were drawn only for clarity.

**Figure 4.** Shows the positions of the hypervariable positions in a structural model of H4S. The model was created with the Swiss Model program in the alignment mode and the crystal structure of the Sac7d protein at a resolution of 1.6 Å (PDB: 1azp) as a template (Arnold *et al.,* 2006; Robinson et al., 1998). Dark grey, positions where the coupling of the fluorophore strongly decreased the free energy of interaction between H4S and HEL ($\Delta\Delta G \geq 1.5$ kcal $mol^{-1}$); medium grey, positions where the coupling mildly decreased the energy of interaction ($0.5 \leq \Delta\Delta G \leq 1.2$ kcal $mol^{-1}$) and resulted in the most sensitive conjugates; light grey, positions where the coupling did not affect the interaction and resulted in little sensitive or unsensitive conjugates.

**Figure 5.** Shows quenching of the H4S(K24ANBD) fluorescence by KI. *F* and $F^0$, fluorescence of the conjugate with and without quencher respectively. The concentration of conjugate was equal to 0.3 μM and the experiments were performed at 25 ˚C in buffer C. The continuous curves were obtained by fitting Equation 11 to the experimental data. Closed circles, conjugate in the absence of HEL; open circle, conjugate in the presence of a saturating concentration of HEL (10 μM). The corresponding Stern-Volmer constants $K_{SV}$ were equal to $6.7 \pm 0.1$ $M^{-1}$ and $2.5 \pm 0.1$ $M^{-1}$ respectively (values ± SE in the fitting).

## EXAMPLE 1: MATERIALS AND METHODS

### 1.1 Materials and genetic constructions

Phosphate buffered saline (PBS), bovine serum albumin (BSA), hen egg-white lysozyme (HEL), calf serum, dithiothreitol (DTT) and Tween 20 were purchased from Sigma; N-((2-(iodoacetoxy)ethyl)-N-methyl)amino-7-nitrobenz-2-oxa-1,3-diazole (IANBD ester), LB broth and 2-YT broth from Invitrogen; the *Escherichia coli* strain NEB-Express-$I^q$ from New England Biolabs; the plasmid vector pQE30 and the fast-flow NiNTA resin from Qiagen. A stock solution of the IANBD ester was made at a concentration of 10 mg/mL in dimethylformamide. Ampicillin was used at a concentration of 100 μg/mL and chloramphenicol at 10 μg/mL. Buffer A was 500 mM NaCl, 20 mM imidazole, 50 mM Tris-HCl, pH 8.0; buffer B, 500 mM NaCl, 200 mM imidazole, 50 mM Tris-HCl, pH 8.0; buffer C, 150 mM NaCl, 50 mM Tris-HCl, pH 7.4; buffer D, 0.005% (v/v) Tween 20, 0.1 mg/mL BSA in buffer C; buffer E, 5 mM DTT in buffer D.

The H4S protein is a recombinant derivative of the Sac7d protein from *Sulfolobus acidocaldarius* that has been evolved by ribosome display to bind hen egg-white lysozyme HEL (WO 2008/068637). Its sequence is identical to that previously published, except that residue Cys29 has been changed into Ser29: NH2-MRGSHHHHHHGSVKVKFFWNGEEKEVDTSKIVWVKRAGKSVLFIYDD NGKNGYGDVTEKDAPKELLDMLARAEREKKLN-COOH (SEQ ID NO: 58). Note that the numbering does not take the first 11 residues into account. Plasmid pH4S (SEQ ID NO: 64), which codes for the H4S protein, is a derivative of pQE30 (Qiagen). Changes of residues were introduced into H4S at the genetic level, by mutagenesis of pH4S with the Quickchange II site-directed mutagenesis kit (Stratagene).

### 1.2 Protein production, purification and characterization

The parental protein H4S(wt) and its mutant derivatives were produced in the cytoplasm of the recombinant strain NEB-ExpreSS_$I^q$(pH4S) and its mutant derivatives as follows. The producing strains were grown at 30 ˚C. They were streaked on plates of LB agar, supplemented with ampicillin and chloramphenicol. A pre-culture in 2-YT broth, supplemented with the same two antibiotics, was inoculated with an isolated colony and grown overnight. A larger culture (650 mL), supplemented with ampicillin alone, was inoculated with an aliquot of the preculture (30 mL), grown until $A_{600nm}$ = 0.7, induced with 1 mM IPTG, and then grown further for 24 hours. The following purification steps were performed at 4 ˚C. The culture was chilled on ice, centrifuged at 8000 g for 20 min and the resulting pellet was frozen and kept at -20 ˚C. The pellet was thawed, resuspended in 30 mL of Buffer A, and the cells were disintegrated by sonication. The lysate was centrifuged at 8600 g for 30 min and the supernatant filtered through a 0.22 μm Millex filter (Millipore). H4S and its derivatives were purified through their hexahistidine tag by affinity chromatography on a column of Ni-NTA resin. The proteins were eluted from the resin with Buffer B. The purification fractions were analyzed by SDS-PAGE, in the presence or absence of 2.5 % (v/v, 0.4 M) 2-mercaptoethanol, and the protein bands were quantitated with the Un-scan-it software (Silk Scientific) as described (Brient-Litzler et al., 2010). The pure fractions (> 98 % homogeneous in reducing conditions), were pooled and kept at -80 ˚C. The protein concentrations were measured by absorbance spectrometry. The coefficients of molar extinction were calculated from the amino acid sequences with the ExPAsy ProtParam tool (Gasteiger et al., 2003). All the binding experiments were performed at 25 ˚C.

### 1.3 Fluorophore coupling

The conjugates between the cysteine mutants of H4S (Nanofitin) and the IANBD ester were prepared as follows. The H4S mutants were reduced with 5 mM DTT for 30 min at 30˚C with gentle shaking and then the buffer was exchanged to PBS by size exclusion chromatography with a PD10 column (GE Healthcare). From this point on, all the experiments were done in the dark. The thiol-reactive fluorophore IANBD ester was added in a 10:1 molar excess over the Nanofitin and the coupling reaction was carried out for 2.5 hours at 30 ˚C with gentle shaking. The denatured proteins were removed by centrifugation for 30 min at 13200 g, 4 ˚C. The conjugate was separated from the unreacted fluorophore by chromatography on a NiNTA column (0.5 mL of resin) and eluted with Buffer B. The conjugate between 2-mercaptoethanol and the IANBD ester was prepared by mixing the two molecules in stoechiometric amounts and then incubating the mixture for 30 min at 25 ˚C. The coupling yield $y_c$, i. e. the average number of fluorophore molecule coupled to each Nanofitin molecule, was calculated as described below, with $\varepsilon_{280}$(ANBD) = 2100 $M^{-1}$ $cm^{-1}$ and $\varepsilon_{500}$(ANBD) = 31800 $M^{-1}$ $cm^{-1}$, both measured with a conjugate between IANBD and 2-mercaptoethanol (Renard *et al.,* 2002).

Let P be a protein; B, a monoconjugate between P and IANBD; Φ, the conjugated form of IANBD; $A_{280}$ and $A_{500}$, the absorbances of the mixture of P and B that results from the coupling reaction and elimination of the unconjugated fluorophore. By definition,

$$y_c = [B]/([B] + [P]) \quad (1)$$

where [B] and [P] are concentrations. Then, the reciprocal of the coupling yield is given by the following equation where ε is a molar absorbance (Brient-Litzler *et al.,* 2010):

$$y_c^{-1} = (A_{280}/\varepsilon_{280}(P))(A_{500}/\varepsilon_{500}(\Phi))^{-1} - \varepsilon_{280}(\Phi)/\varepsilon_{280}(P) \quad (2)$$

### 1.4 Fluorescence measurements and antigen binding

The binding and fluorescence experiments were performed at equilibrium as if the preparations of conjugates were homogeneous, i. e. as if $y_c = 1$. The binding reactions were conducted by incubating 0.3 μM of conjugate with variable concentrations of the HEL or BSA antigens in a volume of 1 mL, for 30 min in the dark with gentle shaking. The reactions were carried out in Buffer C or in a mixture v:(1-v) of calf serum and Buffer C as indicated in example 2.

The conjugate (or biosensor) B and antigen A form a 1:1 complex B:A according to the reaction:

$$B + A \leftrightarrow B{:}A \quad (3)$$

At equilibrium, the concentration [B:A] of the complex is given by the equation:

$$[B{:}A] = 0.5\{[B]_0 + [A]_0 + K_d - (([B]_0 + [A]_0 + K_d)^2 - 4\,[B]_0[A]_0)^{1/2}\} \quad (4)$$

where $K_d$ is the dissociation constant, and $[A]_0$ and $[B]_0$ are the total concentrations of A and B, respectively (Renard *et al.,* 2003).

The fluorescence of the IANBD conjugates was excited at 485 nm (5 nm slit width) and its intensity measured between 520 and 550 nm (20 nm slit width) with a FP-6300 spectrofluorometer (Jasco). The signal of HEL alone or BSA alone was measured in an independent experiment and subtracted from the global signal of the binding mixture to give the specific fluorescence intensity *F* of each conjugate. The intensity *F* at a given value of $[A]_0$ satisfies the following equation:

$$(F - F_0)/F_0 = \Delta F/F_0 = (\Delta F_\infty/F_0)([B{:}A]/[B]_0) \quad (5)$$

where $F_0$ and $F_\infty$ are the values of *F* at zero and saturating concentrations of A *(Renard et al.,* 2003). The values of $\Delta F_\infty/F_0$, $K_d$, and $[B]_0$ could be determined by fitting Equation 5, in which [B:A] is given by Equation 4, to the experimental values of $\Delta F/F_0$, measured in a titration experiment. The fittings were performed with the Kaleidagraph software (Synergy Software).

The sensitivity *s* and relative sensitivity $s_r$ of a conjugate can be defined by the following equations for the low values of $[A]_0$, i. e. in the initial part of the titration curve:

$$\Delta F = s[A]_0 \quad (6)$$

$$\Delta F/F_0 = s_r[A]_0/[B]_0 \quad (7)$$

*s* and $s_r$ can be expressed as functions of characteristic parameters of the conjugate:

$$s_r = (\Delta F_\infty/F_0)([B]_0/(K_d + [B]_0)) \qquad (8)$$

$$s = f_b s_r \qquad (9)$$

where $f_b = F_0/[B]_0$ is the molar fluorescence of the free conjugate (Renard and Bedouelle, 2004). Equation 8 implies that $s_r$ is maximal when the biosensor B is present at a concentration $[B]_0 >> K_d$, as every newly titrated molecule A then forms a complex. Equation 6 implies that the lower limit of detection $\delta[A]_0$ of the conjugate is linked to the lower limit of measurement of the spectrofluorometer $\delta F$ by the following equations:

$$\delta[A]_0 = s^{-1}\delta F = s_r^{-1}[B]_0(\delta F/F_0) \qquad (10)$$

**1.5 Quenching by potassium iodide**

The experiments of fluorescence quenching by KI were performed at 25˚C in Buffer C, essentially as described above. The Stern-Volmer Equation 11 was fitted to the experimental data, where $F$ and $F^0$ are the intensities of fluorescence for the H4S conjugate in the presence or absence of quencher, respectively. The Stern-Volmer constant $K_{sv}$ was used as a fitting parameter.

$$F^0/F = 1 + K_{SV}\,[KI] \quad (11)$$

**1.6 Affinity in solution as determined by competition Biacore**

The affinity in solution between H4S and HEL was determined with the following modifications of a published procedure (Brient-Litzler *et al.,* 2010). The binding reactions (250 μl) were conducted by incubating 20 nM of H4S with variable concentrations of HEL for 30 min in buffer D. The concentration of free H4S was then measured by surface plasmon resonance with a Biacore 2000 instrument (Biacore). A high density of HEL (1700 Resonance Units, RU) was immobilized on the surface of a CM5 sensorchip (GE Healthcare) and each reaction mixture was injected in the sensor chip at a flow rate of 30 μL $min^{-1}$. The chip surface was regenerated between the runs by injecting 10 μL of a 0.05 % SDS solution.

**1.7 Kinetic measurements by Biacore**

The kinetics were measured in buffer D at a flow rate of 30 μL $min^{-1}$ with CM5 sensor chips. A first cell of the sensor chip was used as a reference, i.e. no ligand was immobilized on the corresponding surface. A second cell was loaded with 500-1000 resonance units (RU) of HEL. Solutions (200 μL) of the H4S derivatives at 15 different concentrations (1 nM to 6 μM) were injected to monitor association and then buffer alone (150 μL) for dissociation. The chip surface was regenerated between the runs by injecting 10 μL of a 0.05% SDS solution. The signal of the buffer alone was subtracted from the raw signals to obtain the protein signals, and then the protein signal on cell 1 was subtracted from the protein signal on cell 2 to obtain the specific signal of interaction. The kinetic data were cleaned up with the Scrubber program (Biologic Software) and then the kinetic parameters were calculated by applying a simple kinetic model of Langmuir binding and a procedure of global fitting, as implemented in the Bia-evaluation 4.1 software (Biacore).

**EXAMPLE 2: BIOSENSORS FROM NANOFITINS-RESULTS USING BIOSENSORS DERIVED FROM H4S**

**2.1 Production and oligomeric state of cysteine mutants**

The artificial family of Nanofitins is derived from Sac7d, an OB-fold protein, and comprises 14 randomized positions. Two additional positions, 28 and 39, have not been randomized although the residues at the corresponding positions form contacts between some OB-fold proteins and their cognate partners (Mouratou et *al.,* 2007). The residues at the 14 fully randomized positions of the H4S protein, directed against HEL, and at its positions Lys28 and Lys39 were changed individually into cysteine by site-directed mutagenesis of the coding gene. The parental protein, H4S(wt), and its mutant derivatives were produced in the cytoplasm of *E. coli* and purified through their hexahistidine tag. The yields of purified soluble protein varied between 4 mg/L and 46 mg/L of culture in flask, with an average of 18 ± 3 mg/L (mean ± standard error). The yields for the mutant proteins were not very different from the yield for the parental protein, i. e. 40 mg/L.

The introduction of a cysteine residue could lead to intermolecular disulfide bonds. To characterize the oligomeric state of the H4S mutants, their purified preparations were analyzed by SDS-PAGE after denaturation in the presence or absence of a reducing agent. Under reducing conditions, a single protein species was observed with an apparent molecular mass that was consistent with the theoretical mass of an H4S(wt) monomer, 9146.3. Under non-reducing conditions, a second species was observed with an apparent molecular mass that was consistent with the theoretical mass of a dimer. The proportion of protomers in a dimeric state was calculated from the intensities of the protein bands. It was ≤ 20 % for the five mutants that we analyzed.

### 2.2 Conjugation and its yield

The purified preparations of the H4S mutants was submitted to a reaction of reduction before coupling with the thiol reactive fluorophore N-((2-(iodoacetoxy)ethyl)-N-methyl)amino-7-nitrobenz-2-oxa-1,3-diazole (IANBD ester), to break open the potential intermolecular disulfide bonds and ensure that the mutant cysteine residue would be in a reactive state. The products of the coupling reaction were separated from the unreacted fluorophore by chromatography on a nickel ion column. The coupling yield $y_c$, defined as the number of fluorophore groups per H4S molecule, was calculated from the absorbance spectra of the purified reaction product (Materials and Methods; Table 1). It varied from 43 to 90 % (72 ± 3, mean ± SE). Such position dependent variations have already been observed for other Antigen Binding Proteins (AgBPs; Brient-Litzler *et al.,* 2010; Renard *et al.,* 2003; Renard et *al.,* 2002). The synthesis yield $y_s$ of the coupling procedure, i. e. the proportion of protein molecules that remained at the end of the procedure, was similar for all the H4S mutants, 65 ± 3 % (mean ± SE).

**Table 1.**

Table 1 shows the properties of H4S conjugates, as derived from fluorescence experiments. Column 1, residue with which the fluorophore was coupled. $y_c$, number of molecules of fluorophore per molecule of H4S in a purified preparation of the conjugate (coupling yield). $\lambda_{max}$, wavelength of the maximal value of $F_0$. $f_b$, molar fluorescence of the free conjugate at $\lambda_{max}$. The total concentration of conjugate was equal to $0.3y_c$ μM. $\Delta F_\infty/F_0$, maximal variation of $F$ at $\lambda_{max}$. The entries for $\Delta F_\infty/F_0$ and $K_d$ give the value and associated SE from the fitting of Equation 5 to the data points in the titration experiments. $s_r$, relative sensitivity of the conjugate at a fixed concentration of 0.3 μM. nm, not measurable. The fluorescence experiments were performed in buffer C. The experiments for Trp8, Val21, Lys24, Ala26, Ser29, Leu31 and Lys39 were performed in duplicate, with nearly identical results. The Pearson parameter in the fittings was $R$ > 0.992 except for Phe7 ($R$ = 0.98) and Trp22 ($R$ = 0.72). The $K_d$ value for H4S(wt) was equal to 40.3 ±1.6 nM, as measured by competition Biacore in buffer D (see Materials and Methods for details).

| Residue | $y_c$ | $\lambda_{max}$ (nm) | $f_b$ (FU $\mu M^{-1}$) | $\Delta F_\infty/F_0$ | $K_d$ (nM) | $s_r$ |
|---|---|---|---|---|---|---|
| Phe7 | 0.86 | 539.0 | 1760 | 0.26±0.01 | 42±13 | 0.22 |
| Trp8 | 0.72 | 536.5 | 427 | 1.47±0.04 | 189±50 | 0.90 |
| Asn9 | 0.43 | 539.0 | 232 | 0.52±0.01 | 7±2 | 0.50 |
| Val21 | 0.87 | 537.0 | 531 | 6.30±0.04 | 296±19 | 3.17 |
| Trp22 | 0.77 | 529.5 | 4375 | 0.3±0.1 | 1226±1141 | 0.06 |
| Lys24 | 0.68 | 536.0 | 433 | 8.8±0.1 | 98±17 | 6.61 |
| Ala26 | 0.54 | 539.0 | 664 | 7.9±0.1 | 206±27 | 4.66 |
| Lys28 | 0.75 | 537.5 | 1888 | 0.26±0.01 | 28±8 | 0.24 |
| Ser29 | 0.73 | 538.5 | 1428 | 0.55±0.01 | 33±6 | 0.50 |
| Leu31 | 0.80 | 538.0 | 193 | 4.9±0.4 | 8356±2754 | 0.17 |
| Ile33 | 0.90 | 537.5 | 191 | nm | nm | nm |
| Lys39 | 0.68 | 538.5 | 764 | 1.65±0.02 | 126±14 | 1.16 |
| Asn40 | 0.65 | 537.5 | 458 | 0.57±0.05 | 2004±894 | 0.07 |
| Tyr42 | 0.69 | 537.5 | 286 | 6.6±0.2 | 2479±487 | 0.72 |

(continued)

| Residue | $y_c$ | $\lambda_{max}$ (nm) | $f_b$ (FU $\mu M^{-1}$) | $\Delta F_\infty/F_0$ | $K_d$ (nM) | $s_r$ |
|---|---|---|---|---|---|---|
| Asp44 | 0.70 | 537.0 | 689 | 1.50±0.04 | 623±106 | 0.49 |
| Thr46 | 0.85 | 538.5 | 1922 | 0.36±0.01 | 100±32 | 0.27 |

### 2.3 Fluorescence properties of the conjugates

The free conjugates were excited at 485 nm and their emission spectra were recorded. The maximum of fluorescence intensity had a wavelength $\lambda_{mas}$ that varied slightly between conjugates, from 529.5 to 540 nm (Table 1). The following experiments of spectrofluorometry were performed at the $\lambda_{max}$ value thus determined for each conjugate.

The responsiveness of the H4S conjugates to the binding of their antigen was tested by measuring the relative variation $\Delta F/F_0 = (F - F_0)/F_0$ in their fluorescence intensity $F$ between their HEL-bound and free states. The total concentration of conjugate $[B]_0$ was chosen to fulfill two requirements. (i) The fluorescence intensity $F_0$ of the free conjugate had to be within the dynamic interval of the spectrofluorometer. (ii) The dynamic interval of $F$ had to cover more than one order of magnitude in antigen concentration. In these experiments, a concentration that was identical for all the conjugates and equal to 0.3 $\mu$M was chosen. The values of $F_0$ for the various conjugates were comprised between 37 and 1921 FU (fluorescence units) at this concentration and corresponded to molar fluorescences $f_b$ comprised between 191 and 4375 FU $\mu M^{-1}$ (Table 1).

The theoretical Equation 5, linking $\Delta F/F_0$ and the total concentration of antigen [A]o, was fitted to the experimental data with the maximal variation $\Delta F_\infty/F_0$, the dissociation constant $K_d$ and the total concentration of conjugate $[B]_0$ as floating parameters (Materials and Methods). The titrations of the conjugates were performed with ≥ 17 concentrations of their antigen, HEL (Figure 1). The corresponding values of $\Delta F_\infty/F_0$ and $K_d$ are given in Table 1. The values of $K_d$ varied widely between conjugates, between 7 nM and 8 $\mu$M, with five values being close to the value for H4S(wt) (see legend to Table 1). The value of $\Delta F_\infty/F_0$ was > 0.5 for eleven of the conjugates, i. e. their fluorescence intensity $F$ increased by > 1.5 fold on HEL binding.

### 2.4 Classification of the conjugates

The conjugates of H4S gave a wide range of values for $\Delta F_\infty/F_0$ and $K_d$. They were classified according to their sensitivity, a parameter which is used to characterize any measuring instrument. This sensitivity can take two forms for a RF biosensor, a relative sensitivity $s_r$ and an absolute sensitivity $s$. The relative sensitivity $s_r$ relates the relative variation $\Delta F/F_0$ of the fluorescence signal to the relative concentration $[A]_0/[B]_0$ of antigen for the low values of the latter, where [A]o and $[B]_0$ are the total concentrations of antigen and conjugate, respectively, in the titration reaction (Equation 7 in Materials and Methods; $[A]_0/[B]_0$ can be viewed as the concentration of antigen, normalized to the concentration of conjugate). $s_r$ is an intrinsic dimensionless parameter. Its value does not depend on the spectrofluorometer or its set up, and should remain constant between experiments, instruments and laboratories. The value of $s_r$ depends on the values of $[B]_0$ and $K_d$ according to a saturation law and its maximal value is equal to $\Delta F_\infty/F_0$ (Equation 8). The absolute sensitivity s relates $\Delta F$ and $[A]_0$ for the low values and is equal to $f_b s_r$, where $f_b$ is the molar fluorescence of the free conjugate (Equations 6 and 9). The $s^{-1}$ parameter relates the lower limit of detection $\delta[A]_0$ for the conjugate to the lower limit of measurement $\delta F$ for the spectrofluorometer.

The variations of $s_r$ (Figure 2) and $s^{-1}$ were calculated for each conjugate as functions of $[B]_0$ in buffer C from Equations 8 and 9. These variations showed that the classification of the conjugates according to their values of $s_r$ could vary as a function of $[B]_0$. For $s_r$ and with $[B]_0$ = 0.3 $\mu$M, i. e. the concentration at which the experiments were performed, the coupling positions ranked in the following decreasing order, i. e. starting with the highest relative sensitivity: Lys24 > Ala26 > Val21 > Lys39 > Trp8 > Tyr42 (Table 1). For $s^{-1}$ and $[B]_0$ = 0.3 $\mu$M, the coupling positions ranked in the following increasing order, starting with the lowest limit of detection: Ala26 < Lys24 < Val21 < Lys39 < Ser29 < Thr46. The conjugate at position Lys24, H4S(Lys24ANBD), had a value $s_r$ = 6.6 when used at a concentration $[B]_0$ = 0.3 $\mu$M, and therefore a lower limit of detection $\delta[A]_0$ = 0.35 nM since the Jasco FP-6300 spectrofluorometer which was used could detect a relative variation of fluorescence $\delta F/F_0$ = 1.5 % in these experimental conditions (Equation 10). The ranking of the conjugates according to $s^{-1}$ was valid because all our measurements were done with the same spectrofluorometer and set-up of the instrument.

### 2.5 Specificity and selectivity

To test the specificity of the recognition between the H4S conjugates and HEL, H4S(K24ANBD) and H4S(A26ANBD) were titrated with bovine serum albumin (BSA) in buffer C. Both conjugates bound BSA but with values of $K_d$ much higher and values of $\Delta F_\infty/F_0$ much lower than for HEL: $K_d = 1.0 \pm 0.3$ µM and $\Delta F_\infty/F_0 = 0.26 \pm 0.01$ for H4S (K24ANBD); $K_d = 1.4 \pm 0.3$ µM and $\Delta F_\infty/F_0 = 0.73 \pm 0.04$ for H4S(A26ANBD) (Figure 1). As a result, the relative sensitivities $s_r$ of the two conjugates were 106 and 36 fold lower for BSA than for HEL, respectively, at the concentration of conjugate $[B]_0 = 0.3$ µM used for these experiments. Therefore, the variation of the $\Delta F/F_0$ signal was indeed specific for HEL, the cognate antigen.

The selectivity of a biosensor refers to the extent to which it can recognize a particular analyte in a complex mixture without interference from other components (Vessman *et al.,* 2001). The selectivity of the H4S(K24ANBD) conjugate was characterized by comparing its fluorescence properties in serum and in buffer C. More specifically, the variations of the $F_0$ and $F_{10\mu M}$ parameters were measured as functions of the concentration in serum (Figure 3). Given that $K_d = 0.1$ µM for H4S(K24ANBD), this conjugate was fully saturated with HEL at 10 µM. We observed that the value of $F_{10\mu M}$ for H4S(K24ANBD) decreased linearly with the concentration in serum. As expected, the absorbance of the serum alone increased linearly with its concentration, in agreement with the Beer-Lambert law, at both 485 nm and 536 nm, which were the wavelengths of fluorescence excitation and emission in our experiments. Therefore, the absorption of the excitation and emission lights by serum could account for the variation of $F_{10\mu M}$. Surprisingly, $F_0$ increased with the concentration in serum, up to 40 % (v/v) of serum, and then decreased. The same increase and decrease was observed when measuring the fluorescence of a conjugate between IANBD and 2-mercaptoethanol. Thus, the increase resulted from the interaction between the fluorescent group and molecules of the serum, and the decrease from the absorbance of the serum, as observed for $F_{10\mu M}$. The variation of $(F_{10\mu M} - F_0)/F_0$ with the concentration of serum led to the conclusion that H4S(K24ANBD) could still operate in ≤ 50 % serum ($(F_{10\mu M} - F_0)/F_0 \geq 0.25$).

### 2.6 Binding parameters

The dissociation constants $K_d$ in solution between the H4S conjugates and HEL were determined by fluorescence in titration experiments (Table 1). This method was not applicable to the parental Nanofitin H4S(wt). Therefore, the value of its $K_d$ was determined in solution by experiments of competition Biacore. It was equal to $40.3 \pm 1.6$ nM (legend to Table 1). Comparison of the $K_d$ values for the conjugates and H4S(wt), all of them determined in solution, showed that conjugation of the fluorophore at positions Trp22, Leu31, Ile33, Asn40, Tyr42 and Asp44 strongly decreased the free energy of interaction between H4S and HEL ($\Delta\Delta G \geq 1.5$ kcal mol$^{-1}$, Set 1). Conjugation at positions Trp8, Val21, Lys24, Ala26 and Lys39 affected it more mildly ($0.5 \leq \Delta\Delta G \leq 1.2$ kcal mol$^{-1}$, Set 2). Conjugation at positions Phe7, Asn9, Lys28, Ser29 and Thr46 did not affect adversely the interaction ($\Delta\Delta G \leq 0.5$ kcal mol$^{-1}$, Set 3). Note that the conjugation at position Asn9 improved the interaction. These experiments suggested that the residues of Set 1, which form a continuous patch at the surface of H4S, belong to the binding site for HEL; that the residues of Set 2 are at the periphery of the binding site; and that those of Set 3 are located outside of it (Figure 4). The positions that gave the most sensitive conjugates belonged to Set 2 (Table 1).

To determine whether the variations of affinity were due to the mutation of the parental side chain into Cys or to the grafting of the fluorescent group, the kinetic parameters of interaction were measured between H4S(wt) and five of its cysteine mutants on the one hand, and HEL on the other hand by Biacore (Materials and Methods). The kinetics were measured in reducing conditions to prevent the dimerization of the cysteine mutants. The kinetic data were analyzed with a 1:1 model, the corresponding dissociation constant were calculated from the rate constants, i. e. $K_d' = k_{off}/k_{on}$, and the dissociation constant at steady state $K_d''$ were also derived from these kinetic experiments (Table 2). The values of $K_d$, $K_d'$ and $K_d''$ were found close for H4S(wt), $40 \pm 2$ nM, 31 nM and 18 nM respectively. The value determined in solution was slightly higher than the values determined at the interface between a solid and a liquid phase. Comparison of the $K_d$, $K_d'$ and $K_d''$ values between H4S(wt) and either the Cys mutants or the conjugates indicated that the lower affinity of the conjugates relative to H4S(wt) was mainly due to the mutation into Cys at positions Val21 and Lys24, whereas it was mainly due to the grafting of the fluorophore at positions Trp8, Ala26 and Lys39.

**Table 2.**

| Mutation | Monomer (%) | $k_{on}$ ($10^5$ M$^{-1}$s$^{-1}$) | $k_{off}$ ($10^{-2}$ s$^{-1}$) | $K_d'$ (nM) | $K_d''$ (nM) |
|---|---|---|---|---|---|
| WT | 100 | 4.9 | 1.5 | 31 | 18 |
| W8C | 86 | 4.5 | 3.0 | 68 | 66 |
| V21C | 88 | 0.4 | 1.1 | 332 | 389 |
| K24C | 100 | 1.6 | 2.3 | 117 | 104 |
| A26C | 80 | 3.8 | 2.3 | 60 | 54 |
| K39C | 85 | 3.1 | 1.2 | 38 | 24 |

Table 2 shows binding parameters of H4S derivatives, as determined by Biacore experiments. The percentage of monomers was quantified with the Un-scan-it software, as described in Materials and Methods. HEL was immobilized on a CM5 sensorchip. The association and dissociation rate constants, $k_{on}$ and $k_{off}$, were determined at 25 °C in buffer E and used to calculate $K_d' = k_{off}/k_{on}$ (Materials and Methods). $K_d''$ is the dissociation constant at steady state. A simple kinetic model of Langmuir binding was applied.

### 2.7 Mechanism of fluorescence variation

Eleven of the conjugates were sensitive to the binding of HEL, with $\Delta F_{\infty}/F_0$ between 0.5 and 8.9. Potassium iodide (KI) was used to explore the physicochemical mechanism by which the fluorescence intensity of the conjugates varied on antigen binding. KI, up to 250 mM, does not affect the interaction between proteins (Brient-Litzler *et al.,* 2010). The fluorescence of the H4S(K24ANBD) conjugate was quenched by KI, both in its free and HEL-bound states. The quenching varied linearly with the concentration of KI (Figure 5). This law of variation indicated that the molecules of fluorophore were identically exposed to KI and constituted a homogeneous population in either case (Lakowicz, 1999). It confirmed that the fluorescent group was specifically coupled to the mutant cysteine. The Stern-Volmer constant was higher for the free conjugate than for its complex with the target antigen: $K_{sv} = 6.7 \pm 0.1$ $M^{-1}$ versus $2.5 \pm 0.1$ $M^{-1}$ (Figure 5). These values indicated a lower accessibility of the fluorophore to KI in the bound state of the conjugate than in its free state. They showed that the fluorescence increase was due to a shielding of the fluorescent group from the solvent by the binding of the antigen, as previously observed for other conjugates with IANBD (Brient-Litzler *et al.,* 2010; Renard *et al.,* 2003).

### 2.8 Conclusion

The inventors have constructed reagentless fluorescent biosensors from Nanofitins in the absence of specific structural data, by using their peculiar method of construction. The method comprises: (i) individually changing the residues of the hypervariable positions into cysteine at the genetic level, (ii) chemically coupling a solvatochromic fluorophore with the mutant cysteine, and (iii) ordering the resulting conjugates through their relative sensitivity $s_r$, that involves both their affinity for the antigen and their relative variation of fluorescence signal. This method could be applied to any antigen binding protein in the same conditions. The inventors have validated the method by constructing sixteen conjugates between the IANBD fluorophore and H4S, a Nanofitin which is directed against hen egg-white lysozyme (HEL) and for which no specific structural data is available.

The residues at the 14 fully randomized positions and at positions Lys28 and Lays39 of H4S were changed individually into cysteine by site-directed mutagenesis of the coding gene. The sixteen cysteine mutants were then conjugated to the thiol reactive fluorophore IANBD ester, and the fluorescence and binding properties of the conjugates were analyzed.

Five conjugates had values of $K_d$ close to that of H4S(wt). The value of $\Delta F_{\infty}/F_0$ was > 0.5 for eleven of the conjugates, i. e. their fluorescence intensity *F* increased by > 1.5 fold on HEL binding. The inventors ranked the conjugates according to their relative sensitivity $s_r$ and their lower limit of detection (proportional to $s^{-1}$). The positions that gave the most sensitive conjugates were at the periphery of the binding site ($0.5 \leq \Delta\Delta G \leq 1.2$ kcal $mol^{-1}$). Comparison of the $K_d$, $K_d'$ and $K_d''$ values between H4S(wt) and either the Cys mutants or the conjugates indicated that the lower affinity of the conjugates relative to H4S(wt) was mainly due to the mutation into Cys at positions Val21 and Lys24, whereas it was mainly due to the grafting of the fluorophore at positions Trp8, Ala26 and Lys39. One of the conjugate, H4S (Lys24ANBD), had a value $s_r = 6.6$ when used at a concentration $[B]_0 = 0.3$ μM, and therefore a lower limit of detection $\delta[A]_0 = 0.35$ nM. This biosensor had an affinity only two-fold lower than the affinity of the parental protein. H4S (Lys24ANBD) bound its cognate antigen specifically and selectively and could still operate in ≤ 50 % serum. Its fluorescence signal increased linearly and specifically with the concentration of antigen. Titration with potassium iodide indicated that the fluorescence variation was due to a shielding of the fluorescent group from the solvent by the antigen.

These new results on Nanofitins and previous results on antibodies and DARPins show that it is possible to construct reagentless fluorescence biosensors from any natural or artificial family of antigen binding protein by targeting in priority the hypervariable positions that are not important for the interaction with the antigen.

The sensitivities of the Nanofitin conjugates were much higher than those for scFv fragments of antibodies and generally higher than those for DARPins.

Therefore, the Nanofitins which have a higher stability than antibodies and may have an extended range of antigens compared to DARPins constitute a promising family of target binding proteins, for the construction and the multiple applications of reagentless fluorescent biosensors directed to various targets.

**Table 3.**

| SEQ ID NO: | Protein Data Bank (PDB) accession number | Protein name Organism | UniProt/ GenBank accession number | SEQUENCE |
|---|---|---|---|---|
| 12 | 1krs | Lysyl-tRNA synthetase LysS (*Escherichia coli*) | B7UHT9 | MSEQHAQGADAVVDLNNELKTRREKLANLREQGIAFPNDFRRDHTSDQLHAEFDGKENEELEALNIEVAVAG<br>RMMTRRIMGKASFVTLQDVGGRIQLYVARDDLPEGVYNEQFKKWDLGDILGAKGKLFKTKTGELSIHCTELR<br>LLTKALRPLPDKFHGLQDQEARYRQRYLDLISNDESRNTFKVRSQILSGIRQFMVNRGFMEVETPMMQVIPG<br>GAAARPFITHHNALDLDMYLRIAPELYLKRLVVGGFERVFEINRNFRNEGISVRHNPEFTMMELYMAYADYK<br>DLIELTESLFRTLAQDILGKTEVTYGDVTLDFGKPFEKLTMREAIKKYRSETDMADLDNFDSAKAIAESIGI<br>HVEKSWGLGRIVTEIFEEVAEAHLIQPTFITEYPAEVSPLARRNDVNPEITDRFEFFIGGREIGNGFSELND<br>AEDQAQRFLDQVAAKDAGDDEAMFYDEDYVTALEHGLPPTAGLGIGIDRMVMLFTNSHTIRDVILFPAMRPV<br>K |
| 13 | 1c0aA | Asp-tRNA synthetase (*Escherichia coli*) | P21889 | MRTEYCGQLRLSHVGQQVTLCGWVNRRRDLGSLIFIDMRDREGIVQVFFDPDRADALKLASELRNEFCIQVT<br>GTVRARDEKNINRDMATGEIEVLASSLTIINRADVLPLDSNHVNTEEARLKYRYLDLRRPEMAQRLKTRAKI<br>TSLVRRFMDDHGFLDIETPMLTKATPEGARDYLVPSRVHKGKFYALPQSPQLFKQLLMMSGFDRYYQIVKCF<br>RDEDLRADRQPEFTQIDVETSFMTAPQVREVMEALVRHLWLEVKGVDLGDFPVMTFAEAERRYGSDKPDLRN<br>PMELTDVADLLKSVEFAVFAGPANDPKGRVAALRVPGGASLTRKQIDEYGNFVKIYGAKGLAYIKVNERAKG<br>LEGINSPVAKFLNAEIIEDILDRTAAQDGDMIFFGADNKKIVADAMGALRLKVGKDLGLTDESKWAPLWVID<br>FPMFEDDGEGGLTAMHHPFTSPKDMTAAELKAAPENAVANAYDMVINGYEVGGGSVRIHNGDMQQTVFGILG<br>INEEEQREKFGFLLDALKYGTPPHAGLAFGLDRLTMLLTGTDNIRDVIAFPKTTAAACLMTEAPSFANPTAL<br>AELSIQVVK |
| 14 | 1b8aA | Asp-tRNA synthetase (*Thermococcus kodakaraensis*) | Q52428 | MYRTHYSSEITEELNGQKVKVAGWVWEVKDLGGIKFLWIRDRDGIVQITAPKKKVDPELFKLIPKLRSEDVV<br>AVEGVVNFTPKAKLGFEILPEKIVVLNRAETPLPLDPTGKVKAELDTRLDNRFMDLRRPEVMAIFKIRSSVF<br>KAVRDFFHENGFIEIHTPKIIATATEGGTELFPMKYFEEDAFLAQSPQLYKQIMMASGLDRVYEIAPIFRAE<br>EHNTTRHLNEAWSIDSEMAFIEDEEEVMSFLERLVAHAINYVREHNAKELDILNFELEEPKLPFPRVSYDKA<br>LEILGDLGKEIPWGEDIDTEGERLLGKYMMENENAPLYFLYQYPSEAKPFYIMKYDNKPEICRAFDLEYRGV<br>EISSGGQREHRHDILVEQIKEKGLNPESFEFYLKAFRYGMPPHGGFGLGAERLIKQMLDLPNIREVILFPRD<br>RRRLTP |
| 15 | 11ylA | Lysyl-tRNA synthetase LysU (*Escherichia coli*) | P0A8N6 | SEQETRGANEAIDFNDELRNRREKLAALRQQGVAFPNDFRRDHTSDQLHEEFDAKDNQELESLNIEVSVAGR<br>MMTRRIMGKASFVTLQDVGGRIQLYVARDSLPEGVYNDQFKKWDLGDIIGARGTLFKTQTGELSIHCTELRL<br>LTKALRPLPDKFHGLQDQEVRYRQRYLDLIANDKSRQTFVVRSKILAAIRQFMVARGFMEVETPMMQVIPGG<br>ASARPFITHHNALDLDMYLRIAPELYLKRLVVGGFERVFEINRNFRNEGISVRHNPEFTMMELYMAYADYHD<br>LIELTESLFRTLAQEVLGTTKVTYGEHVFDFGKPFEKLTMREAIKKYRPETDMADLDNFDAAKALAESIGIT<br>VEKSWGLGRIVTEIFDEVAEAHLIQPTFITEYPAEVSPLARRNDVNPEITDRFEFFIGGREIGNGFSELNDA<br>EDQAERFQEQVNAKAAGDDEAMFYDEDYVTALEYGLPPTAGLGIGIDRMIMLFTNSHTIRDVILFPAMRPQK |

(continued)

| SEQ ID NO: | Protein Data Bank (PDB) accession number | Protein name Organism | UniProt/ GenBank accession number | SEQUENCE |
|---|---|---|---|---|
| 16 | 1quqA | Replication protein A, 32 kDa subunit (*Homo sapiens*) | P15927 | MWNSGFESYGSSSYGGAGGYTQSPGGFGSPAPSQAEKKSRARAQHIVPCTISQLLSATLVDEVFRIGNVEIS<br>QVTIVGIIRHAEKAPTNIVYKIDDMTAAPMDVRQWVDTDDTSSENTVVPPETYVKVAGHLRSFQNKKSLVAF<br>KIMPLEDMNEFTTHILEVINAHMVLSKANSQPSAGRAPISNPGMSEAGNFGGNSFMPANGLTVAQNQVLNLI<br>KACPRPEGLNFQDLKNQLKHMSVSSIKQAVDFLSNEGHIYSTVDDDHFKSTDAE |
| 17 | 1quqB | Replication protein A, 14 kDa subunit (*Homo sapiens*) | P35244 | MVDMMDLPRSRINAGMLAQFIDKPVCFVGRLEKIHPTGKMFILSDGEGKNGTIELMEPLDEEISGIVEVVGR<br>VTAKATILCTSYVQFKEDSHPFDLGLYNEAVKIIHDFPQFYPLGIVQHD |
| 18 | 1jmcA | Replication protein A, 70 kDa subunit (RPA70) fragment (*Homo sapiens*) | P27694 | MVGQLSEGAIAAIMQKGDTNIKPILQVINIRPITTGNSPPRYRLLMSDGLNTLSSFMLATQLNPLVEEEQLS<br>SNCVCQIHRFIVNTLKDGRRVVILMELEVLKSAEAVGVKIGNPVPYNEGLGQPQVAPPAPAASPAASSRPQP<br>QNGSSGMGSTVSKAYGASKTFGKAAGPSLSHT**SGGT**QSKVVPIASLTPYQSKWTICARVTNKSQIRTWSNSR<br>GEGKLFSLELVDESGEIRATAFNEQVDKFFPLIEVNKVYYFSKGTLKIANKQFTAVKNDYEMTFNNETSVMP<br>CEDDHHLPTVQFDFTGIDDLENKSKDSLVDIIGICKSYEDATKITVRSNNREVAKRNIYLMDTSGKVVTATL<br>WGEDADKFDGSRQPVLAIKGARVSDFGGRSLSVLSSSTIIANPDIPEAYKLRGWFDAEGQALDGVSISDLKS<br>GGVGGSNTNWKTLYEVKSENLGQGDKPDYFSSVATVVYLRKENCMYQACPTQDCNKKVIDQQNGLYRCEKCD<br>TEFPNFKYRMILSVNIADFQENQWVTCFQESAEAILGQNAAYLGELKDKNEQAFEEVFQNANFRSFIFRVRV<br>KVETYNDESRIKATVMDVKPVDYREYGRRLVMSIRRSALM |
| 19 | 1otcA | Telomere-end-binding protein (*Oxytricha nova*) | P29549 | MSTAAKQNRSTSRVSKKKTAAPKEGAAKKSDKGHKYEYVELAKASLTSAQPQHFYAVVIDATFPYKTNQERY<br>ICSLKIVDPTLYLKQQKGAGDASDYATLVLYAKRFEDLPIIHRAGDIIRVHRATLRLYNGQRQFNANVFYSS<br>SWALFSTDKRSVTQEINNQDAVSDTTPFSFSSKHATIEKNEISILQNLRKWANQYFSSYSVISSDMYTALNK<br>AQAQKGDFDVVAKILQVHELDEYTNELKLKDASGQVFYTLSLKLKFPHVRTGEVVRIRSATYDETSTQKKVL<br>ILSHYSNIITFIQSSKLAKELRAKIQDDHSVEVASLKKNVSLNAVVLTEVDKKHAALPSTSLQDLFHHADSD<br>KELQAQDTFRTQFYVTKIEPSDVKEWVKGYDRKTKKSSSLKGASGKGDNIFQVQFLVKDASTQLNNNTYRVL<br>LYTQDGLGANFFNVKADNLHKNADARKKLEDSAELLTKFNSYVDAVVERRNGFYLIKDTKLIY |
| 21 | 3ullA | Mitochondrial ssDNA-binding protein (*Homo sapiens*) | Q567R6 | MFRRPVLQVLRQFVRHESETTTSLVLERSLNRVHLLGRVGQDPVLRQVEGKNPVTIFSLATNEMWRSGDSEV<br>YQLGDVSQKTTWHRISVFRPGLRDVAYQYVKKRSRIYLEGKIDYGEYMDKNNVRRQATTIIADNIIFLSDQT<br>KEKE |
| 22 | 1prtF | Pertussis toxin S5 subunit (*Bordetella pertussis*) | P04981 | MQRQAGLPLKANPMHTIASILLSVLGIYSPADVAGLPTHLYKNFTVQELALKLKGKNQEFCLTAFMSGRSLV<br>RACLSDAGHEHDTWFDTMLGFAISAYALKSRIALTVEDSPYPGTPGDLLELQICPLNGYCE |

(continued)

| SEQ ID NO: | Protein Data Bank (PDB) accession number | Protein name Organism | UniProt/ GenBank accession number | SEQUENCE |
|---|---|---|---|---|
| 23 | 1bcpD | Pertussis toxin S5 subunit (ATP bound; *Bordetella. pertussis*) | P0A3R5 | MLRRFPTRTTAPGQGGARRSRVRALAWLLASGAMTHLSPALADVPYVLVKTNMVVTSVAM<br>KPYEVTPTRMLVCGIAAKLGAAASSPDAHVPFCFGKDLKRPGSSPMEVMLRAVFMQQRPL<br>RMFLGPKQLTFEGKPALELIRMVECSGKQDCP |
| 24 | 3chbD | Cholera Toxin (*Vibrio cholerae*) | D0UTQ9 | MTPQNITDLCAEYHNTQIHTLNDKIFSYTESLAGKREMAIITFKNGATFQVEVPGSQHIDSQKKAIERMKDT<br>LRIAYLTE |
| 25 | 1tiiD | Heat-labile toxin (*Escherichia coli*) | P43529 | MSFKKIIKAFVIMAALVSVQAHAGASQFFKDNCNRTTASLVEGVELTKYISDINNNTDGM<br>YVVSSTGGVWRISRAKDYPDNVMTAEMRKIAMAAVLSGMRVNMCASPASSPNVIWAIELE<br>AE |
| 26 | 2bosA | Verotoxin/Shiga toxin, B-pentamer (*Escherichia coli*) | Q9MBZ7 | MKKMFIAVLFALVSVNAMAADCAKGKIEFSKYNEDNTFTVKVSGREYWTNRWNLQPLLQSAQLTGMTVTIIS<br>NTCSSGSGFAQVKFN |
| 27 | 1br9 | TIMP-2 (*Homo sapiens*) | P16035 | MGAAARTLRLALGLLLLATLLRPADACSCSPVHPQQAFCNADVVIRAKAVSEKEVDSGNDIYGNPIKRIQYE<br>IKQIKMFKGPEKDIEFIYTAPSSAVCGVSLDVGGKKEYLIAGKAEGDGKMHITLCDFIVPWDTLSTTQKKSL<br>NHRYQMGCECKITRCPMIPCYISSPDECLWMDWVTEKNINGHQAKFFACIKRSDGSCAWYRGAAPPKQEFLD<br>IEDP |
| 28 | 1an8 | Superantigen SPE-C (*Streptococcus pyogenes*) | Q8NKX2 | MKKINIIKIVFIITVILISTISPIIKSDSKKDISNVKSDLLYAYTITPYDYKDCRVNFSTTHTLNIDTQKYR<br>GKDYYISSEMSYEASQKFKRDDHVDVFGLFYILNSHTGEYIYGGITPAQNNKVNHKLLGNLFISGESQQNLN<br>NKIILEKDIVTFQEIDFKIRKYLMDNYKIYDATSPYVSGRIEIGTKDGKHEQIDLFDSPNEGTRSDIFAKYK<br>DNRIINMKNFSHFDIYLEK |
| 29 | 3seb | Superantigen SPE (*Staphylococcus aureus*) | Q5MAA8 | ILVISTPNVLAESQPDPKPDELHKSSKFTGLMENMKVLYDDNHVSAINVKSIDQFLYFDLIYSIKDTKLGNY<br>DNVRVEFKNKDLADKYKDKYVDVFGANYYYQCYFSKKTNDINSHQTDKRKTCMYGGVTEHNGNQLDKYRSIT<br>VRVFEDGKNLLSFDVQTNKKKVTAQELDYLTRHYLVKNKKLYEFNNSPYETGYIKFIENENSFWYDMMPAPG<br>DKFDQSKYLMMYNDNKMVDSKDVKIEVYLTTKKK |
| 30 | law7A | Toxic shock syndrome toxin (*Staphylococcus aureus*) | AOFIN2 | MNKKLLMIFFIVSPLLLATIATDFTPVPLLSNQIIKTAKASTNDNIKDLLDWYSSGSDTFTNSEVLDNSLGS<br>MRIKNTDGSISLIIFPSPYYSPAFTKGEKVDLNTKRTKKSQHTSEGTYIHFQISGVTNTEKLPTPIELPLKV<br>KVHGKDSPLKYWPKFDKKQLAISTLDFEIRHQLTQIHGLYRSSDKTGGYWKITMNDGSTYQSDLSKKFEYNT<br>EKPPINIDEIKTIEAEIN |

(continued)

| SEQ ID NO: | Protein Data Bank (PDB) accession number | Protein name Organism | UniProt/ GenBank accession number | SEQUENCE |
|---|---|---|---|---|
| 31 | 1mjc | Major cold-shock protein, (*Escherichia coli*) | P0A9Y1 | MSGKMTGIVKWFNADKGFGFITPDDGSKDVFVHFSAIQNDGYKSLDEGQKVSFTIESGAKGPAAGNVTSL |
| 32 | 1bkb | Initiation translation factor a (*Pyrobaculum aerophylum*) | P56635 | MVLKWVMSTKYVEAGELKEGSYVVIDGEPCRVVEIEKSKTGKHGSAKARIVAVGVFDGGKRTLSLPVDAQVE<br>VPIIEKFTAQILSVSGDVIQLMDMRDYKTIEVPMKYVEEEAKGRLAPGAEVEVWQILDRYKIIRVK |
| 33 | 1sro | S1 RNA-binding domain of PNPase (*Escherichia coli*) | P05055 | MLNPIVRKFQYGQHTVTLETGMMARQATAAVMVSMDDTAVFVTVVGQKKAKPGQDFFPLTVNYQERTYAAGR<br>IPGSFFRREGRPSEGETLIARLIDRPIRPLFPEGFVNEVQVIATVVSVNPQVNPDIVAMIGASAALSLSGIP<br>FNGPIGAARVGYINDQYVLNPTQDELKESKLDLVVAGTEAAVLMVESEAQLLSEDQMLGAVVFGHEQQQVVI<br>QNINELVKEAGKPRWDWQPEPVNEALNARVAALAEARLSDAYRITDKQERYAQVDVIKSETIATLLAEDETL<br>DENELGEILHAIEKNVVRSRVLAGEPRIDGREKDMIRGLDVRTGVLPRTHGSALFTRGETQALVTATLGTAR<br>DAQVLDELMGERTDTFLFHYNFPPYSVGETGMVGSPKRREIGHGRLAKRGVLAVMPDMDKFPYTVRVVSEIT<br>ESNGSSSMASVCGASLALMDAGVPIKAAVAGIAMGLVKEGDNYVVLSDILGDEDHLGDMDFKVAGSRDGISA<br>LQMDIKIEGITKEIMQVALNQAKGARLHILGVMEQAINAPRGDISEFAPRIHTIKINPDKIKDVIGKGGSVI<br>RALTEETGTTIEIEDDGTVKIAATDGEKAKHAIRRIEEITAEIEVGRVYTGKVTRIVDFGAFVAIGGGKEGL<br>VHISQIADKRVEKVTDYLQMGQEVPVKVLEVDRQGRIRLSIKEATEQSQPAAAPEAPAAEQGE |
| 34 | 1d7qA | Initiation translation factor elfa (*Homo sapiens*) | P47813 | MPKNKGKGGKNRRRGKNENESEKRELVFKEDGQEYAQVIKMLGNGRLEAMCFDGVKRLCHIRGKLRKKVWIN<br>TSDIILVGLRDYQDNKADVILKYNADEARSLKAYGELPEHAKINETDTFGPGDDDEIQFDDIGDDDEDIDDI |
| 35 | 1ah9 | Initiation translation factor IF (*Escherichia coli*) | P69224 | MAKFDNIEMQGTVLETLPNTMFRVELENGHVVTAHISGKMRKNYIRILTGDKVTVELTPYDLSKGRIVFRSR |
| 36 | 1ckmA | RNA guanylyltransferase (Chlorella virus, PBCV-1) | Q84424 | MVPPTINTGKNITTERAVLTLNGLQIKLHKVVGESRDDIVAKMKDLAMDDHKFPRLPGPNPVSIERKDFEKL<br>KQNKYVVSEKTDGIRFMMFFTRVFGFKVCTIIDRAMTVYLLPFKNIPRVLFQGSIFDGELCVDIVEKKFAFV<br>LFDAVVVSGVTVSQMDLASRFFAMKRSLKEFKNVPEDPAILRYKEWIPLEHPTIIKDHLKKANAIYHTDGLI<br>IMSVDEPVIYGRNFNLFKLKPGTHHTIDFIIMSEDGTIGIFDPNLRKNVPVGKLDGYYNKGSIVECGFADGT<br>WKYIQGRSDKNQANDRLTYEKTLLNIEENITIDELLDLFKWE |

(continued)

| SEQ ID NO: | Protein Data Bank (PDB) accession number | Protein name Organism | UniProt/ GenBank accession number | SEQUENCE |
|---|---|---|---|---|
| 37 | 1a0i | ATP-dependent DNA ligase, (Bacteriophage T7) | P00969 | MMNIKTNPFKAVSFVESAIKKALDNAGYLIAEIKYDGVRGNICVDNTANSYWLSRVSKTIPALEHLNGFDVR<br>WKRLLNDDRCFYKDGFMLDGELMVKGVDFNTGSGLLRTKWTDTKNQEFHEELFVEPIRKKDKVPFKLHTGHL<br>HIKLYAILPLHIVESGEDCDVMTLLMQEHVKNMLPLLQEYFPEIEWQAAESYEVYDMVELQQLYEQKRAEGH<br>EGLIVKDPMCIYKRGKKSGWWKMKPENEADGIIQGLVWGTKGLANEGKVIGFEVLLESGRLVNATNISRALM<br>DEFTETVKEATLSQWGFFSPYGIGDNDACTINPYDGWACQISYMEETPDGSLRHPSFVMFRGTEDNPQEKM |
| 38 | 1snc | Staphylococcal nuclease, (*Staphylococcus aureus*) | gi\|224650 | RLILKLTKKKEVLVMTEYLLSAGICMAIVSILLIGMAISNVSKGQYAKRFFFFATSCLVLTLVVVSSLSS<br>SANASQTDNGVNRSGSEDPTVYSATSTKKLHKEPATLIKAIDGDTVKLMYKGQPMTFRLLLVDTPETKHP<br>KKGVEKYGPEASAFTKKMVENAKKIEVEFDKGQRTDKYGRGLAYIYADGKMVNEALVRQGLAKVAYVYKP<br>NNTHEQHLRKSEAQAKKEKLNIWSEDNADSGQ |
| 39 | 1hjp | DNA helicase RuvA subunit N-terminal domain (*Escherichia coli*) | P0A811 | MIGRLRGIIIEKQPPLVLIEVGGVGYEVHMPMTCFYELPEAGQEAIVFTHFVVREDAQLLYGFNNKQERTLF<br>KELIKTNGVGPKLALAILSGMSAQQFVNAVEREEVGALVKLPGIGKKTAERLIVEMKDRFKGLHGDLFTPAA<br>DLVLTSPASPATDDAEQEAVAALVALGYKPQEASRMVSKIARPDASSETLIREALRAAL |
| 20 | 1otcB | Telomere-end binding protein (*Oxytricha nova*) | P16458 | MSKGASAPQQQSAFKQLYTELFNNEGDFSKVSSNLKKPLKCYVKESYPHFLVTDGYFFVAPYFTKEAVNEFH<br>AKFPNVNIVDLTDKVIVINNWSLELRRVNSAEVFTSYANLEARLIVHSFKPNLQERLNPTRYPVNLFRDDEF<br>KTTIQHFRHTALQAAINKTVKGDNLVDISKVADAAGKKGKVDAGIVKASASKGDEFSDFSFKEGNTATLKIA<br>DIFVQEKGKDALNKAADHTDGAKVKGGAKGKGKAAAKAAKGKKL |
| 40 | 1pfsA | Gene V protein (*Pseudomonas* bacteriophage pf3) | P03672 | MNIQITFTDSVRQGTSAKGNPYTFQEGFLHLEDKPFPLQCQFFVESVIPAGSYQVPYRINVNNGRPELAFDF<br>KAMKRA |
| 41 | 1gvp | Gene V protein (Filamentous bacteriophage f1, M13) | D0U157 | MIKVEIKPSQAQFTTRSGVSRQGKPYSLNEQLCYVDLGNEYPVLVKITLDEGQPAYAPGLYTVHLSSFKIGQ<br>FGSLMIDRLRLVPAK |
| 42 | 1gpc | Gene protein gp32 core (Bacteriophage T4) | B3IYU0 | MFKRKSTAELAAQMAKLNGNKGFSSEDKGEWKLKLDNAGNGQAVIRFLPSKNDEQAPFAILVNHGFKKNGKW<br>YIETCSSTHGDYDSCPVCQYISKNDLYNTDNKEYSLVKRKTSYWANILVVKDPAAPENEGKVFKYRFGKKIW<br>DKINAMIAVDVEMGETPVDVTCPWEGANFVLKVKQVSGFSNYDESKFLNQSAIPNIDDESFQKELFEQMVDL<br>SEMTSKDKFKSFEELNTKFGQVMGTAVMGGAAATAAKKADKVADDLDAFNVDDFNTKTEDDFMSSSSGSSSS<br>ADDT |

(continued)

| SEQ ID NO: | Protein Data Bank (PDB) accession number | Protein name Organism | UniProt/ GenBank accession number | SEQUENCE |
|---|---|---|---|---|
| 43 | 2prd | Inorganic pyrophosphatase (*Thermus thermophilus*) | Q72H95 | MANLKSLPVGDKAPEVVHMVIEVPRGSGNKYEYDPDLGAIKLDRVLPGAQFYPGDYGFIPSTLAEDGDPLDG LVLSTYPLLPGVVVEVRVVGLLLMEDEKGGDAKVIGVVAEDQRLDHIQDIGDVPEGVKQEIQHFFETYKALE AKKGKWVKVTGWRDRKAALEEVRACIARYKG |

Table 3 shows OB-fold protein sequences. The OB-fold domain sequence is underlined. Sequence differences between PDB and UniProt/GenBank data-bases are indicated in bold.

## REFERENCES

Agrawal, V., and Kishan, K. V. (2003). OB-fold: growing bigger with functional consistency. Curr Protein Pept Sci 4, 195-206.

Altschuh, D., Oncul, S., and Demchenko, A.P. 2006. Fluorescence sensing of intermolecular interactions and development of direct molecular biosensors. J Mol Recognit 19: 459-477.

Arcus, V. (2002). OB-fold domains: a snapshot of the evolution of sequence, structure and function. Curr Opin Struct Biol 12, 794-801.

Arnold, K., Bordoli, L., Kopp, J., and Schwede, T. (2006). The SWISS-MODEL workspace: a web-based environment for protein structure homology modelling. Bioinformatics 22, 195-201.

Binz, H.K., Stumpp, M.T., Forrer, P., Amstutz, P., and Pluckthun, A. 2003. Designing repeat proteins: well-expressed, soluble and stable proteins from combinatorial libraries of consensus ankyrin repeat proteins. J Mol Biol 332: 489-503.

Binz, H. K., Amstutz, P., Kohl, A., Stumpp, M. T., Briand, C., Forrer, P., Grutter, M. G., and Pluckthun, A. (2004). High-affinity binders selected from designed ankyrin repeat protein libraries. Nat Biotechnol 22, 575-582.

Brient-Litzler, E., Pluckthun, A., and Bedouelle, H. (2010). Knowledge-based design of reagentless fluorescent biosensors from a designed ankyrin repeat protein. Protein Eng Des Sel 23, 229-241.

Cinier, M., Petit, M., Williams, M. N., Fabre, R. M., Pecorari, F., Talham, D. R., Bujoli, B., and Tellier, C. (2009). Bisphosphonate adaptors for specific protein binding on zirconium phosphonate-based microarrays. Bioconjug Chem 20, 2270-2277.

Foote, J., and Winter, G. 1992. Antibody framework residues affecting the conformation of the hypervariable loops. J Mol Biol 224: 487-499.

Gasteiger, E., Gattiker, A., Hoogland, C., Ivanyi, I., Appel, R. D., and Bairoch, A. (2003). ExPASy: The proteomics server for in-depth protein knowledge and analysis. Nucleic Acids Res 31, 3784-3788.

Holm, L., and Park, J. (2000). DaliLite workbench for protein structure comparison. Bioinformatics 16, 566-567.

Holm, L., and Sander, C. (1998). Touring protein fold space with Dali/FSSP. Nucleic Acids Res 26, 316-319.

Jespers, L., Bonnert, T.P., and Winter, G. 2004. Selection of optical biosensors from chemisynthetic antibody libraries. Protein Eng Des Sel 17: 709-713.

Kitov, P. I., Sadowska, J. M., Mulvey, G., Armstrong, G. D., Ling, H., Pannu, N. S., Read, R. J., and Bundle, D. R. (2000). Shiga-like toxins are neutralized by tailored multivalent carbohydrate ligands. Nature 403, 669-672.

Kloks, C. P., Spronk, C. A., Lasonder, E., Hoffmann, A., Vuister, G. W., Grzesiek, S., and Hilbers, C. W. (2002). The solution structure and DNA-binding properties of the cold-shock domain of the human Y-box protein YB-1. J Mol Biol 316, 317-326.

Krehenbrink, M., Chami, M., Guilvout, I., Alzari, P. M., Pecorari, F., and Pugsley, A. P. (2008). Artificial binding proteins (Affitins) as probes for conformational changes in secretin PulD. J Mol Biol 383, 1058-1068.

Lakowicz, J. R. (1999). Principles of fluorescent spectroscopy, 2nd edn (New York: Kluwer Academic/Plenum).

Li, J., Mahajan, A., and Tsai, M. D. (2006). Ankyrin repeat: a unique motif mediating protein-protein interactions. Biochemistry 45, 15168-15178.

Lopez, R., Silventoinen, V., Robinson, S., Kibria, A., and Gish, W. (2003). WU-Blast2 server at the European Bioinformatics Institute. Nucleic Acids Res 31, 3795-3798.

Lowe, C.R. 1984. Biosensors. Trends Biotechnol 2: 59-65.

McAfee, J. G., Edmondson, S. P., Datta, P. K., Shriver, J. W., and Gupta, R. (1995). Gene cloning, expression, and characterization of the Sac7 proteins from the hyperthermophile Sulfolobus acidocaldarius. Biochemistry 34, 10063-10077.

Mitton-Fry, R. M., Anderson, E. M., Hughes, T. R., Lundblad, V., and Wuttke, D. S. (2002). Conserved structure for single-stranded telomeric DNA recognition. Science 296, 145-147.

Morgan, C.L., Newman, D.J., and Price, C.P. 1996. Immunosensors: technology and opportunities in laboratory medicine. Clin Chem 42: 193-209.

Mosavi, L. K., Cammett, T. J., Desrosiers, D. C., and Peng, Z. Y. (2004). The ankyrin repeat as molecular architecture for protein recognition. Protein Sci 13, 1435-1448.

Mouratou, B., Schaeffer, F., Guilvout, I., Tello-Manigne, D., Pugsley, A. P., Alzari, P. M., and Pecorari, F. (2007). Remodeling a DNA-binding protein as a specific in vivo inhibitor of bacterial secretin PulD. Proc Natl Acad Sci U S A 104, 17983-17988.

Murzin, A. G. (1993). OB(oligonucleotide/oligosaccharide binding)-fold: common structural and functional solution for non-homologous sequences. Embo J 12, 861-867.

Notredame, C., Higgins, D. G., and Heringa, J. (2000). T-Coffee: A novel method for fast and accurate multiple sequence alignment. J Mol Biol 302, 205-217.

Papageorgiou, A. C., Tranter, H. S., and Acharya, K. R. (1998). Crystal structure of microbial superantigen staphylococcal enterotoxin B at 1.5 A resolution: implications for superantigen recognition by MHC class II molecules and T-cell receptors. J Mol Biol 277, 61-79.

Pearl, F. M., Bennett, C. F., Bray, J. E., Harrison, A. P., Martin, N., Shepherd, A., Sillitoe, I., Thornton, J., and Orengo, C. A. (2003). The CATH database: an extended protein family resource for structural and functional genomics. Nucleic Acids Res 31, 452-455.

Qian, J., Stenger, B., Wilson, C. A., Lin, J., Jansen, R., Teichmann, S. A., Park, J., Krebs, W. G., Yu, H., Alexandrov, V., et al. (2001). PartsList: a web-based system for dynamically ranking protein folds based on disparate attributes, including whole-genome expression and interaction information. Nucleic Acids Res 29, 1750-1764.

Renard, M., and Bedouelle, H. (2004). Improving the sensitivity and dynamic range of reagentless fluorescent immunosensors by knowledge-based design. Biochemistry 43, 15453-15462.

Renard, M., Belkadi, L., and Bedouelle, H. (2003). Deriving topological constraints from functional data for the design of reagentless fluorescent immunosensors. J Mol Biol 326, 167-175.

Renard, M., Belkadi, L., Hugo, N., England, P., Altschuh, D., and Bedouelle, H. (2002). Knowledge-based design of reagentless fluorescent biosensors from recombinant antibodies. J Mol Biol 318, 429-442.

Robinson, H., Gao, Y. G., McCrary, B. S., Edmondson, S. P., Shriver, J. W., and Wang, A. H. (1998). The hyperthermophile chromosomal protein Sac7d sharply kinks DNA. Nature 392, 202-205.

Roussel, A., Anderson, B. F., Baker, H. M., Fraser, J. D., and Baker, E. N. (1997). Crystal structure of the streptococcal superantigen SPE-C: dimerization and zinc binding suggest a novel mode of interaction with MHC class II molecules. Nat Struct Biol 4, 635-643.

Samygina, V. R., Popov, A. N., Rodina, E. V., Vorobyeva, N. N., Lamzin, V. S., Polyakov, K. M., Kurilova, S. A., Nazarova, T. I., and Avaeva, S. M. (2001). The structures of Escherichia coli inorganic pyrophosphatase complexed with Ca(2+) or CaPP(i) at atomic resolution and their mechanistic implications. J Mol Biol 314, 633-645.

Smith, J.J., Conrad, D.W., Cuneo, M.J., and Hellinga, H.W. 2005. Orthogonal site-specific protein modification by engineering reversible thiol protection mechanisms. Protein Sci 14: 64-73.

Stumpp, M. T., Binz, H. K., and Amstutz, P. (2008). DARPins: A new generation of protein therapeutics Drug Discov. Today, 13, 695-701.

Theobald, D. L., Mitton-Fry, R. M., and Wuttke, D. S. (2003). Nucleic acid recognition by OB-fold proteins. Annu Rev Biophys Biomol Struct 32, 115-133.

Thevenot, D.R., Toth, K., Durst, R.A., and Wilson, G.S. 2001. Electrochemical biosensors: recommended definitions and classification. Biosens Bioelectron 16: 121-131.

## Claims

1. A reagentless biosensor for a target, comprising at least:

   an OB-fold protein;
   a cysteine residue coupled to a tluorophore.

2. The biosensor of claim 1, wherein the OB-fold protein is derived from a starting OB-fold protein by 5 to 32, preferably 8 to 20, more preferably 11 to 16 substitutions at positions of the residues of the binding interface of said starting OB-fold protein with its native ligand, possibly combined with the deletion of 1 to 4 residues and/or the insertion of 1 to 50 residues.

**3.** The biosensor of claim 2, wherein the residues which are substituted are selected amongst those of said OB-fold protein sequence which correspond to V2, K3, K5, K7, Y8, K9, G10, E14, T17, K21, K22, W24, V26, G27, K28, M29, S31, T33, D36, N37, G38, K39, T40, R42, A44, S46, E47, K48, D49, A50 and P51 of SEQ ID NO: 1.

**4.** The biosensor of claim 3, which comprises an OB-fold protein which is isolated from a combinatorial library obtained by the randomization of 11, 12, 13, 14, 15 or 16 residues selected amongst those of said OB-fold protein sequence which correspond to K7, Y8, K9, K21, K22, W24, V26, K28, M29, S31, T33, K39, T40, R42, A44 and S46 of SEQ ID NO: 1.

**5.** The biosensor of claim 4, wherein the randomized residues comprise at least those of said OB-fold protein sequence which correspond to K7, Y8, K9, W24, V26, M29, S31, T33, R42, A44 and S46 of SEQ ID NO: 1.

**6.** The biosensor of claim 5, wherein one, two or three additional residues selected amongst those of said OB-fold protein sequence which correspond to K21, K22 and T40 of SEQ ID NO: 1 are also randomized.

**7.** The biosensor of any one of claims 2 to 6, wherein said OB-fold protein comprises the insertion of 1 to 15 random amino acid residues in the region which corresponds to the residues in positions 25 to 30 of SEQ ID NO : 1, preferably between the residues corresponding to G27 and K28 of SEQ ID NO : 1, and/or the insertion of 1 to 15 random amino acid residues in the region corresponding to the residues in positions 35 to 40 of SEQ ID NO: 1, preferably between the residues corresponding to N37 and G38 of SEQ ID NO : 1, and/or the insertion of 1 to 20 random amino acid residues in the region corresponding to the residues in positions 7 to 12 of SEQ ID NO : 1, preferably between the residues corresponding to K9 and G10 of SEQ ID NO : 1.

**8.** The biosensor of any one of claims 2 to 7, wherein said OB-fold protein comprises the insertion of 1 to 4 amino acid residues selected amongst those which correspond to A59, R60, A61 and E64 of SEQ ID NO : 1.

**9.** The biosensor of claim 1, wherein said OB-fold protein comprises a sequence selected from the group consisting of SEQ ID NO: 1 to 45.

**10.** The biosensor of any one of claims 2 to 8, wherein the OB-fold protein is derived from a starting OB-fold protein comprising a sequence selected from the group consisting of SEQ ID NO: 1 to 45.

**11.** The biosensor of claim 10, wherein said OB-fold protein is selected from the group consisting of the sequence: $SVKVKFX_1X_2X_3GEEKEVDTSKIRDVCRQGKNVKFLYNDNGKYGAGX_4VX_5EK$ DAPKELLDMLARAEREKK (SEQ ID NO: 46), wherein $X_1$ to $X_5$ are, independently from each other, any amino acids, and the variants derived from SEQ ID NO: 46 by 1 to 15, more particularly one, two, three, four, five or six mutations or deletions in positions selected in the group consisting of positions 1-6, 11-20, 23, 25, 27-28, 30, 32, 36-39, 41, 43, 45 and 47-66; and wherein said biosensor is specific for human IgG.

**12.** The biosensor of claim 10 or claim 11, wherein said OB-fold protein comprises any of SEQ ID NO: 47 to 63.

**13.** The biosensor of any one of claims 1 to 12, wherein said OB-fold protein is coupled to a second protein moiety.

**14.** The biosensor of any one of claims 1 to 13, wherein said cysteine is either present in the OB-fold protein or substituted for another suitable residue.

**15.** The biosensor of claim 14, wherein said cysteine or substituted residue have a solvent accessible surface area (ASA) which is modified when said OB-fold protein binds to the target but are not in direct contact with said target.

**16.** The biosensor of claim 14, wherein said cysteine or substituted residue are located in proximity or are in spatial proximity with a residue which is functionally important for interaction of said OB-fold protein with said target.

**17.** The biosensor of any one of claims 1 to 16, wherein said fluorophore is coupled to one residue of said OB-fold protein which is selected from the residues which correspond to V2, K3, K5, K7, Y8, K9, G10, E14, T17, K21, K22, W24, V26, G27, K28, M29, S31, T33, D36, N37, G38, K39, T40, R42, A44, S46, E47, K48, D49, A50 and P51 of SEQ ID NO: 1, the residue being changed to cysteine residue if it is not already a cysteine residue.

**18.** The biosensor of claim 17, wherein said fluorophore is coupled to one residue of said OB-fold protein which is selected from the residues which correspond to K7, Y8, K9, K21, K22, W24, V26, M29, S31, T33, T40, R42, A44 and S46 of SEQ ID NO: 1.

**19.** The biosensor of claim 17, wherein said fluorophore is coupled to one residue of said OB-fold protein which corresponds to K39 of SEQ ID NO: 1.

**20.** The biosensor of any one of claims 1 to 19, wherein said fluorophore is selected from the group consisting of: 6-acryloyl-2-dimethylaminophtalene, 4-chloro-7-nitrobenz-2-oxa-1,3-diazole, 5-iodoacetamidoflurescein, (N-((2-(iodoacetoxy)ethyl)-N-methyl)amino-7-nitrobenz-2-oxa-1,3-diazole or a fluorophore having an aliphatic chain of 1 to 6 carbon atoms.

**21.** The biosensor of any one of claims 1 to 20, wherein said target is selected from the group consisting of: a peptide, a protein, an oligosaccharide, a lipid, a lipopeptide, a carbohydrate, a single-stranded DNA, a double-stranded DNA, a RNA, an hapten, a vitamin or a metallic ion.

**22.** A protein-based chip, **characterized in that** it consists of a solid support on which at least one biosensor as claimed in any one of claims 1 to 21 is immobilized.

**23.** An optical fibre comprising at a first end thereof at least one biosensor as claimed in any one of claims 1 to 21 and comprising at a second end thereof means to attach the optical fibre to a device configured to receive and interpret the output of the at least one biosensor.

**24.** The use of a biosensor as claimed in any one of claims 1 to 23 for screening libraries of molecules, sorting molecules, sorting cells, producing protein-based chips, or detecting, assaying or locating a target.

**25.** A method for producing reagentless biosensors which comprise at least an OB-fold protein and are specific for a target, as claimed in any one of claims 1 to 21, **characterized in that** it comprises the following steps:

(a) substituting each of the variable residues of said OB-fold protein, individually with a cysteine residue, thereby obtaining a set of cysteine mutants,
(b) coupling said cysteine residue of each of the cysteine mutants of step (a) to a fluorophore, thereby obtaining bioensors,
(c) purifying the biosensors of step (b), and
(d) measuring the equilibrium constant ($K_D$) between each of said purified biosensors of step (c) and said target, or the dissociation ($K_{off}$) and association ($k_{on}$) rate constants for each of said biosensors of step (c) and said target; and measuring the fluorescence variation of each of said biosensors of step (c) between a free and target bound state; and
(f) determining the sensitivity ($s$) and/or relative sensitivity ($s_r$) of each of said biosensors of step (c) from the measurements of step (d).

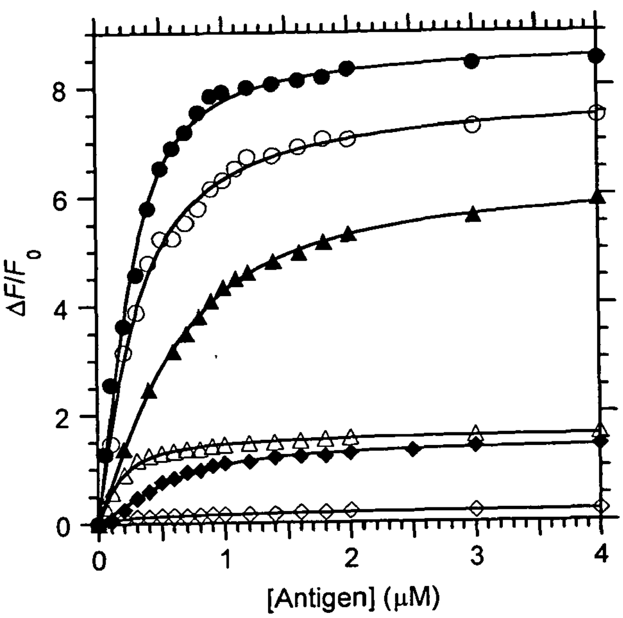


FIGURE 1

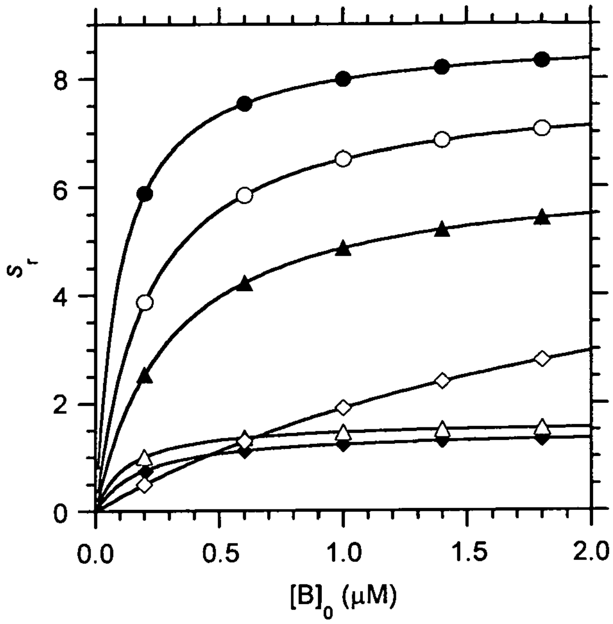


**FIGURE 2**

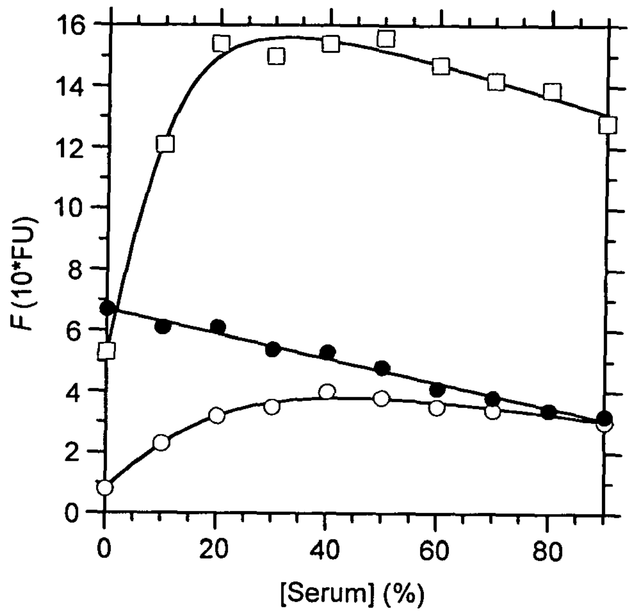


FIGURE 3

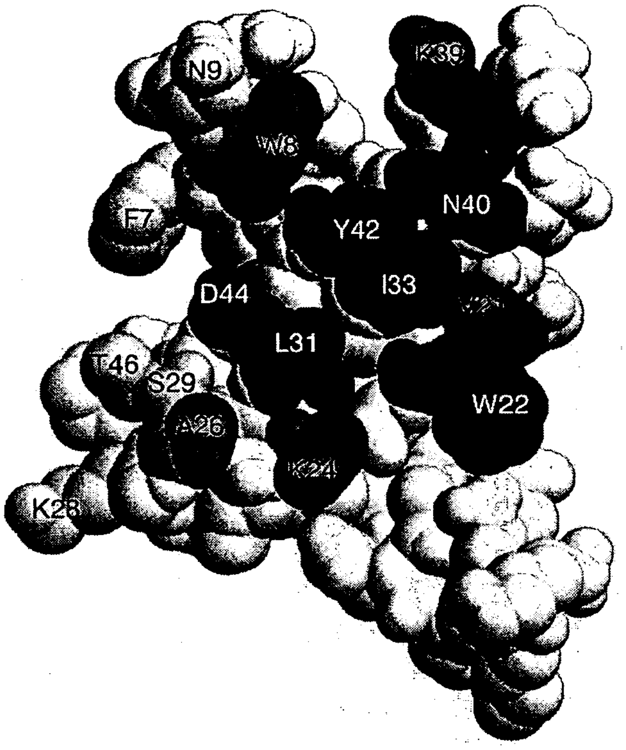


**FIGURE 4**

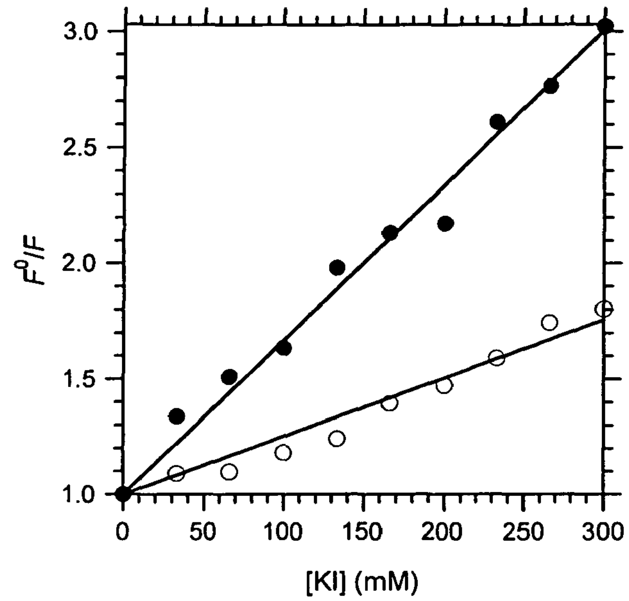


**FIGURE 5**

Europäisches Patentamt
European Patent Office
Office européen des brevets

# EUROPEAN SEARCH REPORT

Application Number
EP 10 29 0670

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | WO 2009/115919 A2 (PASTEUR INSTITUT [FR]; CENTRE NAT RECH SCIENT [FR]; BEDOUELLE HUGUES [) 24 September 2009 (2009-09-24)<br>* the whole document *<br>----- | 1-22 | INV.<br>G01N33/533<br>G01N33/542<br>C07K14/195<br>C12N15/10 |
| X,D | WO 01/65258 A1 (PASTEUR INSTITUT [FR]; CENTRE NAT RECH SCIENT [FR]; RENARD MARTIAL [FR) 7 September 2001 (2001-09-07)<br>* the whole document *<br>----- | 1-22 | |
| X,D | WO 2008/068637 A2 (PASTEUR INSTITUT [FR]; CENTRE NAT RECH SCIENT [FR]; PECORARI FREDERIC) 12 June 2008 (2008-06-12)<br>* the whole document *<br>* page 33 - page 34 *<br>----- | 1-22 | |
| X | WO 2007/139397 A1 (AUCKLAND UNISERVICES LTD [NZ]; ARCUS VICKERY LAURENCE [NZ]; STEEMSON J) 6 December 2007 (2007-12-06)<br>* the whole document *<br>* page 15 *<br>----- | 1-22 | |
| | | | TECHNICAL FIELDS SEARCHED (IPC) |
| X | GEBAUER M ET AL: "Engineered protein scaffolds as next-generation antibody therapeutics", CURRENT OPINION IN CHEMICAL BIOLOGY, CURRENT BIOLOGY LTD, LONDON, GB, vol. 13, no. 3, 1 June 2009 (2009-06-01), pages 245-255, XP026285197, ISSN: 1367-5931, DOI: DOI:10.1016/J.CBPA.2009.04.627 [retrieved on 2009-06-06]<br>* the whole document *<br>* page 246 - page 247, left-hand column *<br>-----<br>-/-- | 1-22 | G01N<br>C07K<br>C12N |

The present search report has been drawn up for all claims

2

| Place of search | Date of completion of the search | Examiner |
|---|---|---|
| Munich | 16 May 2011 | Cervigni, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 10 29 0670

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | B. MOURATOU ET AL: "Remodeling a DNA-binding protein as a specific in vivo inhibitor of bacterial secretin PulD", PROCEEDINGS OF THE NATIONAL ACADEMY OF SCIENCES, vol. 104, no. 46, 13 November 2007 (2007-11-13), pages 17983-17988, XP55000032, ISSN: 0027-8424, DOI: 10.1073/pnas.0702963104<br>* the whole document *<br>----- | 1-22 | |
| | | | TECHNICAL FIELDS SEARCHED (IPC) |

The present search report has been drawn up for all claims

2

| Place of search | Date of completion of the search | Examiner |
|---|---|---|
| Munich | 16 May 2011 | Cervigni, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 10 29 0670

This annex lists the patent family members relating to the patent documents cited in the above-mentioned European search report.
The members are as contained in the European Patent Office EDP file on
The European Patent Office is in no way liable for these particulars which are merely given for the purpose of information.

16-05-2011

| Patent document cited in search report | Publication date | Patent family member(s) | Publication date |
|---|---|---|---|
| WO 2009115919 A2 | 24-09-2009 | EP 2263088 A2 | 22-12-2010 |
| WO 0165258 A1 | 07-09-2001 | AT 348335 T | 15-01-2007 |
| | | AT 426165 T | 15-04-2009 |
| | | AU 4074201 A | 12-09-2001 |
| | | DE 60125145 T2 | 20-09-2007 |
| | | EP 1259809 A1 | 27-11-2002 |
| | | EP 2045603 A1 | 08-04-2009 |
| | | FR 2805820 A1 | 07-09-2001 |
| | | HK 1095881 A1 | 10-07-2009 |
| | | US 2003153012 A1 | 14-08-2003 |
| | | US 2010159480 A1 | 24-06-2010 |
| | | US 2009017454 A1 | 15-01-2009 |
| WO 2008068637 A2 | 12-06-2008 | AU 2007330409 A1 | 12-06-2008 |
| | | CA 2671553 A1 | 12-06-2008 |
| | | CN 101652386 A | 17-02-2010 |
| | | EP 1930342 A1 | 11-06-2008 |
| | | EP 2099817 A2 | 16-09-2009 |
| | | JP 2010511691 T | 15-04-2010 |
| | | US 2010145008 A1 | 10-06-2010 |
| WO 2007139397 A1 | 06-12-2007 | AU 2007268364 A1 | 06-12-2007 |
| | | CA 2653752 A1 | 06-12-2007 |
| | | CN 101506227 A | 12-08-2009 |
| | | EP 2029620 A1 | 04-03-2009 |
| | | JP 2009539346 T | 19-11-2009 |
| | | US 2010048413 A1 | 25-02-2010 |
| | | ZA 200810836 A | 28-07-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2008068637 A
- WO 2001065258 A
- WO 2009115919 A


### Non-patent literature cited in the description


- **AGRAWAL, V. ; KISHAN, K. V.** OB-fold: growing bigger with functional consistency. *Curr Protein Pept Sci,* 2003, vol. 4, 195-206
- **ALTSCHUH, D. ; ONCUL, S. ; DEMCHENKO, A.P.** Fluorescence sensing of intermolecular interactions and development of direct molecular biosensors. *J Mol Recognit,* 2006, vol. 19, 459-477
- **ARCUS, V.** OB-fold domains: a snapshot of the evolution of sequence, structure and function. *Curr Opin Struct Biol,* 2002, vol. 12, 794-801
- **ARNOLD, K. ; BORDOLI, L. ; KOPP, J. ; SCHWEDE, T.** The SWISS-MODEL workspace: a web-based environment for protein structure homology modelling. *Bioinformatics,* 2006, vol. 22, 195-201
- **BINZ, H.K. ; STUMPP, M.T. ; FORRER, P. ; AMSTUTZ, P. ; PLUCKTHUN, A.** Designing repeat proteins: well-expressed, soluble and stable proteins from combinatorial libraries of consensus ankyrin repeat proteins. *J Mol Biol,* 2003, vol. 332, 489-503
- **BINZ, H. K. ; AMSTUTZ, P. ; KOHL, A. ; STUMPP, M. T. ; BRIAND, C. ; FORRER, P. ; GRUTTER, M. G. ; PLUCKTHUN, A.** High-affinity binders selected from designed ankyrin repeat protein libraries. *Nat Biotechnol,* 2004, vol. 22, 575-582
- **BRIENT-LITZLER, E. ; PLUCKTHUN, A. ; BEDOUELLE, H.** Knowledge-based design of reagentless fluorescent biosensors from a designed ankyrin repeat protein. *Protein Eng Des Sel,* 2010, vol. 23, 229-241
- **CINIER, M. ; PETIT, M. ; WILLIAMS, M. N. ; FABRE, R. M. ; PECORARI, F. ; TALHAM, D. R. ; BUJOLI, B. ; TELLIER, C.** Bisphosphonate adaptors for specific protein binding on zirconium phosphonate-based microarrays. *Bioconjug Chem,* 2009, vol. 20, 2270-2277
- **FOOTE, J. ; WINTER, G.** Antibody framework residues affecting the conformation of the hypervariable loops. *J Mol Biol,* 1992, vol. 224, 487-499
- **GASTEIGER, E. ; GATTIKER, A. ; HOOGLAND, C. ; IVANYI, I. ; APPEL, R. D. ; BAIROCH, A.** ExPASy: The proteomics server for in-depth protein knowledge and analysis. *Nucleic Acids Res,* 2003, vol. 31, 3784-3788
- **HOLM, L. ; PARK, J.** DaliLite workbench for protein structure comparison. *Bioinformatics,* 2000, vol. 16, 566-567
- **HOLM, L. ; SANDER, C.** Touring protein fold space with Dali/FSSP. *Nucleic Acids Res,* 1998, vol. 26, 316-319
- **JESPERS, L. ; BONNERT, T.P. ; WINTER, G.** Selection of optical biosensors from chemisynthetic antibody libraries. *Protein Eng Des Sel,* 2004, vol. 17, 709-713
- **KITOV, P. I. ; SADOWSKA, J. M. ; MULVEY, G. ; ARMSTRONG, G. D. ; LING, H. ; PANNU, N. S. ; READ, R. J. ; BUNDLE, D. R.** Shiga-like toxins are neutralized by tailored multivalent carbohydrate ligands. *Nature,* 2000, vol. 403, 669-672
- **KLOKS, C. P. ; SPRONK, C. A. ; LASONDER, E. ; HOFFMANN, A. ; VUISTER, G. W. ; GRZESIEK, S. ; HILBERS, C. W.** The solution structure and DNA-binding properties of the cold-shock domain of the human Y-box protein YB-1. *J Mol Biol,* 2002, vol. 316, 317-326
- **KREHENBRINK, M. ; CHAMI, M. ; GUILVOUT, I. ; ALZARI, P. M. ; PECORARI, F. ; PUGSLEY, A. P.** Artificial binding proteins (Affitins) as probes for conformational changes in secretin PulD. *J Mol Biol,* 2008, vol. 383, 1058-1068
- **LAKOWICZ, J. R.** Principles of fluorescent spectroscopy. Kluwer Academic/Plenum, 1999
- **LI, J. ; MAHAJAN, A. ; TSAI, M. D.** Ankyrin repeat: a unique motif mediating protein-protein interactions. *Biochemistry,* 2006, vol. 45, 15168-15178
- **LOPEZ, R. ; SILVENTOINEN, V. ; ROBINSON, S. ; KIBRIA, A. ; GISH, W.** WU-Blast2 server at the European Bioinformatics Institute. *Nucleic Acids Res,* 2003, vol. 31, 3795-3798
- **LOWE, C.R.** *Biosensors. Trends Biotechnol,* 1984, vol. 2, 59-65

- **MCAFEE, J. G. ; EDMONDSON, S. P. ; DATTA, P. K. ; SHRIVER, J. W. ; GUPTA, R.** Gene cloning, expression, and characterization of the Sac7 proteins from the hyperthermophile Sulfolobus acidocaldarius. *Biochemistry,* 1995, vol. 34, 10063-10077
- **MITTON-FRY, R. M. ; ANDERSON, E. M. ; HUGHES, T. R. ; LUNDBLAD, V. ; WUTTKE, D. S.** Conserved structure for single-stranded telomeric DNA recognition. *Science,* 2002, vol. 296, 145-147
- **MORGAN, C.L. ; NEWMAN, D.J. ; PRICE, C.P.** Immunosensors: technology and opportunities in laboratory medicine. *Clin Chem,* 1996, vol. 42, 193-209
- **MOSAVI, L. K. ; CAMMETT, T. J. ; DESROSIERS, D. C. ; PENG, Z. Y.** The ankyrin repeat as molecular architecture for protein recognition. *Protein Sci,* 2004, vol. 13, 1435-1448
- **MOURATOU, B. ; SCHAEFFER, F. ; GUILVOUT, I. ; TELLO-MANIGNE, D. ; PUGSLEY, A. P. ; ALZARI, P. M. ; PECORARI, F.** Remodeling a DNA-binding protein as a specific in vivo inhibitor of bacterial secretin PulD. *Proc Natl Acad Sci U S A,* 2007, vol. 104, 17983-17988
- **MURZIN, A. G.** OB(oligonucleotide/oligosaccharide binding)-fold: common structural and functional solution for non-homologous sequences. *Embo J,* 1993, vol. 12, 861-867
- **NOTREDAME, C. ; HIGGINS, D. G. ; HERINGA, J.** T-Coffee: A novel method for fast and accurate multiple sequence alignment. *J Mol Biol,* 2000, vol. 302, 205-217
- **PAPAGEORGIOU, A. C. ; TRANTER, H. S. ; ACHARYA, K. R.** Crystal structure of microbial superantigen staphylococcal enterotoxin B at 1.5 A resolution: implications for superantigen recognition by MHC class II molecules and T-cell receptors. *J Mol Biol,* 1998, vol. 277, 61-79
- **PEARL, F. M. ; BENNETT, C. F. ; BRAY, J. E. ; HARRISON, A. P. ; MARTIN, N. ; SHEPHERD, A. ; SILLITOE, I. ; THORNTON, J. ; ORENGO, C. A.** The CATH database: an extended protein family resource for structural and functional genomics. *Nucleic Acids Res,* 2003, vol. 31, 452-455
- **QIAN, J. ; STENGER, B. ; WILSON, C. A. ; LIN, J. ; JANSEN, R. ; TEICHMANN, S. A. ; PARK, J. ; KREBS, W. G. ; YU, H. ; ALEXANDROV, V. et al.** PartsList: a web-based system for dynamically ranking protein folds based on disparate attributes, including whole-genome expression and interaction information. *Nucleic Acids Res,* 2001, vol. 29, 1750-1764
- **RENARD, M. ; BEDOUELLE, H.** Improving the sensitivity and dynamic range of reagentless fluorescent immunosensors by knowledge-based design. *Biochemistry,* 2004, vol. 43, 15453-15462
- **RENARD, M. ; BELKADI, L. ; BEDOUELLE, H.** Deriving topological constraints from functional data for the design of reagentless fluorescent immunosensors. *J Mol Biol,* 2003, vol. 326, 167-175
- **RENARD, M. ; BELKADI, L. ; HUGO, N. ; ENGLAND, P. ; ALTSCHUH, D. ; BEDOUELLE, H.** Knowledge-based design of reagentless fluorescent biosensors from recombinant antibodies. *J Mol Biol,* 2002, vol. 318, 429-442
- **ROBINSON, H. ; GAO, Y. G. ; MCCRARY, B. S. ; EDMONDSON, S. P. ; SHRIVER, J. W. ; WANG, A. H.** The hyperthermophile chromosomal protein Sac7d sharply kinks DNA. *Nature,* 1998, vol. 392, 202-205
- **ROUSSEL, A. ; ANDERSON, B. F. ; BAKER, H. M. ; FRASER, J. D. ; BAKER, E. N.** Crystal structure of the streptococcal superantigen SPE-C: dimerization and zinc binding suggest a novel mode of interaction with MHC class II molecules. *Nat Struct Biol,* 1997, vol. 4, 635-643
- **SAMYGINA, V. R. ; POPOV, A. N. ; RODINA, E. V. ; VOROBYEVA, N. N. ; LAMZIN, V. S. ; POLYAKOV, K. M. ; KURILOVA, S. A. ; NAZAROVA, T. I. ; AVAEVA, S. M.** The structures of Escherichia coli inorganic pyrophosphatase complexed with Ca(2+) or CaPP(i) at atomic resolution and their mechanistic implications. *J Mol Biol,* 2001, vol. 314, 633-645
- **SMITH, J.J. ; CONRAD, D.W. ; CUNEO, M.J. ; HELLINGA, H.W.** Orthogonal site-specific protein modification by engineering reversible thiol protection mechanisms. *Protein Sci,* 2005, vol. 14, 64-73
- **STUMPP, M. T. ; BINZ, H. K. ; AMSTUTZ, P.** DARPins: A new generation of protein therapeutics. *Drug Discov. Today,* 2008, vol. 13, 695-701
- **THEOBALD, D. L. ; MITTON-FRY, R. M. ; WUTTKE, D. S.** Nucleic acid recognition by OB-fold proteins. *Annu Rev Biophys Biomol Struct,* 2003, vol. 32, 115-133
- **THEVENOT, D.R. ; TOTH, K. ; DURST, R.A. ; WILSON, G.S.** Electrochemical biosensors: recommended definitions and classification. *Biosens Bioelectron,* 2001, vol. 16, 121-131